(19) Europäisches Patentamt
European Patent Office
Office européen des brevets

(11) Veröffentlichungsnummer: **0 262 448 B1**

(12) **EUROPÄISCHE PATENTSCHRIFT**

(45) Veröffentlichungstag der Patentschrift: **18.12.91**

(51) Int. Cl.$^5$: **C07D 233/64**, C07D 401/12, C07D 409/12, A61K 31/415, A61K 31/44, A61K 31/38

(21) Anmeldenummer: **87112906.0**

(22) Anmeldetag: **03.09.87**

Die Akte enthält technische Angaben, die nach dem Eingang der Anmeldung eingereicht wurden und die nicht in dieser Patentschrift enthalten sind.

(54) **Imidazolylguanidinderivate, Verfahren zu ihrer Herstellung und diese Verbindungen enthaltende Arzneimittel.**

(30) Priorität: **15.09.86 DE 3631334**

(43) Veröffentlichungstag der Anmeldung: **06.04.88 Patentblatt 88/14**

(45) Bekanntmachung des Hinweises auf die Patenterteilung: **18.12.91 Patentblatt 91/51**

(84) Benannte Vertragsstaaten: **AT BE CH DE ES FR GB GR IT LI LU NL SE**

(56) Entgegenhaltungen:
**EP-A- 0 199 845**
**DE-A- 2 433 625**

(73) Patentinhaber: **HEUMANN PHARMA GMBH & CO**
**Heideloffstrasse 18 - 28**
**W-8500 Nürnberg 1(DE)**

(72) Erfinder: **Buschauer,Armin,Dr.**
**Bachestrasse 5**
**W-1000 Berlin 41(DE)**
Erfinder: **Schickaneder,Helmut,Dr.Dipl.-Chem.**
**Moosäcker 25**
**W-8501 Eckental(DE)**
Erfinder: **Mörsdorf,Peter,Dr.Dipl.-Chem.**
**Ziegelstrasse 64**
**W-8506 Langenzehn(DE)**
Erfinder: **Schunack,Walter,Prof.Dr.Dr.**
**Spanische Allee 95**
**W-1000 Berlin 38(DE)**
Erfinder: **Baumann,Gert,Dr.**
**Ismaninger Strasse 22**
**W-8000 München 80(DE)**
Erfinder: **Henning Ahrens,Kurt,Dr.**
**Praterstrasse 9**
**W-8500 Nürnberg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

EP 0 262 448 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Gegenstand der Erfindung sind neue Imidazolylguanidinderivate, die aufgrund ihrer agonistischen Wirkung auf Histamin-$H_2$-Rezeptoren sowie Zum Teil wegen ihrer zusätzlichen $H_1$-antagonistischen Rezeptoraktivität bei Erkrankungen des Herzens, bei bestimmten Formen der Hypertonie sowie bei arteriellen Verschlußkrankheiten eingesetzt werden können. Histamin als spezifischer Stimulator der $H_2$-Rezeptoren löst wegen seiner $H_1$-agonistischen Wirkung negative, zum Teil tödliche Effekte in Form eines Bronchospasmus und anaphylaktischen Schocks aus, so daß die therapeutische Nutzung des Histamins bei der Behandlung der genannten Erkrankungen nicht möglich ist.

Aus der DE-A-2 433 625 sind spezielle Harnstoffderivate bekannt, welche die durch Histamin stimulierte Sekretion der Magensäure hemmen. Chem. Soc. Review 14, (1985), Seiten 375-399 "Guanidine Derivatives acting at Histaminergic Receptors" beschreibt, daß bestimmte Imidazolylguanidinderivate Histamin-$H_2$-Rezeptor-agonistische Aktivität besitzen.

Der Erfindung liegt die Aufgabe zugrunde, die unvorteilhaften Wirkungen des Histamins zu kompensieren und bessere und selektiver wirkende $H_2$-Agonisten zur Verfügung zu stellen, bei denen die durch eine $H_1$-agonistische Wirkkomponente bedingten schädlichen Nebenwirkungen u.U. durch ein zusätzliches $H_1$-antagonistisches Wirkprofil vermieden werden können.

Diese Aufgabe wird durch die Erfindung gelöst.

Gegenstand der Erfindung sind Imidazolylguanidinderivate der allgemeinen Formel I

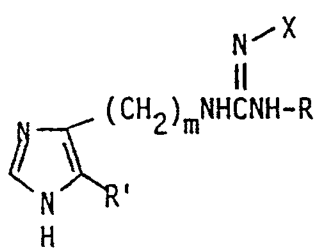

(I)

in der R die Gruppierung

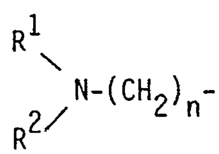

bedeutet, wobei $R^1$ für eine unsubstituierte oder eine ein- bis dreifach mit $C_1$-$C_3$-Alkyl, $C_1$-$C_3$-Alkoxy, Halogen, Trifluormethyl und/oder Hydroxy substituierte Phenylgruppe oder einen unsubstituierten oder einen ein bis dreifach mit $C_1$-$C_3$-Alkyl, $C_1$-$C_3$-Alkoxy und/oder Halogen substituierten Pyridinring steht, $R^2$ für ein Wasserstoffatom, eine $C_1$-$C_3$-Alkylgruppe, eine gegebenenfalls ein- bis dreifach mit $C_1$-$C_3$-Alkyl, $C_1$-$C_3$-Alkoxy, Halogen, Trifluormethyl und/oder Hydroxy substituierte Phenylgruppe, eine unsubstituierte oder eine ein- bis dreifach mit $C_1$-$C_3$-Alkyl, $C_1$-$C_3$-Alkoxy, Halogen und/oder Trifluormethyl substituierte Benzylgruppe oder eine unsubstituierte oder eine ein- bis dreifach mit $C_1$-$C_3$-Alkyl, $C_1$-$C_3$-Alkoxy und/oder Halogen substituierte Thiophenylmethyl-, Furanylmethyl- oder Pyridylmethylgruppe steht und n den Wert 2, 3 oder 4, hat, oder

in der R die Gruppierung

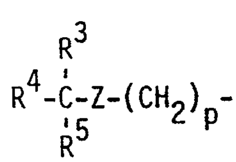

bedeutet, worin $R^3$ für eine unsubstituierte oder eine ein- bis dreifach mit $C_1$-$C_3$-Alkyl, $C_1$-$C_3$-Alkoxy,

Halogen, Trifluormethyl und/oder Hydroxy substituierte Phenylgruppe oder einen unsubstituierten oder einen ein- bis dreifach mit $C_1$-$C_3$-Alkyl, $C_1$-$C_3$-Alkoxy und/oder Halogen substituierten Pyridinring steht, $R^4$ ein Wasserstoffatom oder eine gegebenenfalls ein- bis dreifach mit $C_1$-$C_3$-Alkyl, $C_1$-$C_3$-Alkoxy, Halogen, Trifluormethyl und/oder Hydroxy substituierte Phenylgruppe bedeutet, $R^5$ für ein Wasserstoffatom oder eine Methyl- oder Hydroxygruppe und Z für eine Einfachbindung oder ein Sauerstoffatom stehen und p den Wert 2 oder 3 hat,

X ein Wasserstoffatom oder eine Benzoylgruppe bedeutet, m den Wert 2 oder 3 hat und $R^1$ ein Wasserstoffatom oder eine Methylgruppe bedeutet, sowie die physiologisch annehmbaren Salze davon.

In der allgemeinen Formel I bedeutet R die Gruppierung

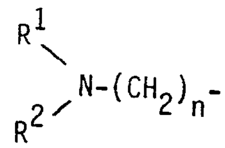

In dieser Gruppierung steht $R^1$ für eine unsubstituierte oder eine einbis dreifach substituierte Phenylgruppe, vorzugsweise eine zweifach substituierte Phenylgruppe. Im Falle der Substitution kommen insbesondere 1 bis 3 Halogenatome, wie Fluor-, Chlor- oder Bromatome, vorzugsweise Fluor- oder Chloratome, 1 bis 3 $C_1$-$C_3$-Alkylgruppen, vorzugsweise Methyl- oder Ethylgruppen, und 1 bis 3 $C_1$-$C_3$-Alkoxygruppen, wie Methoxy- oder Ethoxygruppen, in Betracht. Die Einfachsubstitution und die Zweifachsubstitution werden bevorzugt. Im Falle der Einfachsubstitution wird die Substitution in 4-Position und im Falle der Zweifachsubstitution die Substitution in 3- und 4-Position des Phenylrings bevorzugt. Als weiterer Substituent des Phenylrings kommt die Trifluormethylgruppe in Betracht. Vorzugsweise ist die Phenylgruppe mit 1 bis 3 Trifluormethylgruppen, besonders bevorzugt einer Trifluormethylgruppe, substituiert. In diesem Fall erfolgt die Bindung vorzugsweise in 4-Position des Phenylrings.

Der Substituent $R^1$ kann weiterhin ein unsubstituierter oder ein einbis dreifach substituierter Pyridinring sein. Im Falle der Mehrfachsubstitution ist der Pyridinring vorzugsweise ein- oder zweifach substituiert. Als Substituenten des Pyridinrings kommen beispielsweise Halogenatome, wie Fluor-, Chlor- oder Bromatome, vorzugsweise Fluor- oder Chloratome, ganz besonders bevorzugt Fluoratome, $C_1$-$C_3$-Alkylgruppen, wie Methyl- oder Ethylgruppen, sowie $C_1$-$C_3$-Alkoxygruppen, wie Methoxy-, Ethoxy- oder Propoxygruppen, vorzugsweise Methoxygruppen, in Betracht.

Die Verknüpfung des durch $R^1$ angegebenen Pyridinrings mit dem Stickstoffatom in der Gruppierung R kann in 2-, 3- oder 4-Position des Pyridinrings erfolgen, wobei die 2- oder 3-Position bevorzugt wird. Dabei wird die Verknüpfung in der 2-Position des Pyridinrings ganz besonders bevorzugt.

$R^2$ steht für ein Wasserstoffatom, eine $C_1$-$C_3$-Alkylgruppe, insbesondere eine Methyl-, Ethyl- oder Propylgruppe, eine Phenylgruppe, die unsubstituiert sein kann oder gegebenenfalls ein- bis dreifach, insbesondere ein- oder zweifach substituiert sein kann, eine Benzylgruppe, die unsubstituiert oder ein- bis dreifach substituiert sein kann, oder eine wie oben definierte Heteroarylmethylgruppe, die unsubstituiert sein kann oder ein- bis dreifach substituiert sein kann. Im Falle der Substitution kann die durch $R^2$ angegebene Phenylgruppe in der gleichen Weise und mit den gleichen Substituenten wie oben im Zusammenhang mit der Substitution der durch $R^1$ angegebenen Phenylgruppe beschrieben substituiert sein.

Im Falle der Substitution kann die Benzylgruppe mit 1 bis 3, vorzugsweise 2 Halogenatomen, wie Fluor-, Chlor- oder Bromatomen, vorzugsweise Chlor- oder Fluoratomen, oder $C_1$-$C_3$-Alkoxygruppen, wie Methoxy- oder Ethoxygruppen, vorzugsweise Methoxygruppen, substituiert sein. Im Falle der Einfachsubstitution der durch $R^2$ angegebenen Benzylgruppe ist der Substituent vorzugsweise in para-Position zur Methylengruppe gebunden, während im Falle der Zweifachsubstitution die 3- und 4-Position der Benzylgruppe bevorzugt wird. Als weiterer Substituent für die durch $R^2$ angegebene Benzylgruppe kommt die Trifluormethylgruppe in Betracht, wobei die Benzylgruppe durch 1 bis 3 Trifluormethylgruppen substituiert sein kann. Bevorzugt wird die Einfachsubstitution mit der Trifluormethylgruppe, wobei diese vorzugsweise in para-Position zur Benzylgruppe gebunden ist.

Wenn $R^2$ eine Heteroarylmethylgruppe darstellt, dann ist diese Gruppe eine Thiophenylmethyl-, Furanmethyl- oder Pyridinmethylgruppe. Auch diese kann unsubstituiert oder vorzugsweise ein- bis dreifach substituiert sein. Die Einfach- oder Zweifachsubstitution wird bevorzugt. Als Substituenten kommen Halogenatome, wie Fluor-, Chlor- oder Bromatome, $C_1$-$C_3$-Alkylgruppen, wie Methyl- oder Ethylgruppen, und lineare $C_1$-$C_3$-Alkoxygruppen, wie Methoxygruppen, in Betracht.

n hat den Wert 2, 3 oder 4, wobei der Wert 3 bevorzugt wird.

R kann weiterhin die Gruppierung

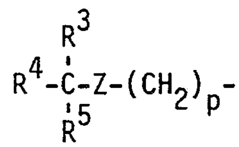

bedeuten. In dieser Gruppierung kann $R^3$ eine unsubstituierte oder eine ein- bis dreifach substituierte Phenylgruppe oder einen ein- bis dreifach substituierten Pyridinring bedeuten. Im Falle der Substitution kommen insbesondere 1 bis 3 Halogenatome, wie Fluor-, Chlor- oder Bromatome, vorzugsweise Fluor- oder Chloratome, 1 bis 3 $C_1$-$C_3$-Alkylgruppen, vorzugsweise Methyl- oder Ethylgruppen, und 1 bis 3 $C_1$-$C_3$-Alkoxygruppen, wie Methoxy- oder Ethoxygruppen, in Betracht. Die Einfachsubstitution und die Zweifachsubstitution werden bevorzugt. Im Falle der Einfachsubstitution wird die Substitution in 4-Position und im Fall der Zweifachsubstitution die Substitution in 3- und 4-Position des Phenylrings bevorzugt. Als weitere Substitution des Phenylrings kommen die Trifluormethylgruppe und die Hydroxygruppe in Betracht. Vorzugsweise ist die Phenylgruppe mit einer Trifluormethylgruppe oder einer Hydroxygruppe substituiert, wobei die Bindung jeweils vorzugsweise in 4-Position des Phenylrings erfolgt. Der Substituent $R^3$ kann weiterhin ein unsubstituierter oder ein ein- bis d reifach substituierter Pyridinring, vorzugsweise ein unsubstituierter Pyridinrings oder ein einfach substituierter Pyridinring sein. Als Substituenten des Pyridinrings kommen beispielsweise Halogenatome, wie Fluor-, Chlor- oder Bromatome, vorzugsweise Fluor-, oder Chloratome, ganz besonders bevorzugt Fluoratome, $C_1$-$C_3$-Alkylgruppen, wie Methyl- oder Ethylgruppen, sowie $C_1$-$C_3$-Alkoxygruppen, wie Methoxy-, Ethoxy- oder Propoxygruppen, vorzugsweise Methoxygruppen, in Betracht.

Die Verknüpfung des durch $R^3$ angegebenen Pyridinrings mit dem Stickstoffatom in der Gruppierung R kann in 2-, 3- oder 4-Position des Pyridinrings erfolgen, wobie die 2- oder 3-Position bevorzugt wird. Dabei wird die Verknüpfung in der 2-Position des Pyridinrings ganz besonders bevorzugt.

$R^4$ bedeutet ein Wasserstoffatom oder eine unsubstituierte oder eine ein- bis dreifach substituierte Phenylgruppe. Im Falle der Substitution ist die durch $R^4$ angegebene Phenylgruppe in der gleichen Weise substituiert wie die durch $R^3$ angegebene Phenylgruppe. $R^5$ bedeutet ein Wasserstoffatom oder eine Methyl- oder Hydroxygruppe. Z stellt eine Einfachbindung oder ein Sauerstoffatom dar, während p den Wert 2 oder 3 hat.

In der allgemeinen Formel I bedeutet weiterhin X ein Wasserstoffatom oder eine Benzoylgruppe. m hat den Wert 2 oder 3, vorzugsweise 3, und R'bedeutet ein Wasserstoffatom oder eine Methylgruppe, vorzugsweise ein Wasserstoffatom.

Bei einer bevorzugten Gruppe von erfindungsgemäßen Verbindungen steht in der allgemeinen Formel I R für die Gruppierung

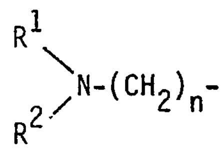

wobei $R^1$ für einen unsubstituierten oder ein- bis dreifach, vorzugsweise einfach oder zweifach, ganz bevorzugt einen unsubstituierten, Pyridinring steht. Im Fall der Substitution des Pyridinrings können die Substituenten Halogenatome, wie zum Beispiel ein Fluor-, Chlor- oder Bromatom, vorzugsweise ein Fluor- oder Chloratom, insbesondere ein Fluoratom, $C_1$-$C_3$-Alkylgruppen, wie eine Methyl- oder Ethylgruppe, oder $C_1$-$C_3$-Alkoxygruppen, wie eine Methoxy-, Ethoxy- oder Propoxygruppe, vorzugsweise eine Methoxygruppe, sein.

Die Verknüpfung des Pyridinrings mit dem Stickstoffatom in R kann in 2-, 3- oder 4-Position des Pyridinrings erfolgen, wobei die 2- oder 3-Position bevorzugt wird. Ganz besonders bevorzugt ist die Verknüpfung in der 2-Position des Pyridinrings.

$R^2$ steht für eine gegebenenfalls ein- bis dreifach substituierte Benzylgruppe oder eine unsubstituierte oder ein- bis dreifach substituierte, wie oben definierte Heteroarylmethylgruppe. Die Benzylgruppe kann beispielsweise mit einem bis drei, vorzugsweise zwei Halogenatomen, wie zum Beispiel einem Fluor-,

Chlor- oder Bromatom, vorzugsweise einem Chlor- oder Fluoratom, oder einer $C_1$-$C_3$-Alkoxygruppe, wie einer Methoxy- oder Ethoxygruppe, bevorzugt einer Methoxygruppe, substituiert sein, wobei der Substituent vorzugsweise in para-Position zur Methylengruppe gebunden ist. Bei einer Zweifachsubstitution ist die 3- und 4-Position der Benzylgruppe bevorzugt.

Als weiterer Substituent kommt die $CF_3$-Gruppe in Frage. Vorzugsweise ist die Trifluormethylgruppe in para-Position der Benzylgruppe gebunden.

Die Heteroarylmethylgruppe ist eine Thiophenmethyl-, Furanmethyl- oder Pyridinmethylgruppe, die gegebenenfalls mit einem oder zwei Halogenatomen, wie Fluor-, Chlor- oder Bromatomen, einer $C_1$-$C_3$-Alkylgruppe, zum Beispiel einer Methyl- oder Ethylgruppe, oder einer linearen $C_1$-$C_3$-Alkoxygruppe, zum Beispiel einer Methoxygruppe, substituiert ist.

Bei dieser bevorzugten Gruppe von erfindungsgemäßen Verbindungen hat n den Wert 2, 3 oder 4, vorzugsweise 2 oder 3. X und R' bedeuten vorzugsweise ein Wasserstoffatom, während m den Wert 2 oder 3, vorzugsweise 3, hat.

Bei einer weiteren bevorzugten Gruppe von erfindungsgemäßen Verbindungen steht R für die Gruppierung

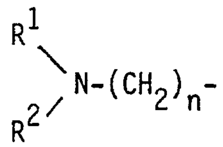

wobei $R^1$ einen unsubstituierten oder ein- bis dreifach substituierten Phenylring bedeutet. Der Phenylring kann mit 1 bis 3 Halogenatomen, wie Fluor-, Chlor- oder Bromatomen, vorzugsweise Fluor- oder Chloratomen, $C_1$-$C_3$-Alkylgruppen, vorzugsweise Methyl- oder Ethylgruppen, oder $C_1$-$C_3$-Alkoxygruppen, wie Methoxy- oder Ethoxygruppen, substituiert sein, die sich vorzugsweise in 4-Position oder im Falle einer Zweifachsubstitution in 3- und 4-Position befinden. Ein weiterer bevorzugter Substituent ist die Trifluormethylgruppe, die vorzugsweise in 4-Position des Phenylrings gebunden ist.

$R^2$ bedeutet eine unsubstituierte oder eine ein- bis dreifach substituierte Heteroarylmethylgruppe, nämlich eine Thiophenmethyl-, Furanmethyl- oder Pyridinmethylgruppe, vorzugsweise eine Thiophenmethylgruppe. Bezüglich der Substitution der Heteroarylmethylgruppe gilt das oben Gesagte.

Der Index n hat den Wert 2, 3 oder 4, vorzugsweise 2 oder 3, wobei der Wert 2 besonders bevorzugt ist. X und R' bedeuten vorzugsweise ein Wasserstoffatom, während m den Wert 2 oder 3, vorzugsweise 3, hat.

Bei einer weiteren bevorzugten Gruppe von erfindungsgmäßen Verbindungen der allgemeinen Formel I steht R für die Gruppierung

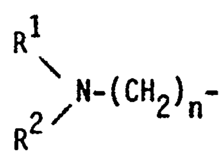

wobei $R^1$ für einen unsubstituierten oder ein- bis dreifach substituierten, vorzugsweise einen unsubstituierten, Pyridinring und $R^2$ für ein Wasserstoffatom stehen. Ist der Pyridinring mehrfach substituiert, so kommen ein bis drei gleiche oder verschiedene Substituenten aus der Gruppe Halogenatome, lineare $C_1$-$C_3$-Alkylgruppen und lineare $C_1$-$C_3$-Alkoxygruppen in Betracht. Der Pyridinring kann in den Positionen 2, 3 oder 4 mit dem Stickstoffatom verknüpft sein, wobei die Positionen 2 und 3, ganz besonders die Position 2, bevorzugt sind.

n hat den Wert 2, 3 oder 4, bevorzugt den Wert 3. X und R′ stehen vorzugsweise für ein Wasserstoffatom, während m den Wert 2 oder 3, vorzugsweise 3, hat.

Bei einer weiteren bevorzugten Gruppe von erfindungsgemäßen Verbindungen steht in der allgemeinen Formel I R für die Gruppierung

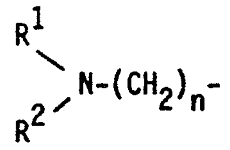

wobei $R^1$ für einen unsubstituierten oder ein- bis dreifach substituierten Pyridinring und $R^2$ für einen unsubstituierten oder ein- bis dreifach substituierten Phenylring steht. Wenn der Pyridinring oder der Phenylring substituiert ist, sind die Substituenten vorzugsweise Halogenatome, zum Beispiel Fluor-, Chlor- oder Bromatome, vorzugsweise Fluor- oder Chloratome, lineare $C_1$-$C_3$-Alkoxygruppen, wie Methoxy- oder Ethoxygruppen, vorzugsweise eine Methoxygruppe, oder lineare $C_1$-$C_3$-Alkylgruppen, zum Beispiele eine Methyl- oder Ethylgruppe, vorzugsweise eine Methylgruppe.

Der Pyridinring kann mit dem Stickstoffatom in R in den Positionen 2, 3 und 4 verknüpft sein, wobei die Position 2 besonders bevorzugt ist. In diesem Fall stehen gegebenenfalls vorhandene Substituenten in den Positionen 3 und/oder 5.

Ist der Phenylring einfach substituiert, dan wird die Substitution in 4-Position bevorzugt. Ganz besonders bevorzugt ist die Substitution mit einem Chlor- oder Fluoratom in 4-Position. Ist der Phenylring zweifach substituiert, dann werden die Substitutionen in 3- und 4-Position bevorzugt. Weiterhin als Substituent bevorzugt ist die Trifluormethylgruppe, die vorzugsweise in Position 4 des Phenylrings gebunden ist. X und R′ bedeuten vorzugsweise ein Wasserstoffatom, n hat den Wert 2, 3 oder 4, vorzugsweise den Wert 3, während für m der Wert 3 bevorzugt wird.

Bei einer weiteren bevorzugten Gruppe von erfindungsgemäßen Verbindungen steht in der allgemeinen Formel I R für die Gruppierung

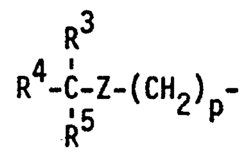

wobei $R^3$ und $R^4$, die gleich oder verschieden sein können, jeweils für eine unsubstituierte oder ein- bis dreifach substituierte Phenylgruppe stehen. Als Substituenten kommen beispielsweise Halogenatome, wie Fluor-Chlor- oder Bromatome, lineare $C_1$-$C_3$-Alkoxygruppen, wie zum Beispiel vorzugsweise Methoxygruppen, oder lineare $C_1$-$C_3$-Alkylgruppen, vorzugsweise Methyl-oder Ethylgruppen, in Betracht. Bevorzugt werden Halogenatome, insbesondere Fluor- oder Chloratome, als Substituenten, die in 2-, 3-oder vorzugsweise in 4-Position oder bei Zweifachsubstitution in 3-/4-oder 3-/5-Position, vorzugsweise in 3-/4-Position, des Phenylrings stehen. Als weiterer bevorzugter Substituent kommt die Trifluomethylgruppe in Betracht, wobei die 4-Position im Phenylring bevorzugt ist.

$R^5$ steht, wie oben definiert, für ein Wasserstoffatom oder eine Methylgruppe, Z bedeutet eine Einfachbindung, und p hat den Wert 2 oder 3, bevorzugt den Wert 2. X und R' bedeuten vorzugsweise ein Wasserstoffatom, während m bevorzugt den Wert 3 besitzt.

Bei einer weiteren bevorzugten Gruppe von erfindungsgemäßen Verbindungen der allgemeinen Formel I steht R für die Gruppierung

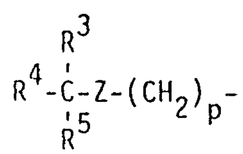

wobei $R^4$ für eine unsubstituierte oder ein- bis dreifach substituierte Phenylgruppe steht. Im Fall einer substituierten Phenylgruppe sind Halogenatome, wie Fluor-, Chlor- oder Bromatome, insbesondere Fluor- oder Chloratome, lineare $C_1$-$C_3$-Alkoxygruppen, zum Beispiel eine Methoxy-, Ethoxy- oder Propoxygruppe, und lineare $C_1$-$C_3$-Alkylgruppen, wie Methyl- oder Ethylgruppen, oder eine Hydroxygruppe als Substituenten bevorzugt. Die Substituenten stehen dann vorzugsweise in 4-Position oder bei Zweifachsubstitution in 2-/6-, 3-/5- oder in 3-/4-Position. Aber auch alle anderen noch denkbaren Substitutionen sind möglich. Ganz

besonders bevorzugt ist aber die Gruppe von erfindungsgemäßen Verbindungen, bei denen $R^4$ eine 4-Fluorphenyl-, 4-Chlorphenyl- 3,4-/3,5-Difluorphenyl-oder 3,4-/3,5-Dichlorphenylgruppe bedeutet. Die Zweifachsubstitution umfaßt für $R^4$ zum Beispiel auch eine 4-Fluor-3-chlorphenyl- oder 4-Chlor-3-fluorphenylgruppe.

Weiterhin bevorzugt als Substituent ist die Trifluormethylgruppe. Sie kann in 2-, 3- oder 4-Position, vorzugsweise in 3- und 4-Position, ganz besonders bevorzugt in 4-Position, des Phenylrings gebunden sein.

$R^3$ bedeutet einen unsubstituierten oder ein- bis dreifach substituierten Pyridinring, der über die 2-, 3- oder 4-Position gebunden sein kann, wobei die 2- und 3-Positionen besonders bevorzugt sind. Als Substituenten des Pyridinrings kommen Halogenatome, zum Beispiel Fluor-, Chlor- oder Bromatome, vorzugsweise Bromatome, $C_1$-$C_3$-Alkylgruppen, wie Methyl- oder Ethylgruppen, vorzugsweise Methylgruppen, oder $C_1$-$C_3$-Alkoxygruppen, wie Methoxy -oder Ethoxygruppen, insbesondere Methoxygruppen, in Betracht. Ist der Pyridinring, wie oben als besonders bevorzugt angegeben über die 2-Position verknüpft, dann stehen gegebenenfalls vorhandene Substituenten in 3- und/oder 4-Position des Pyridinrings. $R^5$ bedeutet ein Wasserstoffatom oder eine Methyl- oder Hydroxygruppe, vorzugsweise ein Wasserstoffatom, Z steht für eine Einfachbindung, und p hat den Wert 2 oder 3, besonders bevorzugt den Wert 2. X stellt ein Wasserstoffatom dar, R' ein Wasserstoffatom oder eine Methylgruppe, vorzugsweise ein Wassertoffatom, während m den Wert 2 oder 3, insbesondere den Wert 3, hat.

Bei einer weiteren bevorzugten Gruppe von erfindungsgemäßen Verbindungen steht in der allgemeinen Formel I R für die Gruppierung

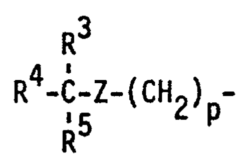

wobei $R^3$ für einen unsubstituierten oder ein- bis dreifach substituierten Pyridinring steht und $R^4$ und $R^5$ jeweils für ein Wasserstoffatom stehen. Der Pyridinring ist vorzugsweise unsubstituiert, kann aber auch durch Halogenatome, wie Fluor-, Chlor- oder Bromatome, $C_1$-$C_3$-Aklygruppen, wie Methyl-, Ethyl- oder Propylgruppen, oder $C_1$-$C_3$-Alkoxygruppen, wie Methoxy-, Ethoxy- oder Propoxygruppen, ein- bis dreifach substituiert sein.

Z steht für eine Einfachbindung, p für den Wert 2 oder 3, wobei der Wert 3 bevorzugt ist. X und R' bedeuten jeweils ein Wasserstoffatom, während m vorzugsweise den Wert 3 hat.

Bei einer weiteren bevorzugten Gruppe von erfindungsgemäßen Verbindungen steht in der allgemeinen Formel I R für die Gruppierung

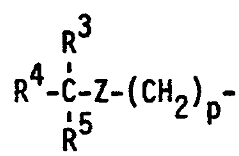

wobei $R^3$ und $R^4$, die gleich oder verschieden sein können, jeweils unabhänging voneinander für einen unsubstituierten oder ein- bis dreifach substituierten Phenylring stehen. Die Substituenten des Phenylrings, die bevorzugt in 3- oder 4-Position, insbesondere in 4-Position, stehen, können Halogenatome, wie Fluor- oder Chloratome, $C_1$-$C_3$-Alkylgruppen, wie zum Beispiel Methyl-, Ethyl- oder Propylgruppen, oder $C_1$-$C_3$-Alkoxygruppen, bevorzugt Methoxygruppen, sein. $R^5$ steht bei dieser bevorzugten Gruppe von erfindungsgemäßen Verbindungen für ein Wasserstoffatom, Z bedeutet ein Sauerstoffatom, und p kann den Wert 2 oder 3, bevorzugt 2, annehmen. X und R' bedeuten vorzugsweise ein Wasserstoffatom, während für m der Wert 3 bevorzugt wird.

Erfindungsgemäße Verbindungen der allgemeinen Formel I, bei denen R, R' und m wie oben definiert sind und X für eine Benzoylgruppe steht, können nach zwei verschiedenen Verfahrensvarianten hergestellt werden, nämlich

$a_1$) durch Umsetzung einer Verbindung der allgemeinen Formel II

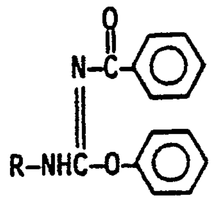

(II)

in der R die oben angegebene Bedeutung besitzt, mit einer Verbindung der allgemeinen Formel III

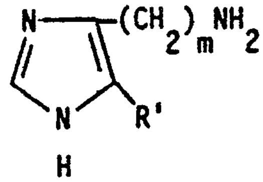

(III)

in der R' und m die oben angegebenen Bedeutungen besitzen, zu einer Verbindung der allgemeinen Formel I.

Die Umsetzung der Reaktanten erfolgt vorzugsweise in äquimolaren Mengen und in einem polaren Lösungsmittel, wie zum Beispiele einem Alkohol, wie Methanol, Ethanol oder Isopropanol, vorzugsweise Ethanol, oder in Acetonitril, Dimethylsulfoxid, Dimethylformamid oder Pyridin, vorzugsweise in Acetonitril, bei Temperaturen von Raumtemperatur bis zur Rückflußtemperatur des verwendeten Lösungsmittels.

$a_2$) oder durch Umsetzung einer Verbindung der allgemeinen Formel IV

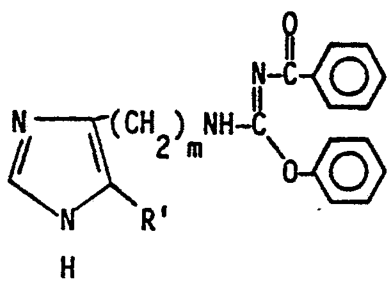

(IV)

in der R' und m die oben angegebenen Bedeutungen besitzen, mit einer Verbindung der allgemeinen Formel V

$R\text{-}NH_2$ (V)

in der R wie oben definiert ist, zu einer Verbindung der allgemeinen Formel I.

Die verwendeten Mengen und Lösungsmittel sowie Reaktionsbedingungen sind die gleichen wie oben im Zusammenhang mit der Verfahrensvariante $a_1$) beschrieben.

Erfindungsgemäße Verbindungen der allgemeinen Formel I, bei denen R, R' und m wie oben definiert sind und X für ein Wasserstoffatom stehen, können nach einer der folgenden vier Verfahrensvarianten hergestellt werden:

$b_1$) durch Hydrolyse einer Verbindung der allgemeinen Formel Ia

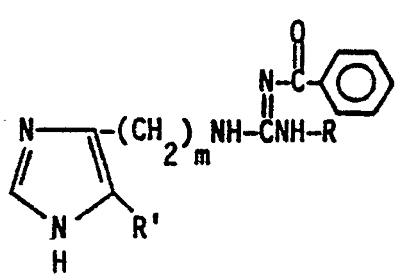

(Ia)

in der R, R' und m wie oben definiert sind.
Die Hydrolyse kann sauer oder basisch durchgeführt werden, wobei eine saure Hydrolyse, zum Beispiel unter Verwendung von verdünnter Schwefelsäure oder verdünnter Salzsäure, insbesondere Salzsäure, bevorzugt wird. Die Hydrolyse wird bei erhöhter Temperatur, vorzugsweise bei Rückflußtemperatur, durchgeführt.
$b_2$) durch Hydrolyse einer Verbindung der allgemeinen Formel VI,

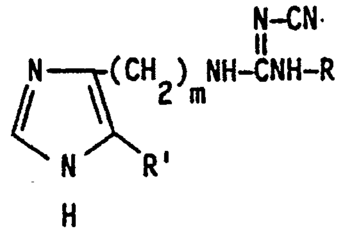

(VI)

in der R, R' und m die oben angegebenen Bedeutungen besitzen, mit Hilfe einer Säure, zum Beispiel verdünnter Schwefelsäure oder verdünnter Salzsäure, vorzugsweise Salzsäure, wie oben angegeben zu einer Verbindung der Formel I.
$b_3$) durch Umsetzung einer Verbindung der allgemeinen Formel VII

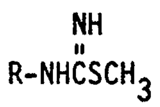

(VII)

in der R die oben angegebene Bedeutung besitzt, mit einer Verbindung der allgemeinen Formel III

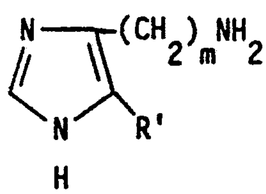

(III)

in der R' und m die oben angegebenen Bedeutungen haben, zu einer Verbindung der allgemeinen Formel I.
Die Umsetzung findet in einem polaren Lösungsmittel, wie Pyridin oder Acetonitril, vorzugsweise in Acetonitril, und bei Rückflußtemperatur des verwendeten Lösungsmittels statt.
$b_4$) durch Umsetzung einer Verbindung der allgemeinen Formel VIII

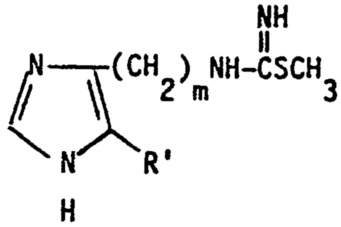

(VIII)

in der R' und m wie oben definiert sind, mit einer Verbindung der allgemeinen Formel V

$R-NH_2$ (V)

in der R die oben angegebene Bedeutung besitzt, zu einer Verbindung der allgemeinen Formel I.
Auch hierbei wird die Umsetzung in einem polaren Lösungsmittel, wie Pyridin oder Acetonitril, vorzugsweise Acetonitril, und bei Rückflußtemperatur des verwendeten Lösungsmittels durchgeführt.

Die nach den einzelnen Verfahrensvarianten erhaltenen Verbindungen werden in üblicher Weise isoliert und gereinigt, beispielsweise durch chromatographische Arbeitsweisen, Umkristallisation etc.

Die bei den einzelnen Verfahrensvarianten erhaltenen Verbindungen können gegebenenfalls in ihr physiologisch annehmbares Salz umgewandelt werden.

Die Erfindung umfaßt neben den stereoisomeren Verbindungen und Hydraten der Substanzen der allgemeinen Formel I daher auch die physiologisch annehmbaren Salze dieser Verbindung. Diese Salze können zum Beispiel mit Mineralsäuren, wie Chlor-, Brom- oder Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich durch eine neuartige, bisher nicht bekannte und beschriebene pharmakologische Gesamtaktivität aus. Die erfindungsgemäße neue Strukturklasse zeigt sowohl eine $H_1$-antagonistische als auch eine $H_2$-agonistische Wirkkomponente. Dies zeigen die folgenden pharmakologischen Ergebnisse. Eine anerkannte Methode zur Bestimmung $H_1$-antagonistischer Wirksamkeit ist die Ermittlung der $pA_2$-Werte in vitro (Arunlakshana, O. u. Schild, H.O. (1959), "Some Quantitative Uses of Drug Antagonists", Br.J.Pharmac.Chemother. 14, 48-58). Zur Bestimmung der $H_2$-agonistischen Aktivität ($pD_2$-Werte) wird die Methode nach van Rossum, J.M. (1963), "Cumulative Dose-Response Curves. II. Technique for the Making of Dose-Response Curves in Isolated Organs and the Evaluation of Drug Parameters", Arch.Intern.Pharmacodyn. Therap. 143, 299-307, herangezogen.

| Pharmakologische Daten (ermittelt am isolierten Atrium bzw. Ileum des Meerschweinchens) | | |
|---|---|---|
| | $pD_2$-Wert (Atrium) | $pA_2$-Wert (Ileum) |
| Beispiel 2 | 6,95 | 6,51 |
| Beispiel 3 | 5,67 | 8,60 |
| Beispiel 4 | 6,36 | 8,04 |
| Beispiel 8 | 6,91 | 7,43 |
| Beispiel 14 | 8,20 | 6,94 |
| Beispiel 16 | 7,86 | 6,95 |
| Beispiel 17 | 8,12 | 7,07 |
| Beispiel 18 | 8,05 | 6,55 |
| Beispiel 20 | 7,94 | 7,05 |
| Beispiel 24 | 8,09 | 6,71 |

Beispiel 1

$N^1$-[3-(1H-Imidazol-4-yl)propyl]-$N^2$-[3-(pyridin-2-yl) propyl]-guanidin

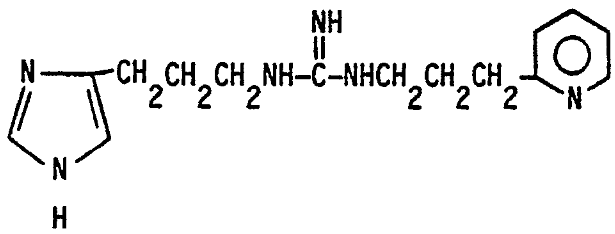

a) $N^1$-Benzoyl-$N^2$-[3-(pyridin-2-yl)propyl]-thioharnstoff

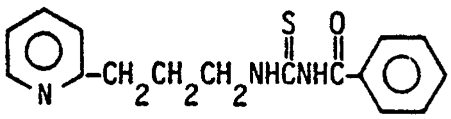

3,30 g (24 mmol) 3-(Pyridin-2-yl)propylamin und 3,95 g (24 mmol) Benzoylisothiocyanat werden in 100 ml Dichlormethan 2 h unter Rückfluß gekocht. Nach Abdampfen des Lösungsmittels i. Vak. wird der Rückstand aus MTBE kristallisiert.
Man erhält 5,16 g (71%) farblose Kristalle vom Schmp. 85 - 86° C.
$C_{15}H_{17}N_3OS$ (299,4)

b) S-Methyl-N-[3-(pyridin-2-yl)propyl]-isothiuroniumjodid

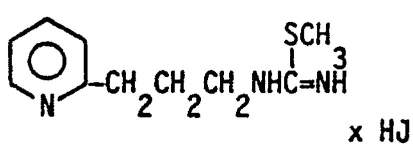

4,50 g (15 mmol) $N^1$-Benzoyl-$N^2$-[3-(pyridin-2-yl)propyl]-thioharnstoff werden in 200 ml Methanol und 60

ml Wasser mit 4,15 g (30 mmol) Kaliumcarbonat 40 Min. gekocht. Nach Abdampfen der Lösungsmittel i. Vak. wird der Rückstand in 20 ml Wasser aufgenommen und die wäßrige Phase viermal mit 30 ml Dichlormethan extrahiert. Die vereinigten organischen Phasen werden mit Natriumsulfat getrocknet, filtriert und i. Vak. eingedampft. Der Rückstand wird in 100 ml Ethanol aufgenommen und mit 1.2 ml (19 mmol) Methyljodid 20 h bei Raumtemperatur gerührt. Nach Abdampfen des Lösungsmittels i. Vak. wird der Rückstand aus MTBE kristallisiert.
3,52 g (69%) farblose Kristalle mit Schmp. 131 = 132° C.
$C_{10}H_{16}JN_3S$ (337,2)

c) N[1]-[3-(1H-Imidazol-4-yl)propyl]-N[2]-[3-(pyridin-2-yl)propyl] -guanidin

1,00 g (3,0 mmol) S-Methyl-N-[3-(pyridin-2-yl)propyl]-isothiuroniumjodid und 0,40 g (3,2 mmol) 3-(1H-Imidazol-4-yl)propylamin werden in 20 ml Acetonitril 3 h unter Rückfluß gekocht. Nach Abdampfen des Lösungsmittels i. Vak. wird der erhaltene Rückstand an Aluminiumoxid (neutral) mit Ethanol/Ethylacetat (1:1) chromatographiert. Die Hauptfraktion ergibt nach Eindampfen i. Vak. 0,75 g (88 %) eines blaßgelben, amorphen Feststoffs.
$C_{15}H_{22}N_6$ (286,4)
$^1$H-NMR-Daten:
(DMSO-$d_6$, TMS als interner Standard)
$\delta$ = 1,6 - 2,2 (m) 4 H.
2,5 (t) 2 H,
2,8 (t) 2 H,
3,1 - 3,5 (m) 4 H,
6,9 (s) 1 H,
7,2 - 8,0 (m) 8 H, 4 H austauschbar mit $D_2O$,
8,6 (dd) 1 H ppm.

Beispiel 2

N[1]-[3-(1H-Imidazol-4-yl)propyl]-N[2]-[4-(pyridin-2-yl)butyl]-guanidin-hydrojodid

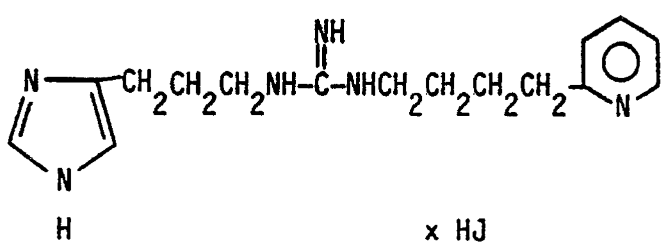

x HJ

a) N[1]-Benzoyl-N[2]-[4-(pyridin-2-yl)butyl]-thioharnstoff

Hergestellt analog zu Beispiel 1 a) aus 17,7 g (118 mmol) 4-(Pyridin-2-yl) butylamin und 19,2 g (118 mmol) Benzoylisothiocyanat. Chromatographische Reinigung des Rohproduktes an Kieselgel mit Dichlormethan/ Methanol (95:5) als Laufmittel ergibt 28,4 g (77%) eines rötlichen, viskosen Öls.
$C_{17}H_{19}N_3OS$ (313,4)

b) S-Methyl-N-[4-(pyridin-2-yl)butyl]-isothiuroniumjodid

Aus N[1]-Benzoyl-N[2]-[4-(pyridin-2-yl)butyl]-thioharnstoff analog zu Beispiel 1 b). Nach Umkristallisieren aus Ethanol/Ethylacetat (1:4) blaßgelbe Nadeln vom Schmp. 105 - 106° C.
$C_{11}H_{18}JN_3S$ (351,2)

c) N[1]-[3-(1H-Imidazol-4-yl)propyl]-N[2]-[4-(pyridin-2-yl)butyl] guanidin-hydrojodid

2,00 g (5,7 mmol) S-Methyl-N-Methyl-N-[4-(pyridin-2-yl)butyl]-isothiuronium-jodid und 0,85 g (6,8 mmol) 3-(1H-Imidazol-4-yl)propylamin werden in 20 ml Acetnitril 2 h gekocht. Nach Abdampfen des Lösungsmittels

i. Vak. wird der Rückstand an Kieselgel mit Dichloromethan/Methanol (8:2) chromatographiert. Die Hauptfraktion liefert nach Eindampfen 1,32 g (54 %) eines gelblichen, amorphen Feststoffs.
$C_{16}H_{25}JN_6$ (428,3)
$^1$H-NMR-Daten:
($CD_3OD$, TMS als interner Standard)
δ = 1.5 - 2,2 (m) 6 H,
2,7 - 3,0 (m) 4 H,
3,2 - 3,5 (m) 4 H
5,0 (breit) 5 H, austauschbar mit $D_2O$,
7,2 (s) 1 H,
7,2 - 8.0 (m) 3 H,
8,3 (s) 1 H,
8,5 (dd) 1 H ppm.

Beispiel 3

N¹-[3-(1H-Imidazol-4-yl)propyl]-N²-[3-[N-(4-chlorbenzyl)-N-(pyridin-2-yl)amino]propyl]guanidin-hydrojodid

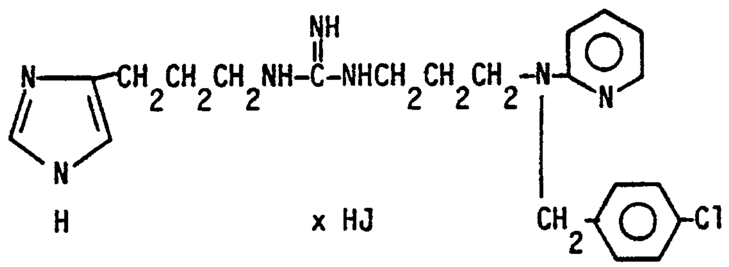

a) N¹-Benzoyl-N²-[3-[4-(4-chlorbenzyl)-N-(pyridin-2-yl)amino]propyl] -thioharnstoff

Hergestellt analog zu Beispiel 1 a) aus 2,76 g (10 mmol) N-(4-Chlorbenzyl)-N-(pyridin-2-yl)-1,3-propandiamin und 1,63 g (10 mmol) Benzoylisothiocyanat. 3,63 g (83 %) blaßgelbes Öl.
$C_{26}H_{23}ClN_4OS$ (483,5)

b) S-Methyl-N-[3-[4-(chlorbenzyl)-N-(pyridin-2-yl)amino]propyl]-isothiuronium-jodid

Erhalten aus N¹-Benzoyl-N²-[3-[N-(4-chlorbenzyl)-N-(pyridin-2-yl) amino]propyl]-thioharnstoff analog zu Beispiel 1 b) als blaßgelber, amorpher Festoff.
$C_{17}H_{22}ClJN_4S$ (680,6)

c) N¹-[3-(1H-Imidazol-4-yl)propyl]-N²-[3-[N-(4-(chlorbenzyl)-N-(pyridin-2-yl)amino]propyl]-guanidin-hydrojodid

1,00 g (2,1 mmol) S-Methyl-N-[3-[N-(4-chlorbenzyl)-N-(pyridin-2-yl)amino] propyl]-isothiuronium-jodid und 0,26 g (2,1 mmol) 3-(1H-Imidazol-4-yl)-propylamin werden in 15 ml Acetonitril 3 h gekocht. Der nach Abdampfen des Lösungsmittels i. Vak. erhaltene Rückstand wird an Kieselgel mit Ethylacetat/Methanol(7:3) als Laufmittel chromatographiert. Man erhält 0,17 g (15 %) eines farblosen, amorphen Feststoffs.
$C_{22}H_{29}ClJN_7$ (553,9)
$^1$H-NMR-Daten:
($CD_3OD$, TMS als interner Standard)
δ = 1,7 - 2,1 (m) 4 H,
2,7 (t) 2 H,
3,1 - 3,4 (m) 4 H
3,6 (t) 2 H,
4,7 (s) 2 H,
4,8 (breit) 5 H, austauschbar mit $D_2O$,
6,6 - 7,7 (m) 8 H,
7,7 (s) 1 H,

8,2 (dd) 1 H ppm.

Beispiel 4

N[1]-[3-(1H-Imidazol-4-yl)propyl]-N[2]-[2-[N-(pyridin-2-yl)-N-(thiophen-2-ylmethyl)amino]ethyl]-guanidin-hydrojodid

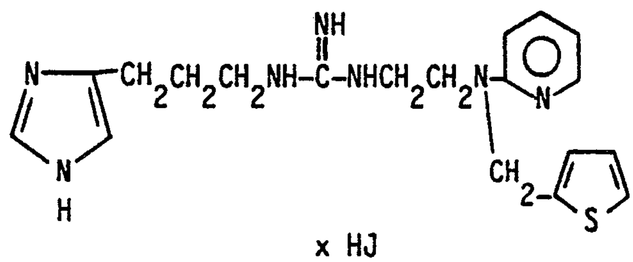

x HJ

a) N[1]-Benzoyl-N[2]-[2-[N-(pyridin-2-yl)-N-(thiophen-2-ylmethyl)amino] ethyl]-thioharnstoff

Hergestellt analog zu Beispeil 1 a) aus 2,00 g (8,6 mmol) N-(Pyridin-2-yl)-N-(thiophen-2-ylmethyl)-ethylendiamin und 1,40 g (8,6 mmol) Benzoylisothiocyanat. Das Rohprodukt wird an Kieselgel mit Dichlormethan/Methanol (99:1) als Laufmittel gereinigt. Man erhält aus der Hauptfraktion nach Eindampfen i. Vak. 2,85 g (84 %) eines blaßgelben, zähen Öls.
$C_{20}H_{20}N_4OS_2$ (396,5)

b) S-Methyl-N-[2-[N-(pyridin-2-yl)-N-(thiophen-2-ylmethyl)amino]ethyl]-isothiuronium-jodid

Aus 2,00 g (5 mmol) N[1]-Benzoyl-N[2]-[2-[N-(pyridin-2-yl)-N-(thiophen-2-ylmelthyl)amino]ethyl] thioharnstoff werden analog zu Beispiel 1 b) 1,84 g (85 %) eines farblosen, amorphen Feststoffs erhalten.
$C_{14}H_{19}JN_4S_2$ (434,4)

c) N[1]-[3-(1H-Imidazol-4-yl)propyl]-N[2]-[2-[N-(pyridin-2-yl)-N-(thiophen-2-ylmethyl)amino]ethyl]-guanidin-hydrojodid

1,00 g (2,3 mmol) S-Methyl-N-[2-[N-(pyridin-2-yl)-N-(thiophen-2-ylmethyl) amino]ethyl]-isothiuronium-jodid werden analog zu Beispiel 1 c) mit 0,29 g (2,3 mmol) 3-(1H-Imidazol-4-yl)propylamin umgesetzt. Das Rohprodukt wird durch Chromatographie an Kieselgel mit Ethylacetat/Methanol (80:20) gereinigt. Es werden 0,31 g (26 %) der Titelverbindung in Form eines farblosen, amorphen Feststoffs erhalten.
$C_{19}H_{26}JN_7S$ (511,4)
$^1$H-NMR-Daten:
($CD_3OD$, TMS als interner Standard)
$\delta$ = 1,8 - 2,1 (m) 2 H,
2,7 (t) 2 H,
3,2 - 3,9 (m) 6 H
4,8 (breit) 5 H, austauschbar mit $D_2O$
4.9 (s) 2 H,
6,6 - 7,8 (m) 8 H,
8,2 (dd) 1 H ppm.

Beispiel 5

N[1]-[3-(1H-Imidazol-4-yl)propyl]-N[2]-[2-[N-phenyl-N-(thiophen-2-yl-methyl)amino]ethyl]-guanidin

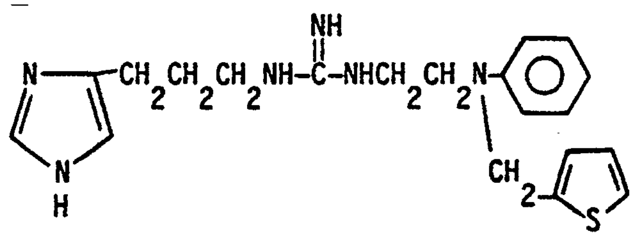

a) $N^1$-Benzoyl-$N^2$-[2-[N-phenyl-N-(thiophen-2-ylmethyl)amino]ethyl] thioharnstoff

Hergestellt analog zu Beispiel 1 a) aus 7.13 g (31 mmol) N-Phenyl-N-(thiophen-2-ylmethyl)-ethylendiamin und 5.00 g (31 mmol) Benzoylisothiocyanat. 10,64 g (88 %) farblose Kristalle vom Schmp. 108 - 109° C.
$C_{21}H_{21}N_3OS_2$ (395,5)

b) S-Methyl-N-[2-[N-phenyl-N-(thiophen-2-ylmethyl)amino]ethyl]-isothiuronium-jodid

Erhalten analog zu Beispiel 1 b) aus 8,0 g (20 mmol) $N^1$-Benzoyl-$N^2$-[2-[N-phenyl-N-(thiophen-2-ylmethyl)amino]ethyl]-thioharnstoff. 8.0 g (92%) beige Kristalle vom Schmp. 127 - 128° C aus Aceton/Diethylether.
$C_{15}H_{20}JN_3S_2$ (433,4)

c) $N^1$-[3-(1H-Imidazol-4-yl)propyl]-$N^2$-[2-[N-phenyl-N-(thiophen-2-ylmethyl)amino]ethyl]-guanidin

Aus 1,00 g (2,3 mmol) S-Methyl-N-[2-[N-phenyl-N-(thiophen-2-ylmethyl)amino] ethyl] isothiuronium-jodid und 0,30 g (2,4 mmol) 3-(1H-Imidazol-4-yl)propylamin. Nach chromatographischer Reinigung an Aluminiumoxid (neutral) mit Ethanol/Ethylacetat (1:1) als Laufmittel 0,35 g (40%) eines farblosen, amorphen Feststoffs.
$C_{20}H_{26}N_6S$ (382,5)
$^1$H-NMR-Daten:
($CD_3OD$, TMS als interner Standard)
$\delta$ = 1,6 - 2,0 (m) 2 H,
2,6 (t) 2 H,
3,2 (t) 2 H,
3,4 - 3,7 (m) 4 H,
4,7 (s) 2 H,
5,3 (breit) 4 H, austauschbar mit $D_2O$,
6,6 - 7,4 (m) 9 H,
7,6 (s) 1 H ppm.

Beispiel 6

$N^1$-[3-(1H-Imidazol-4-yl)propyl]-$N^2$-[3-(pyridin-2-ylamino)-propyl]-guanidin-hydrojodid

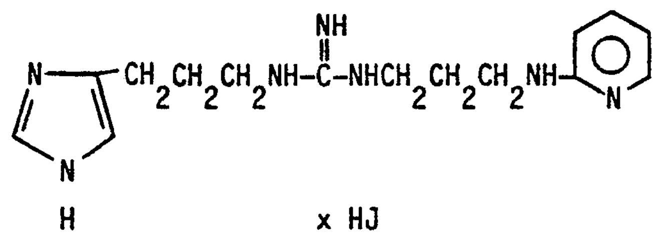

a) $N^1$-Benzoyl-$N^2$-[3-(pyridin-2-ylamino)propyl]-thioharnstoff

Erhalten analog zu Beispiel 1 a) aus 1,0 g (6,6 mmol) N-(Pyridin-2-yl)-1,3-propandiamin und 1,1 g (6,6 mmol) Benzoylisothiocyanat. Nach Umkristallisieren aus Ethylacetat 2,0 g (96 %) farblose Kristalle vom Schmelzpunkt 111 ° C.
$C_{16}H_{18}N_4OS$ (314,4)

b) S-Methyl-N-[3-(pyridin-2-ylamino)propyl]-isothiuroniumjodid

Aus 1,8 g (5,7 mmol) N[1]-Benzoyl-N[2]-[3-(pyridin-2-ylamino)-propyl] -thioharnstoff werden entsprechend Beispiel 1 b) 1,1 g (55 %) eines farblosen, hochviskosen Öls erhalten.
$C_{10}H_{17}JN_4S$ (352,2)

c) N[1]-[3-(1H-Imidazol-4-yl)propyl]-N[2]-[3-(pyridin-2-ylamino)propyl]-guanidin-hydrojodid

1,00 g (2,8 mmol) S-Methyl-N-[3-(pyridin-2-ylamino)propyl]-isothiuroniumjodid und 0,36 g (2,8 mmol) 3-(1H-Imidazol-4-yl)propylamin werden in 30 ml Acetonitril 3 h gekocht. Das nach Abdampfen des Lösungsmittels i. Vak. erhaltene Rohprodukt wird an Kieselgel mit Ethylacetat/Methanol/konz. Ammoniak (80:19:1) chromatographiert. Aus der Hauptfraktion erhält man 0,64 g (53 %) eines blaßgelben, amorphen Feststoffs.
$C_{15}H_{24}JN_7$ (429,3)
$^1$H-DMR-Daten:
($CD_3OD$, TMS als interner Standard)
$\delta$ = 1,7 - 2,1 (m) 4 H,
2,7 (t) 2 H,
3,1 - 3,5 (m) 6 H,
4,9 (breit) 6 H, austauschbar mit $D_2O$,
6,5 - 6,7 (m) 2 H,
6,9 (s) 1 H,
7,3 - 7,5 (m) 1 H,
7,6 (s) 1 H,
7,9 (dd) 1 H ppm.

Beispiel 7

N[1]-[3-(1H-Imidazol-4-yl)propyl]-N[2]-[3-[N-phenyl-N-(pyridin-2-yl)-amino]propyl]-guanidin-hydrojodid

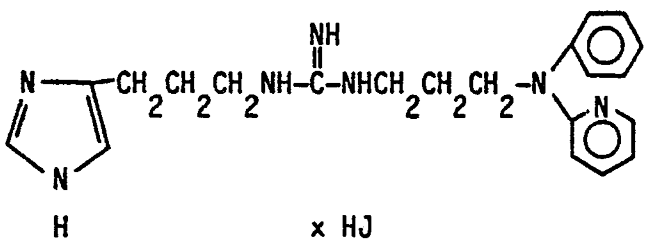

a) N[1]-Benzoyl-N[2]-[3-[N-phenyl-N-(pyridin-2-yl)-amino]propyl]-thioharnstoff

Aus 3,1 g (13,7 mmol) N-phenyl-N-(pyridin-2-yl)-1,3-propandiamin und 2,2 g (13,7 mmol) Benzoylisothiocyanat werden analog zu Beispiel 1 a) 3,7 g (69 %) farblose Kristalle vom Schmp. 124 - 126 ° C (aus Methanol) erhalten.
$C_{22}H_{22}N_4OS$ (390,5)

b) S-Methyl-N-[3-[N-phenyl-N-(pyridin-2-yl)-amino]propyl]-isothiuroniumjodid

Erhalten analog zu Beispiel 1 b) aus 2,70 g (6,9 mmol) N[1]-Benzoyl-N[2]-[3-[N-phenyl-N-(pyridin-2-yl)-amino]propyl]-thioharnstoff. Nach Umkristallisieren aus Ethylacetat 2,61 g (88 %) farblose Kristalle vom Schmp. 130 - 131 ° C.
$C_{16}H_{21}JN_4S$ (428,3)

c) N[1]-[3-(1H-Imidazol-4-yl)propyl]-N[2]-[3-[N-phenyl-N-(pyridin-2-yl)-amino]propyl]-guanidin-hydrojodid

Hergestellt analog zu Beispiel 6 c) aus 1,00 g (2,3 mmol) S-Methyl-N-[3-[N-phenyl-N-(pyridin-2-yl)-amino]propyl]-isothiuronium-jodid und 0,29 g (2,3 mmol) 3-(1H-Imidazol-4-yl)propylamin. 0,78 g (67 %) farbloser, amorpher Feststoff.
$C_{21}H_{28}JN_7$ (505,4)
$^1$H-NMR-Daten:
($CD_3OD$, TMS als interner Standard)
$\delta$ = 1,7 - 2,1 (m) 4 H,
2,7 (t) 2 H,
3,1 - 3,4 (m) 4 H,
4,0 (t) 2 H,
4,8 (breit) 5 H, austauschbar mit $D_2O$,
6,3 - 6,8 (m) 2 H,
6,9 (s) 1 H,
7,2 - 7,7 (m) 7 H,
8,1 (dd) 1 H ppm.

Beispiel 8

N[1]-[3-(1H-Imidazol-4-yl)propyl]-N[2]-(3,3-diphenylbutyl)-guanidin-hydrochlorid

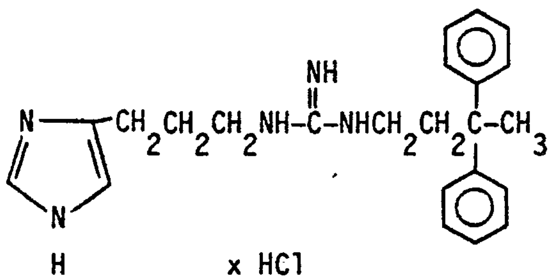

a) N[1]-Benzoyl-N[2]-[3-(1H-imidazol-4-yl)propyl]-N[3]-(3,3-diphenylbutyl)-guanidin

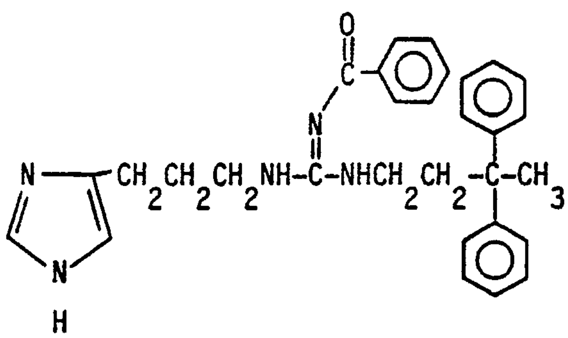

0,70 g (3,1 mmol) 3,3-Diphenyl-butylamin und 1,08 (3.1 mmol) O-Phenyl-N[1]-benzoyl-N[2]-[3-(1H-imidazol-4-yl)propyl]-isoharnstoff werden in 10 ml Ethanol 15 h unter Rückfluß gekocht. Die Lösung wird i. Vak eingedampft und der erhaltene Rückstand an Kieselgel mit Dichlormethan/Methanol (9:1) als Laufmittel chromatographiert. Die Hauptfraktion ergibt nach Eindampften i.Vak. 1,05 g (71 %) eines farblosen, amorphen Feststoffs.
$C_{30}H_{33}N_5O$ (479,6)

b) N[1]-[3-(1H-Imidazol-4-yl)propyl]-N[2]-(3,3-diphenylbutyl)-guanidin-hydrochlorid

1,00 g (2,1 mmol) N[1]-Benzoyl-N[2]-[3-(1H-imidazol-4-yl)propyl]-N[3]-(3,3-diphenylbutyl)-guanidin werden mit 20 ml konz. Salzsäure 20 h unter Rückfluß gekocht. Nach weitgehendem Einengen i. Vak. wird der Rückstand mit 30 ml Wasser verdünnt. Die Lösung wird 5 x mit 20 ml Diethylether extrahiert. Die wäßrige Phase wird dann filtriert, i. Vak. bis 50° C eingedampft und der Rückstand noch zweimal mit 10 ml abs. Ethanol eingedampft. Das erhaltene Rohprodukt wird an Kieselgel mit Dichlormethan/Methanol (80:20) als Laufmittel chromatographiert. Die Hauptfraktion ergibt nach Eindampfen i. Vak. 0,53 g (61 %) eines farblosen, amorphen Feststoffs.
$C_{23}H_{30}ClN_5$ (412,0)
$^1$H-NMR-Daten:
($CD_3OD$, TMS als interner Standard)
$\delta$ = 1,7 (s) 3 H,
1,7 - 2,0 (m) 2 H,
2,3 - 2,8 (m) 4 H,
2,9 - 3,4 (m) 4 H,
4,8 (breit) 5 H, austauschbar mit $D_2O$,
6,95 (s) 1 H,
7,1 - 7,4 (m) 10 H,
7,9 (s) 1 H ppm.

Beispiel 9

N[1]-[3-(1H-Imidazol-4-yl)propyl]-N[2]-[3-(4-fluorphenyl)-3-phenyl-butyl -guanidin-hydrojodid

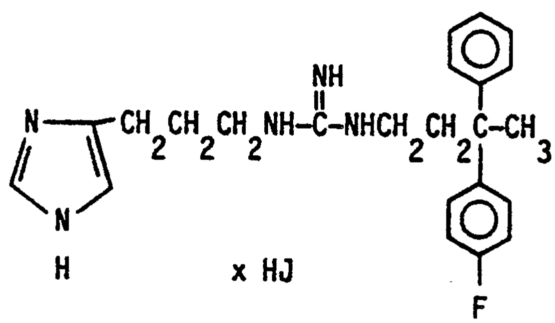

a) N[1]-Benzoyl-N[2]-[3-(4-fluorphenyl)-3-phenyl-butyl]-thioharnstoff

Erhalten aus 0,54 g (2,2 mmol) 3-(4-Fluorphenyl)-3-phenyl-butylamin und 0,36 g (2,2 mmol) Benzoylisothiocyanat analog zu Beispiel 1 a). Das Rohprodukt wird ohne weitere Reinigung weiter umgesetzt.
$C_{24}H_{23}FN_2OS$ (406,5)

b) S-Methyl-N-[3-(4-fluorphenyl)-3-phenyl-butyl]-isothiuronium-jodid

Hergestellt entsprechend Beispiel 1 b) aus 0,90 g (2,2 mmol) N[1]-Benzoyl-N[2]-[3-(4-fluorphenyl)-3-phenylbutyl]-thioharnstoff. 0,92 g (95 %) gelblicher, amorpher Feststoff.
$C_{18}H_{22}FJN_2S$ (444,3)

c) N[1]-[3-(1H-Imidazol-4-yl)propyl]-N[2]-[3-(4-fluorphenyl)-3-phenyl-butyl]-guanidin-hydrojodid

0,92 g (2,1 mmol) S-Methyl-N-[3-(4-fluorphenyl)-3-phenyl-butyl]-isothiuronium-jodid und 0,28 g (2,2 mmol) 3-(1H-Imidazol-4-yl)propylamin werden in 10 ml Acetonitril 3 h gekocht. Chromatographische Reinigung des Rohproduktes an Kieselgel mit Dichlormethan/Methanol (80:20) als Laufmittel ergibt 0,70 g (64%) eines blaßgelben, amorphen Feststoffs.
$C_{23}H_{29}FJN_5$ (521,4)
$^1$H-NMR-Daten:

($CD_3OD$, TMS als interner Standard)
$\delta$ = 1,7 (s) 3 H,
1,7 - 2,1 (m) 2 H,
2,3 - 2,8 (m) 4 H,
2,9 - 3,4 (m) 4 H,
4,8 (breit) 5 H, austauschbar mit $D_2O$,
6,9 - 7,5 (m) 10 H,
7,7 (s) 1 H ppm.

Beispiel 10

N[1]-[3-(1H-Imidazol-4-yl)propyl]-N[2]-[3,3-bis(4-fluorphenyl)-butyl] -guanidin-hydrojodid

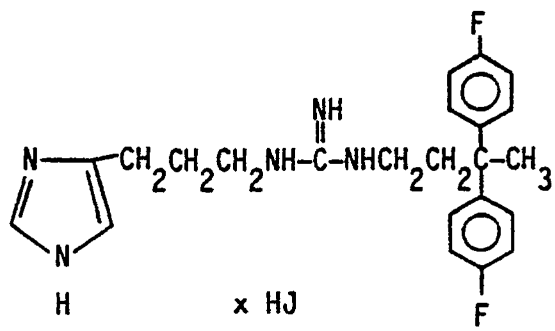

a) N[1]-Benzoyl-N[2]-[3,3-bis(4-fluorphenyl)butyl]-thioharnstoff

Hergestellt aus 2,4 g (9 mmol) 3,3-Bis(4-fluorphenyl)-butylamin und 1,5 g (9 mmol) Benzoylisothiocyanat nach Beispiel 1 a). Nach chromatographischer Reinigung an Kieselgel mit Dichloromethan als Laufmittel. 3,3 g (85 %) eines orangegelben Öls.
$C_{24}H_{22}F_2N_2OS$ (424,5)

b) S-Methyl-N-[3,3-bis(4-fluorphenyl)butyl]-isothiuronium-jodid

Aus 3,3 g (7,8 mmol) N[1]-Benzoyl-N[2]-[3,3-bis(4-fluorphenyl)butyl] -thioharnstoff erhält man analog zu Beispiel 1 b) 2,55 g (71 %) der Titelverbindung als orangegelben, amorphen Feststoff.
$C_{18}H_{21}F_2JN_2S$ (462,3)

c) N[1]-[3-(1H-Imidazol-4-yl)propyl]-N[2]-[3,3-bis(4-fluorphenyl)-butyl]-guanidin-hydrojodid

Hergestellt analog zu Beispiel 1 c) aus 2,55 g (5,5 mmol) S-Methyl-N-[3,3-bis(4-fluorphenyl)butyl]-isothiuronium-jodid und 0,70 g (5,6 mmol) 3-(1H-Imidazol-4-yl)-propylamin. Man erhält 1,61 g (54 %) eines gelben, amorphen Feststoffs.
$C_{23}H_{28}F_2JN_5$ (539,4)
$^1$H-NMR-Daten:
($CD_3OD$, TMS als interner Standard)
$\delta$ = 1,7 (s) 3 H,
1,7 - 2,1 (m) 2 H,
2,4 - 2,9 (m) 4 H,
3,0 - 3,4 (m) 4 H,
4,9 (breit) 5 H, austauschbar mit $D_2O$
6,9 - 7,4 (m) 9 H,
8,1 (s) 1 H ppm.

Beispiel 11

N[1]-[3-(1H-Imidazol-4-yl)propyl]-N[2]-[3-(4-fluorphenyl)-3-(5-brom-3-methyl-pyridin-2-yl)-propyl]-guanidin-

hydrojodid

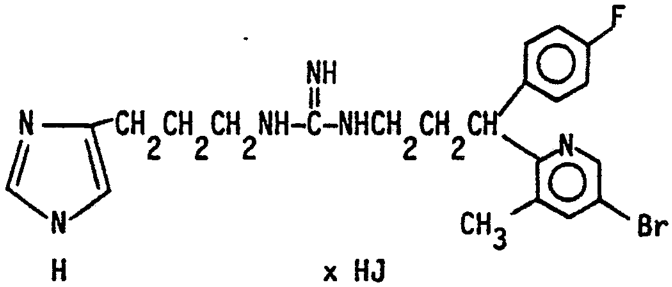

x HJ

a) $N^1$-Benzoyl-$N^2$-[3-(4-fluorphenyl)-3-(5-brom-3-methyl-pyridin-2-yl) propyl]-thioharnstoff

Hergestellt aus 0,78 g (2,4 mmol) 3-(4-Fluorphenyl)-3-(5-brom-3-methyl-pyridin-2-yl)-propylamin und 0,39 g (2,4 mmol) Benzoylisothiocyanat analog zu Beispiel 1 a). Das Rohprodukt wird chromatographisch an Kieselgel mit Dichlormethan als Laufmittel gereinigt und ergibt 1,01 g (86 %) eines blaßgelben, hochviskosen Öls.
$C_{23}H_{21}BrFN_3OS$ (486,4)

b) S-Methyl-N-[3-(4-fluorphenyl)-3-(5-brom-3-methyl-pyridin-2-yl)propyl]-isothiuronium-jodid

Aus 1,00 g (2 mmol) $N^1$-Benzoyl-$N^2$-[3-(4-fluorphenyl)-3-(5-brom-3-methyl-pyridin-2-yl)propyl] thioharnstoff erhält man analog zu Beispiel 1 b) 0,95 g (91 %) eines farblosen, nicht kristallinen Feststoffs.
$C_{17}H_{20}BrFJN_3S$ (524,2)

c) $N^1$-[3-(1H-Imidazol-4-yl)propyl]-$N^2$-[3-(4-fluorphenyl)-3-(5-brom-3-methyl-pyridin-2-yl)propyl]-guanidin-hydrojodid

0,70 g (1,33 mmol) S-Methyl-N-[3-(4-fluorphenyl)-3-(5-brom-3-methyl-pyridin-2-yl)propyl]-isothiuronium-jodid und 0,18 g (1,43 mmol) 3-(1H-Imidazol-4-yl)-propylamin werden in 10 ml n-Butanol 3 h under Rückfluß gekocht. Das Lösungsmittel wird i. Vak. abgedampft und das erhaltene braune Rohprodukt an Kieselgel mit Ethylacetat/Ethanol (60:40) chromatographisch gereinigt. Man erhält 0.48 g (60 %) eines farblosen, amorphen Feststoffs.
$C_{22}H_{27}BrFJN_6$ (601,3)
$^1$H-NMR-Daten:
($CD_3OD$, TMS als interner Standard)
$\delta$ = 1,7 - 2,9 (m) 6 H,
2,3 (s) 3H,
3,1 - 3,5 (m) 4 H,
4,5 (t) 1 H,
5,0 (breit) 5 H, austauschbar mit $D_2O$,
7,0 - 7,6 (m) 5 H,
7,8 (s) 1 H,
7,9 (d) 1 H,
8,7 (d) 1 H ppm.

Beispiel 12

$N^1$-[2-(5-Methyl-1H-imidazol-4-yl)ethyl]-$N^2$-[3-(4-fluorphenyl) -3-(pyridin-2-yl)propyl]-guanidin

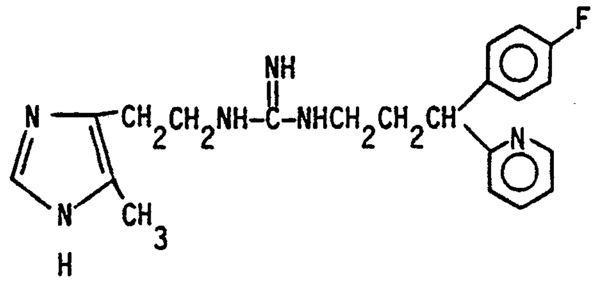

a) $N^1$-Benzoyl-$N^2$-[3-(4-fluorphenyl)3-(pyridin-2-yl)propyl] -thioharnstoff

Hergestellt analog zu Beispiel 1 a) aus 11,5 g (50 mmol) 3-(4-Fluorphenyl)-3-(pyridin-2-yl)propylamin und 8,2 g (50 mmol) Benzoylisothiocyanat in Chloroform. Nach Umkristallisieren aus i-Propanol 18,1 g (92 %) farblose Kristalle vom Schmelzpunkt 94 - 96° C.
$C_{22}H_{20}FN_3OS$ (393,5)

b) S-Methyl-N-[3-(4-fluorphenyl)-3-(pyridin-2-yl)propyl]-isothiuroniumjodid

Aus 11,8 g (30 mmol) $N^1$-Benzoyl-$N^2$-[3-(4-fluorphenyl)-3-(pyridin-2-yl)propyl]-thioharnstoff werden analog zu Beispiel 1 b) 10,4 g (80 %) eines farblosen, amorphen Feststoffs erhalten, der nach mehrmaligem Verrühren mit Diethylether bei 80° C unscharf schmilzt.
$C_{16}H_{19}FJN_3S$ (431,3)

c) $N^1$-[2-(5-Methyl-1H-imidazol-4-yl)ethyl]-$N^2$-[3-(4-fluorphenyl)-3-(pyridin-2-yl)propyl]-guanidin

1,38 g (3,2 mmol) S-Methyl-N-[3-(4-fluorphenyl)-3-(pyridin-2-yl)propyl]-isothiuronium-jodid und 0,40 g (3,2 mmol) 2-(5-Methyl-1H-imidazol-4-yl)ethylamin werden in 20 ml Acetonitril 3 h unter Rückfluß gekocht. Nach Abdampfen des Lösungsmittels i. Vak. wird das Rohprodukt an Kieselgel mit Ethylacetat/Methanol/konz. Ammoniak (50:47:3) als Lauftmittel chromatographiert. Die Hauptfraktion ergibt nach Eindampfen i.Vak. 0,40 g (33 %) eines gelblichen, amorphen Feststoffs.
$C_{21}H_{25}FN_6$ (380,5)
$^1$H-NMR-Daten:
($CD_3OD$, TMS als interner Standard)
$\delta$ = 2,2 (s) 3 H,
2,2 - 2,6 (m) 2 H,
2,8 (t) 2 H,
3,1 - 3,6 (m) 4 H,
4,3 (t) 1 H,
5,0 (breit) 4 H, austauschbar mit $D_2O$,
6,9 - 8,0 (m) 8 H,
8,6 (dd) 1 H ppm.

Beispiel 13

$N^1$-[3-(1H-Imidazol-4-yl)propyl]-$N^2$ -[2-(diphenylmethoxy)ethyl] -guanidin-hydrojodid

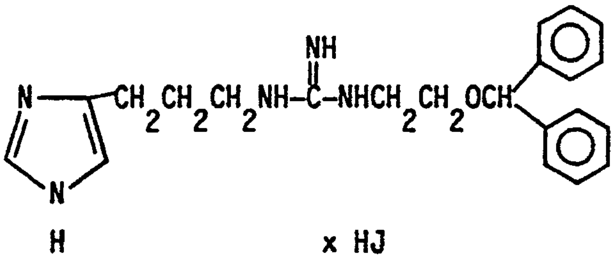

a) N[1]-Benzoyl-N[2]-[2-(diphenylmethoxy)ethyl]-thioharnstoff

7,8 g (34 mmol) 2-(Diphenylmethoxy)-ethylamin und 5,6 g (34 mmol) Benzoylisothiocyanat werden in 60 ml Ethylacetat 2 h bei Raumtemperatur gerührt. Der ausgefallene Feststoff wird abgesaugt, mit etwas Ethylacetat gewaschen und aus Ethanol umkristallisiert. Man erhält 11,1 g (83 %) farblose Kristalle vom Schmp. 126 - 127 ° C.
$C_{23}H_{22}N_2O_2S$ (390,5)

b) S-Methyl-N-[2-(diphenylmethoxy)ethyl]-isothiuronium-jodid

Hergestellt analog zu Beispiel 1 b) aus 11,1 g (28 mmol) N[1]-Benzoyl-N[2]-[2-(diphenylmethoxy)ethyl]-thioharnstoff. Man erhält 11,4 g (94 %) eines farblosen, hochviskosen Öls.
$C_{17}H_{21}JN_2OS$ (428,3)

c) N[1]-[3-(1H-Imidazol-4-yl)propyl]-N[2]-[2-(diphenylmethoxy)ethyl] -guanidin-hydrojodid

Hergestellt analog zu Beispiel 1 c) aus 1,73 g (4 mmol) S-Methyl-N-[2-(diphenylmethoxy)ethyl]-isothiuronium-jodid und 0,50 g (4 mmol) 3-(1H-Imidazol-4-yl)propylamin. Nach chromatographischer Reinigung an Kieselgel mit Dichlormethan/Methanol (80:20) als Laufmittel 1,41 g (70 %) eines farblosen, amorphen Feststoffs.
$C_{22}H_{28}JN_5O$ (505,4)
$^1$H-NMR-Daten:
($CD_3OD$, TMS als interner Standard)
$\delta$ = 1,7 - 2,1 (m) 2 H,
2,7 (t) 2 H,
3,1 - 3,8 (m) 6 H,
4,9 (breit) 5 H, austauschbar mit $D_2O$,
5,6 (s) 1 H,
7,0 (s) 1 H,
7,2 - 7,6 (m) 10 H,
8,0 (s) 1 H, ppm.

Beispiel 14

N[1]-[3-(3,4-Dichlorphenyl)-3-(pyridin-2-yl)propyl]-N[2]-[3-(1H-imidazol-4-yl)propyl]guanidin

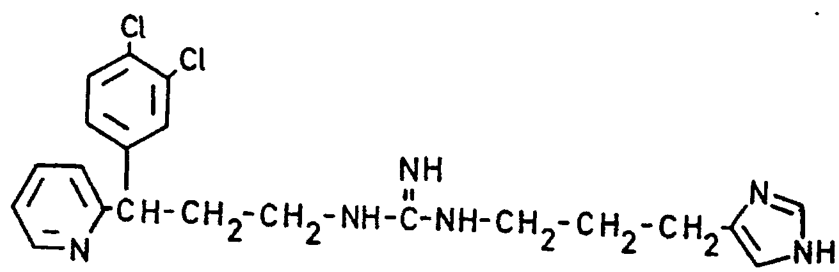

a) N[1]-Benzoyl-N[2]-[3-(3,4-dichlorphenyl)-3-(pyridin-2-yl)propyl]-N[3]-[3-(1H-imidazol-4-yl)propyl]guanidin

1,41 g (5 mmol) 3-(3,4-Dichlorphenyl)-3-(pyridin-2-yl)propylamin und 1,59 g (5 mmol) N-Benzoyldiphenylimidocarbonat werden in 20 ml Methylenchlorid 20 min bei Raumtemperatur gerührt. Anschließend wird das Lösungsmittel 1. Vak. abdestilliert, der ölige Rückstand in 30 ml Pyridin aufgenommen und nach Zusatz von 0,65 g (5,2 mmol) 3-(1H-Imidazol-4-yl)propylamin 45 min auf 100 ° C erhitzt. Der Reaktionsansatz wird i. Vak. eingedampft, der Rückstand in 5prozentiger Salzsäure aufgenommen und mit Ether extrahiert. Anschließend wird mit Ammoniak alkalisiert, mit Methylenchlorid ausgeschüttelt, die organische Phase mit Wasser gewaschen, über Natriumsulfat getrocknet und i. Vak. eingedampft. Das Reaktionsprodukt wird durch präparative Schichtchromatographie an Kieselgel 60 $PF_{254}$ gipshaltig isoliert und gereinigt

(Fließmittel: Chloroform/Methanol, 99,5/0,5, Ammoniakatmosphäre). Nach Eindampfen der Eluate werden 1,35 g (50 %) eines farblosen amorphen Feststoffes (Schaum) erhalten.

$C_{28}H_{28}Cl_2N_6O$ (535,5)

$^1$H-NMR-Daten:

($CDCl_3$, TMS als interner Standard)

δ = 1,94 (m) 2 H,
2,25 (breit) 1 H,
2,5 - 2,8 (m) 3 H,
3,3 (breit) 2 H,
3,5 (breit) 1 H,
3,9 (breit) 1 H
4,14 (dd) 1 H,
6,73 (s) 1 H,
6,9 - 7,8 (m) 10 H,
8,11 (d) 2 H,
8,56 (d) 1 H,
10,2 - 10,9 (breit) 1 H, austauschbar mit $D_2O$, ppm

b) $N^1$-[3-(3,4-Dichlorphenyl)-3-(pyridin-2-yl)propyl]-$N^2$-[3-(1H-imidazol-4-yl)propyl]guanidin

0,8 g (1,5 mmol) $N^1$-Benzoyl-$N^2$-[3-(3,4-dichlorphenyl)-3-(pyridin-2-yl)propyl]-$N^3$-[3-(1H-imidazol-4-yl)-propyl]guanidin werden in 40 ml 20prozentiger Salzsäure 10 h unter Rückfluß erhitzt. Anschließend wird die salzsaure Lösung 3mal mit Ether extrahiert, i. Vak. zur Trockne eingedampft und im Hochvakuum getrocknet. Es verbleiben 0,74 g (91 %) des Trihydrochlorides in Form eines hygroskopischen amorphen Feststoffes.

$C_{21}H_{24}Cl_2N_6$ x 3HCl (540,8)

MS (FAB-Methode): m/z (rel. Int. [%]) = 431 ($[M+H]^+$, 35), 264 (80), 109 (100).

$^1$H-NMR-Daten:

(DMSO-$d_6$, TMS als interner Standard)

δ = 1,84 (m) 2 H,
2,3 - 2,65 (m) 2 H,
2,72 (t) 2 H,
3,0 - 3,35 (m) 4 H,
4,72 (t) 1 H,
7,45 - 8,2 (m) 10 H, 4 H austauschbar mit $D_2O$,
8,28 (dd) 1 H,
8,73 (d) 1 H
9,05 (s) 1 H,
14,45 (breit) 1H, austauschbar mit $D_2O$,
14,8 (breit) 1 H, austauschbar mit $D_2O$, ppm.

Beispiel 15

$N^1$-[3-(3,5-Dichlorphenyl)-3-(pyridin-2-yl)propyl]-$N^2$-[3-(1H-imidazol-4-yl)propyl]guanidin

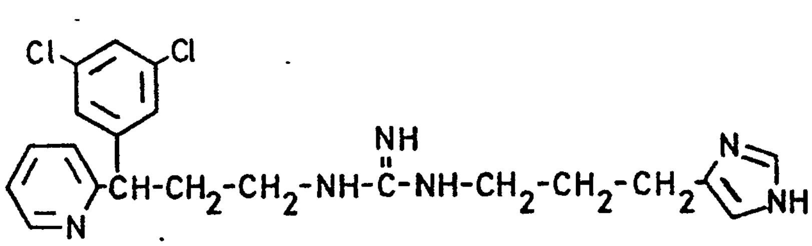

a) $N^1$-Benzoyl-$N^2$-[3-(3,5-dichlorphenyl)-3-(pyridin-2-yl)propyl-$N^3$-[3-(1H-imidazol-4-yl)propyl]guanidin

Die Herstellung und Isolierung erfolgt analog Beispiel 14 a) ausgehend von 1,41 g (5 mmol) 3-(3,5-Dichlorphenyl)-3-(pyridin-2-yl)propylamin.
Ausbeute: 1,25 g (47 %) farbloser amorpher Feststoff (Schaum).
$C_{28}H_{28}Cl_2N_6O$ (535,5)
$^1$H-NMR-Daten:
($CDCl_3$, TMS als interner Standard)
$\delta$ = 1,95 (m) 2 H,
2,3 (breit) 1 H,
2,55 - 2,8 (m) 3 H,
3,3 (breit) 2 H,
3,5 (breit) 1 H,
3,85 (breit) 1 H,
4,12 (dd) 1 H,
6,73 (s) 1 H,
6,9 - 7,75 (m) 10 H
8,12 (d) 2 H,
8,57 (d) 1 H,
10,2 - 10,9 (breit) 1 H, austauschbar mit $D_2O$, ppm.

b) $N^1$-[3-(3,5-Dichlorphenyl)-3-(pyridin-2-yl)propyl]-$N^2$-[3-(1H-imidazol-4-yl)propyl]guanidin

Die Herstellung erfolgt analog Beispiel 14 b), ausgehend von 0,8 g (1,5 mmol) $N^1$-Benzoyl-$N^2$-[3-(3,5-dichlorphenyl)-3-(pyridin-2-yl)propyl]-$N^3$-[3-(1H-imidazol-4-yl)propyl]guanidin.
Ausbeute: 0,7 g (86 %) des Trihydrochlorides in Form eines hygroskopischen amorphen Feststoffes.
$C_{21}H_{24}Cl_2N_6$ x 3HCl (540,8)
MS (FAB-Methode): m/z (rel. Int. [%]) = 431 ([M+H]$^+$, 24), 264 (72), 201 (28), 109 (100).
$^1$H-NMR-Daten:
(DMSO-$d_6$, TMS als interner Standard)
$\delta$ = 1,83 (m) 2 H,
2,3 - 2,65 (m) 2 H,
2,71 (t) 2 H,
3,17 (dt) 2 H,
3,44 (dt) 2 H,
4,60 (t) 1 H,
7,5 - 8,2 (m) 11 H, 4 H austauschbar mit $D_2O$
8,68 (d) 1 H,
9,04 (d) 1 H,
14,35 (breit) 1 H, austauschbar mit $D_2O$,
14,7 (breit) 1 H, austauschbar mit $D_2O$, ppm.

Beispiel 16

$N^1$-[3-(2,4-Dichlorphenyl)-3-(pyridin-2-yl)propyl]-$N^2$-[3-(1H-imidazol-4-yl)propyl]guanidin

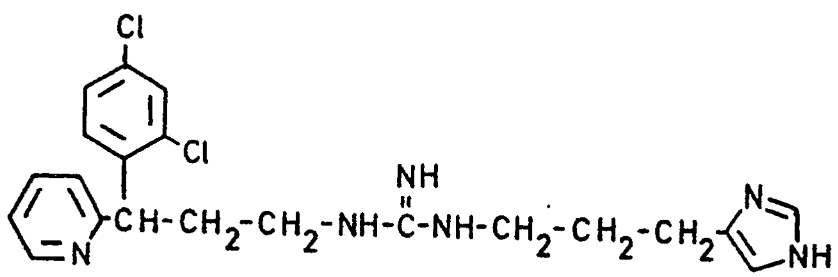

a) $N^1$-Benzoyl-$N^2$-[3-(2,4-dichlorphenyl)-3-(pyridin-2-yl)propyl-$N^3$-[3-(1H-imidazol-4-yl)propyl]guanidin

Die Herstellung erfolgt analog Beispiel 14 a) ausgehend von 1,41 g (5 mmol) 3-(2,4-Dichlorphenyl)-3-(pyridin-2-yl)propylamin.
Ausbeute: 1,1 g (41 %) farbloser amorpher Feststoff (Schaum).
$C_{28}H_{28}Cl_2N_6O$ (535,5)
$^1$H-NMR-Daten:
($CDCl_3$, TMS als interner Standard)
$\delta$ = 1,95 (m) 2 H,
2,25 (breit) 1 H,
2,5 - 2,85 (m) 3 H,
3,1 - 3,7 (breit) 3 H,
3,9 (breit) 1 H,
4,72 (m) 1 H,
6,7 - 7,8 (m) 11 H,
8,12 (d) 2 H,
8,59 (d) 1 H,
10,3 - 11,0 (breit) 1 H, austauschbar mit $D_2O$, ppm.

b) N[1]-[3-(2,4-Dichlorphenyl)-3-(pyridin-2-yl)propyl]-N[2]-[3-(1H-imidazol-4-yl)propyl]guanidin

Die Herstellung erfolgt analog Beispiel 14 b) ausgehend von 0,65 g (1,2 mmol) N[1]-Benzoyl-N[2]-[3-(2,4-dichlorphenyl)-3-(pyridin-2-yl)propyl-N[3]-[3-(1H-imidazol-4-yl)propyl]guanidin.
Ausbeute: 0,6 g (92 %) des Trihydrochlorides in Form eines hygroskopischen amorphen Feststoffes.
$C_{21}H_{24}Cl_2N_6$ x 3HCl (540,8)
MS (FAB-Methode): m/z (rel. Int. [%]) = 431 ($[M+H]^+$, 49), 264 (82), 237 (12), 201 (24), 167 (20), 126 (15), 118 (13), 109 (100), 100 (59).
$^1$H-NMR-Daten:
(DMSO-$d_6$, TMS als interner Standard)
$\delta$ = 1,85 (m) 2 H,
2,36 (m) 1 H,
2,53 (m) 1 H,
2,73 (t) 2 H,
3,18 (m) 4 H,
4,93 (t) 1 H,
7,49 (s) 1 H,
7,50 (d) 1 H,
7,6 - 7,8 (m) 6 H, 2 H austauschbar mit $D_2O$,
8,03 (m) 1 H, austauschbar mit $D_2O$,
8,07 (m) 1 H, austauschbar mit $D_2O$,
8,19 (dd) 1 H,
8,74 (d) 1 H,
9,06 (s) 1 H,
14,5 (breit) 1 H, austauschbar mit $D_2O$,
14,85 (breit), 1 H, austauschbar mit $D_2O$, ppm.

Beispiel 17

N[1]-[3-(3,4-Difluorphenyl)-3-(pyridin-2-yl)propyl]-N[2]-[3-(1H-imidazol-4-yl)propyl]guanidin

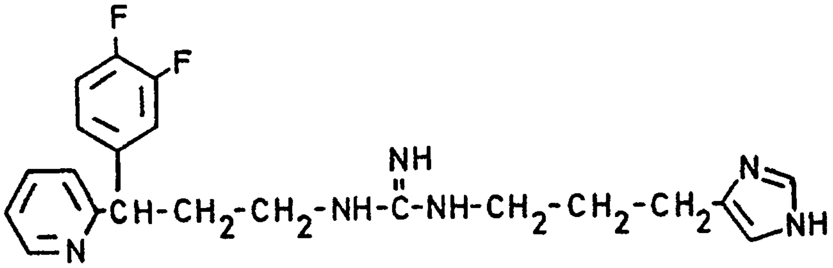

a) $N^1$-Benzoyl-$N^2$-[3-(3,4-difluorphenyl)-3-(pyridin-2-yl)propyl-$N^3$-[3-(1H-imidazol-4-yl)propyl]guanidin

Die Herstellung erfolgt analog Beispiel 14 a), ausgehend von 1,24 g (5 mmol) 3-(3,4-Difluorphenyl)-3-(pyridin-2-yl)propylamin.
Ausbeute: 1,2 g (48 %) farbloser amorpher Feststoff (Schaum).
$C_{28}H_{28}F_2N_6O$ (502,6)
$^1$H-NMR-Daten:
($CDCl_3$, TMS als interner Standard)
$\delta$ = 1,95 (m) 2 H,
2,3 (breit) 1 H,
2,55 - 2,8 (m) 3 H,
3,32 (breit) 2 H,
3,5 (breit) 1 H,
3,85 (breit) 1 H,
4,15 (dd) 1 H,
6,73 (s) 1 H,
6,8 - 7,75 (m) 10 H,
8,12 (d) 2 H,
8,57 (d) 1 H,
10,2 - 10,9 (breit) 1 H, austauschbar mit $D_2O$, ppm.

b) $N^1$-[3-(3,4-Difluorphenyl)-3-(pyridin-2-yl)propyl]-$N^2$-[3-(1H-imidazol-4-yl)propyl]guanidin

Die Herstellung erfolgt analog Beispiel 14 b), ausgehend von 0,76 g (1,5 mmol) $N^1$-Benzoyl-$N^2$-[3-(3,4-difluorphenyl)-3-(pyridin-2-yl)propyl]-$N^3$-[3-(1H-imidazol-4-yl)propyl]guanidin.
Ausbeute: 0,68 g (89 %) des Trihydrochlorides in Form eines hygroskopischen amorphen Feststoffes.
$C_{21}H_{24}F_2N_6$ x 3HCl (507,8)
Molmasse (MS):
Ber.: 398,2031
Gef.: 398,2028
MS (FAB-Methode): m/z (rel. Int [%]) = 399 ($[M+H]^+$, 58), 232 (100), 204 (23), 109 (43),
$^1$H-NMR-Daten:
(DMSO-$d_6$, TMS als interner Standard)
$\delta$ = 1,85 (m) 2 H,
2,34 - 2,65 (m) 2 H,
2,73 (t) 2 H,
3,11 (dt) 2 H,
3,18 (dt) 2 H,
4,73 (t) 1 H,
7,35 - 7,8 (m) 7 H, 2 H austauschbar mit $D_2O$,
7,99 (m) 3 H, 2 H austauschbar mit $D_2O$,
8,31 (dd) 1 H,
8,74 (d) 1 H,
9,05 (s) 1 H,
14,45 (breit) 1 H,austauschbar mit $D_2O$, ppm.
14,8 (breit) 1 H, austausch bar mit $D_2O$, ppm.

Beispiel 18

$N^1$-[3-(3,5-Difluorphenyl)-3-(pyridin-2-yl)propyl]-$N^2$-[3-(1H-imidazol-4-yl)propyl]guanidin

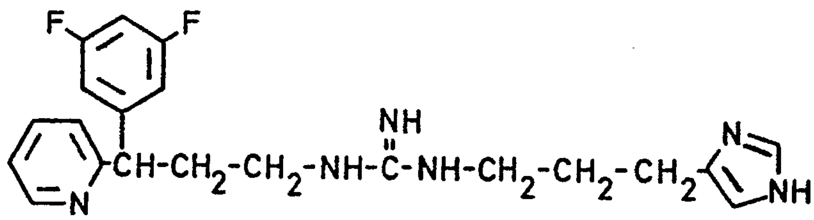

a) $N^1$-Benzoyl-$N^2$-[3-(3,5-difluorphenyl)-3-(pyridin-2-yl)propyl]-$N^3$-[3-(1H-imidazol-4-yl)propyl]guanidin

Die Herstellung erfolgt analog Beispiel 14 a) ausgehend von 1,24 g 3-(3,5-Difluorphenyl)-3-(pyridin-2-yl)-propylamin.
Ausbeute: 1,3 g (52 %) farbloser amorpher Feststoff.
$C_{28}H_{28}F_2N_6O$ (502,6)
$^1$H-NMR-Daten:
($CDCl_3$, TMS als interner Standard)
$\delta$ = 1,96 (m) 2 H,
2,3 (breit) 1 H,
2,6 - 2,8 (m) 3 H,
3,34 (breit) 2 H,
3,5 (breit) 1 H,
3,9 (breit) 1 H,
4,17 (dd) 1 H,
6,6 - 7,8 (m) 11 H,
8,12 (d) 2 H,
8,58 (d) 1 H,
10,3 - 10,9 (breit) 1 H, austauschbar mit $D_2O$, ppm.

b) $N^1$-[3-(3,5-Difluorphenyl)-3-(pyridin-2-yl)propyl]-$N^2$-[3-(1H-imidazol-4-yl)propyl]guanidin

Die Herstellung erfolgt analog Beispiel 14 b) ausgehend von 0,76 g (1,5 mmol) $N^1$-Benzoyl-$N^2$-[3-(3,5-difluorphenyl)-3-(pyridin-2-yl)propyl]-$N^3$-[3-(1H-imidazol-4-yl)propyl]guanidin.
Ausbeute: 0,65 g (85 %) des Trihydrochlorides in Form eines hygroskopischen amorphen Feststoffes.
$C_{21}H_{24}F_2N_6$ x 3HCl (507,8)
MS (FAB-Methode): m/z (rel. Int. [%]) = 399 ($[M+H]^+$, 80), 232 (100), 204 (18), 109 (60), 100 (36), 95 (11).
$^1$H-NMR-Daten:
(DMSO-$d_6$, TMS als interner Standard)
$\delta$ = 1,85 (m) 2 H,
2,35 - 2,65 (m) 2 H,
2,72 (t) 2 H,
3,0 - 3,3 (m) 4 H,
4,78 (t) 1 H,
7,16 (dd) 1 H,
7,36 (d) 2 H,
7,51 (s) 1 H,
7,62 (s) 2 H, austauschbar mit $D_2O$,
7,76 (dd) 1 H,
8,02 (m) 3 H, 2 H austauschbar mit $D_2O$,
8,32 (dd) 1 H,
8,75 (d) 1 H,
9,05 (s) 1 H,
14,45 (breit) 1 H, austauschbar mit $D_2O$,
14,8 (breit) 1 H, austauschbar mit $D_2O$, ppm.

Beispiel 19

N[1]-[3-(1H-Imidazol-4-yl)propyl]-N[2]-[3-(4-methylphenyl)-3-(pyridin--yl)propyl]guanidin

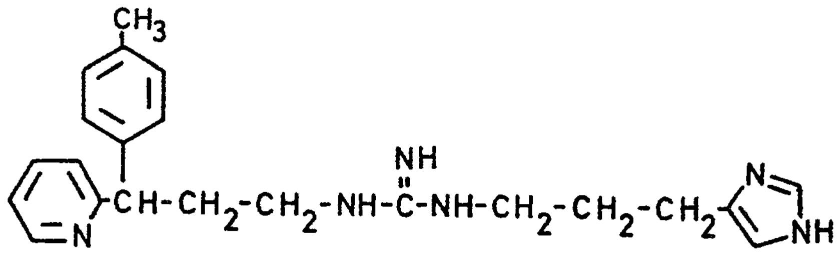

a) N[1]-Benzoyl-N[2]-[3-(1H-imidazol-4-yl)propyl-N[3]-[3-(4-methylphenyl)-3-(pyridin-2-yl)propyl]guanidin

Die Herstellung erfolgt analog Beispiel 14 a), ausgehend von 1,13 g (5 mmol) 3-(4-Methylphenyl)-3-(pyridin-2-yl)propylamin.
Ausbeute: 1,3 g (54 %) farbloser amorpher Feststoff (Schaum).
$C_{29}H_{32}N_6O$ (480,6)
$^1$H-NMR-Daten:
($CDCl_3$, TMS als interner Standard)
$\delta$ = 1,96 (m) 2 H,
2,1 - 2,45 (m) 1 H,
2,30 (s) 3 H,
2,55 - 2,8 (m) 3 H,
3,17 - 3,7 (breit) 3 H,
3,9 (breit) 1 H,
4,16 (dd) 1 H,
6,72 (s) 1 H,
7,0 - 7,7 (m) 11 H,
8,12 (d) 2 H,
8,55 (d) 1 H,
10,75 (breit) 1 H, austauschbar mit $D_2O$, ppm.

b) N[1]-[3-(1H-Imidazol-4-yl)propyl]-N[2]-[3-(4-methylphenyl)-3-(pyridin-2-yl)propyl]guanidin

Die Herstellung erfolgt analog Beispiel 14 b), ausgehend von 0,72 g (1,5 mmol) N[1]-Benzoyl-N[2]-[3-(1H-imidazol-4-yl)propyl]-N[3]-[3-(4-methylphenyl)-3-(pyridin-2-yl)propyl]guanidin.
Ausbeute: 0,66 g (90 %) des Trihydrochlorides in Form eines hygroskopischen amorphen Feststoffes.
$C_{22}H_{28}N_6$ x 3HCl (485,9)
MS (FAB-Methode): m/z (rel. Int. [%]) = 377 ($[M+H]^+$, 71), 210 (100), 182 (17), 109 (37), 100 (29).
$^1$H-NMR-Daten:
(DMSO-$d_6$, TMS als interner Standard)
$\delta$ = 1,84 (m) 2 H,
2,2 - 2,65 (m) 2 H,
2,25 (s) 3 H,
2,72 (t) 2 H,
3,11 (dt) 2 H,
3,18 (dt) 2 H,
4,65 (t) 1 H,
7,16 (d) 2 H,
7,3 - 8,45 (m) 10 H, 4 H austauschbar mit $D_2O$,
8,73 (d) 1 H,
9,05 (d) 1 H,
14,45 (breit) 1 H, austauschbar mit $D_2O$,
14,8 (breit) 1 H, austauschbar mit $D_2O$, ppm.

Beispiel 20

N[1]-[3-(3-Chlorphenyl)-3-(pyridin-2-yl)propyl]-N[2]-[3-(1H-imidazol-4-yl)propyl]guanidin

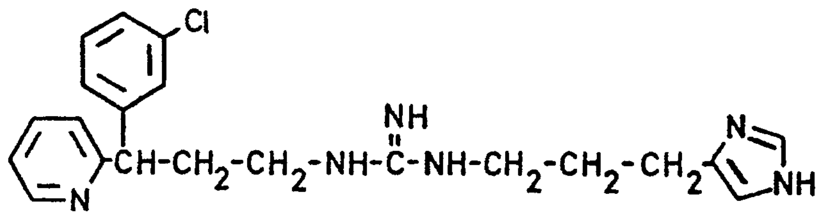

a) N[1]-Benzoyl-N[2]-[3-(3-chlorphenyl)-3-(pyridin-2-yl)propyl]-N[3]-[3-(1H-imidazol-4-yl)propyl]guanidin

Die Herstellung erfolgt analog Beispiel 14 a) ausgehend von 1,23 g (5 mmol) 3-(3-Chlorphenyl)-3-(pyridin-2-yl)propylamin.
Ausbeute: 1,45 g (58 %) farbloser amorpher Feststoff (Schaum).
$C_{28}H_{29}ClN_6O$ (501,0)
$^1$H-NMR-Daten:
($CDCl_3$, TMS als interner Standard)
$\delta$ = 1,96 (m) 2 H,
2,32 (breit) 1 H,
2,55 - 2,75 (m) 1 H,
2,69 (t) 2 H,
3,15 - 3,7 (breit) 3 H,
3,9 (breit) 1 H,
4,16 (dd) 1 H,
6,73 (s) 1 H,
7,0 - 7,75 (m) 11 H,
8,12 (d) 2 H,
8,56 (d) 1 H,
10,2 - 11,0 (breit)1 H, austauschbar mit $D_2O$, ppm.

b) N[1]-[3-(3-Chlorphenyl)-3-(pyridin-2-yl)propyl]-N[2]-[3-(1H-imidazol-4-yl)propyl]guanidin

Die Herstellung erfolgt analog Beispiel 14 b) ausgehend von 0,75 g (1,5 mmol) N[1]-Benzoyl-N[2]-[3-(3-chlorphenyl)-3-(pyridin-2-yl)propyl]-N[3]-[3-(1H-imidazol-4-yl)propyl]guanidin.
Ausbeute: 0,69 g (91 %) des Trihydrochlorides in Form eines hygroskopischen amorphen Feststoffes.
$C_{21}H_{25}ClN_6$ x 3HCl (506,3)
MS (FAB-Methode): m/z (rel. Int. [%]) = 397 ($[M+H]^+$, 77), 230 (100), 109 (65), 100 (41).
$^1$H-NMR-Daten:
(DMSO-$d_6$, TMS als interner Standard)
$\delta$ = 1,83 (m) 2 H,
2,25 - 2,65 (m) 2 H,
2,71 (t) 2 H,
3,10 (dt) 2 H,
3,18 (dt) 2 H,
4,64 (t) 1 H,
7,3 - 7,75 (m) 7 H, 2 H ausbar mit $D_2O$,
7,8 - 8,1 (m) 4 H, 2 H austauschbar mit $D_2O$,
8,20 (dd) 1 H,
8,70 (d) 1 H,
9,04 (s) 1 H,
14,35 (breit) 1 H, austauschbar mit $D_2O$,
14,7 (breit) 1 H, austauschbar mit $D_2O$, ppm.

Beispiel 21

N¹-[3-(2-Chlorphenyl)-3-(pyridin-2-yl)propyl]-N²-[3-(1H-imidazol-4-yl)propyl]guanidin

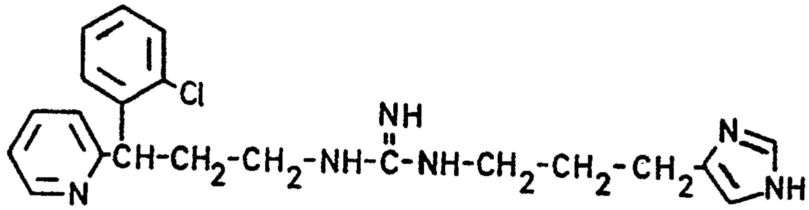

a) N¹-Benzoyl-N²-[3-(2-chlorphenyl)-3-(pyridin-2-yl)propyl]-N³-[3-(1H-imidazol-4-yl)propyl]guanidin

Die Herstellung erfolgt analog Beispiel 14 a) ausgehend von 1,23 g (5 mmol) 3-(2-Chlorphenyl)-3-(pyridin-2-yl)propylamin.
Ausbeute: 1,1 g (44 %) farbloser amorpher Feststoff (Schaum).
$C_{28}H_{29}ClN_6O$ (501,0)
¹H-NMR-Daten:
($CDCl_3$, TMS als interner Standard)
δ = 1,97 (m) 2 H,
2,25 (m) 1 H,
2,5 - 2,7 (m) 3 H,
3,1 - 3,7 (breit) 3 H,
3,90 (breit) 1 H,
4,73 (m) 1 H,
6,71 (s) 1 H,
6,8 - 7,9 (m) 11 H,
8,11 (d) 2 H.
8,57 (d) 1 H,
8,2 - 8,9 (breit) 1 H, austauschbar mit $D_2O$, ppm.

b) N¹-[3-(2-Chlorphenyl)-3-(pyridin-2-yl)propyl]-N²-[3-(1H-imidazol-4-yl)propyl]guanidin

Die Herstellung erfolgt analog Beispiel 14 b) ausgehend von 0,55 g (1,1 mmol) N¹-Benzoyl-N²-[3-(2-chlorphenyl)-3-(pyridin-2-yl)propyl]-N³-[3-(1H-imidazol-4-yl)propyl]guanidin
Ausbeute: 0,49 g (88 %) des Trihydrochlorides in Form eines hygroskopischen amorphen Feststoffes.
$C_{21}H_{25}ClN_6$ x 3HCl (506,3)
MS (FAB-Methode): m/z (rel. Int. [%]) = 397 ($[M+H]^+$, 64, 230 (100), 203 (13), 194 (20), 168 (18), 167 (32), 126 (13), 109 (66), 100 (37), 95 (11).
¹H-NMR-Daten:
(DMSO-$d_6$, TMS als interner Standard)
δ = 1,86 (m) 2 H,
2,35 - 2,7 (m) 2 H,
2,73 (t) 2 H,
3,1 - 3,4 (m) 4 H,
5,01 (t) 1 H,
7,3 - 8,0 (m) 9 H, 2 H austauschbar mit $D_2O$,
8,05 (m) 2 H, austauschbar mit $D_2O$,
8,24 (dd) 1 H,
8,77 (d) 1 H,
9,06 (s) 1 H,
14,50 (breit) 1 H, austauschbar mit $D_2O$,
14,83 (breit) 1 H, austauschbar mit $D_2O$, ppm.

Beispiel 22

N[1]-[3-(3-Fluorphenyl)-3-(pyridin-2-yl)propyl]-N[2]-[3-(1H-imidazol-4-yl)propyl]guanidin

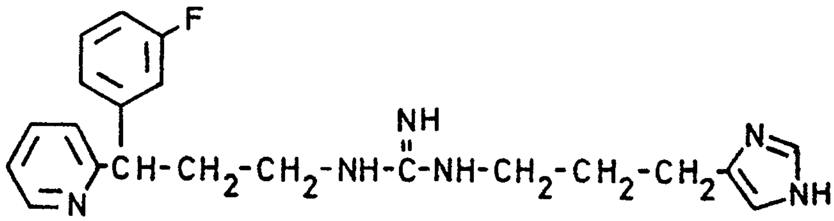

a) N[1]-Benzoyl-N[2]-[3-(3-fluorphenyl)-3-(pyridin-2-yl)propyl]-N[3]-[3-(1H-imidazol-4-yl)propyl]guanidin

Die Herstellung erfolgt analog Beispiel 14 a) ausgehend von 1,15 g (5 mmol) 3-(3-Fluorphenyl)-3-(pyridin-2-yl)propylamin.
Ausbeute: 1,19 g (49 %) farbloser amorpher Feststoff (Schaum).
$C_{28}H_{29}FN_6O$ (484,6)

b) N[1]-[3-(3-Fluorphenyl)-3-(pyridin-2-yl)propyl]-N[2]-[3-(1H-imidazol-4-yl)propyl]guanidin

Die Herstellung erfolgt analog Beispiel 14 b), ausgehend von 0,73 g (1,5 mmol) N[1]-Benzoyl-N[2]-[3-(3-fluorphenyl)-3-(pyridin-2-yl)propyl]-N[3]-[3-(1H-imidazol-4-yl)propyl]guanidin.
Ausbeute: 0,65 g (88 %) des Trihydrochlorides in Form eines hygroskopischen amorphen Feststoffes.
$C_{21}H_{25}FN_6$ x 3HCl (489.9)
MS (FAB-Methode): m/z (rel. Int. [%]) = 381 ($[M+H]^+$, 47), 214 (100), 186 (23), 109 (49), 100 (24).
$^1$H-NMR-Daten:
(DMSO-$d_6$, TMS als interner Standard)
$\delta$ = 1,85 (m) 2 H,
2,35 - 2,7 (m) 2 H,
2,73 (t) 2 H,
3,05 - 3,35 (m) 4 H,
4,76 (t) 1 H,
7,10 (m) 1 H,
7,25 - 7,85 (m) 7 H, 2 H austauschbar mit $D_2O$,
8,03 (m) 3 H, 2 H austauschbar mit $D_2O$,
8,35 (dd) 1 H,
8,75 (d) 1 H,
9,05 (s) 1 H,
14,5 (breit), 1 H, austauschbar mit $D_2O$,
14,85 (breit), 1 H, austauschbar mit $D_2O$, ppm.

<u>Beispiel 23</u>

N[1]-[3-(2-Fluorphenyl)-3-(pyridin-2-yl)propyl]-N[2]-[3-(1H-imidazol-4-yl)propyl]guanidin

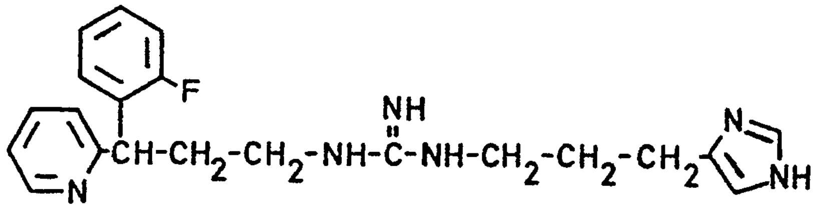

a) N[1]-Benzoyl-N[2]-[3-(2-fluorphenyl)-3-(pyridin-2-yl)propyl]-N[3]-[3-(1H-imidazol-4-yl)propyl]guanidin

Die Herstellung erfolgt analog Beispiel 14 a) ausgehend von 1,15 g (5 mmol) 3-(2-Fluorphenyl)-3-(pyridin-2-yl)propylamin. Ausbeute: 1,24 g (51 %) farbloser amorpher Feststoff (Schaum).
$C_{28}H_{29}FN_6O$ (484,6)

b) $N^1$-[3-(2-Fluorphenyl)-3-(pyridin-2-yl)propyl]-$N^2$-[3-(1H-imidazol-4-yl)propyl]guanidin

Die Herstellung erfolgt analog Beispiel 14 b), ausgehend von 0,73 g (1,5 mmol) $N^1$-Benzoyl-$N^2$-[3-(2-fluorphenyl)-3-(pyridin-2-yl)propyl]-$N^3$-[3-(1H-imidazol-4-yl)propyl]guanidin.
Ausbeute: 0,66 g (90%) des Trihydrochlorides in Form eines hygroskopischen amorphen Feststoffes.
$C_{21}H_{25}FN_6$ x 3HCl (489.9)
MS (FAB-Methode: m/z (rel. Int. [%]) = 381 ($[M+H]^+$, 61), 214 (100), 186 (11), 109 (50), 100 (32).
$^1H$-NMR-Daten:
(DMSO-$d_6$, TMS als interner Standard)
$\delta$ = 1,85 (m) 2 H,
2,37 (m) 1 H,
2,6 (m) 1 H,
2,73 (t) 2 H,
3,18 (m) 4 H,
4,82 (t) 1 H,
7,15 - 7,45 (m) 3 H,
7,47 (s) 1 H,
7,5 - 7,85 (m) 5 H, 2 H austauschbar mit $D_2O$,
7,95 - 8,05 (m) 2 H, austauschbar mit $D_2O$,
8,22 (dd) 1 H,
8,73 (d) 1 H,
9,04 (s) 1 H,
14,45 (breit) 1 H, austauschbar mit $D_2O$,
14,80 (breit) 1 H, austauschbar mit $D_2O$, ppm.

Beispiel 24

$N^1$-[3-(4-Fluorphenyl)-3-(pyridin-3-yl)propyl]-$N^2$-[3-(1H-imidazol-4-yl)propyl]guanidin

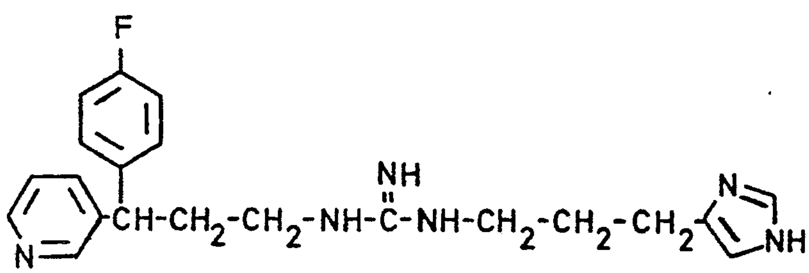

a) $N^1$-Benzoyl-$N^2$-[3-(4-fluorphenyl)-3-(pyridin-3-yl)propyl]-$N^3$-[3-(1H-imidazol-4-yl)propyl]guanidin

Die Herstellung erfolgt analog Beispiel 14 a) ausgehend von 1,15 g (5 mmol) 3-(4-Fluorphenyl)-3-(pyridin-3-yl)propylamin.
Ausbeute: 1,15 g (47 %) farbloser amorpher Feststoff (Schaum).
$C_{28}H_{29}FN_6O$ (484,6)
$^1H$-NMR-Daten:
($CDCl_3$, TMS als interner Standard)
$\delta$ = 1,87 (m) 2 H,
2,41 (dt) 2 H,
2,63 (t) 2 H,
3,1 - 3,8 (breit) 4 H,
4,06 (t) 1 H,

6,72 (s) 1 H,
6,95 (dd) 2 H,
7,1 - 7,6(m) 8 H,
8,10 (d) 2 H,
8,41 (dd) 1 H,
8,45 (d) 1 H,
10,45 (breit) 1 H, austauschbar mit $D_2O$, ppm.

b) $N^1$-[3-(4-Fluorphenyl)-3-(pyridin-3-yl)propyl]-$N^2$-[3-(1H-imidazol-4-yl)propyl]guanidin

Die Herstellung erfolgt analog Beispiel 14 b), ausgehend von 0,73 g (1,5 mmol) $N^1$-Benzoyl-$N^2$-[3-(4-fluorphenyl)-3-(pyridin-3-yl)propyl]-$N^3$-[3-(1H-imidazol-4-yl)propyl]guanidin.
Ausbeute: 0,61 g (83 %) des Trihydrochlorides in Form eines hygroskopischen amorphen Feststoffes.
$C_{21}H_{25}FN_6$ x 3HCl (489.9)
MS (FAB-Methode): m/z (rel. Int. [%]) = 381 ($[M+H]^+$, 99), 214 (9), 186 (21), 109 (100), 100 (36).
$^1$H-NMR-Daten:
(DMSO-$d_6$, TMS als interner Standard)
$\delta$ = 1,84 (m) 2 H,
2,389 (m) 2 H
2,73 (t) 2 H,
3,09 (m) 2 H,
3,18 (dt) 2 H,
4,56 (t) 1 H,
7,18 (dd) 2 H,
7,48 (s) 1 H,
7,51 (dd) 2 H,
7,60 (s) 2 H, austauschbar mit $D_2O$,
7,96 (d) 1 H,
8,07 (m) 2 H, austauschbar mit $D_2O$,
8,54 (d) 1 H,
8,78 (d) 1 H,
9,00 (s) 1 H,
9,05 (s) 1 H,
14,45 (breit) 1 H, austauschbar mit $D_2O$,
14,8 (breit) 1 H, austauschbar mit $D_2O$, ppm.

Beispiel 25

$N^1$-[3-(4-Fluorphenyl)-3-(pyridin-4-yl)propyl]-$N^2$-[3-(1H-imidazol-4-yl)propyl]guanidin

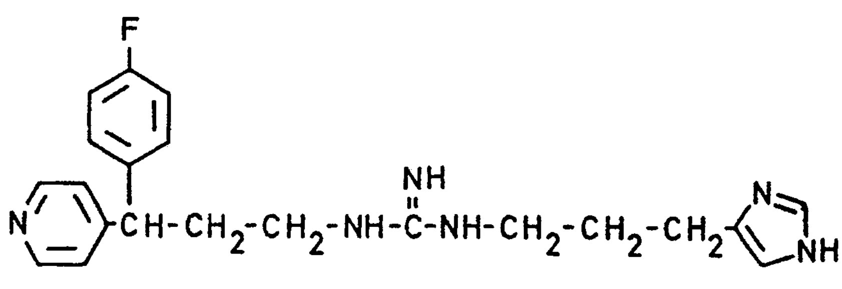

a) $N^1$-Benzoyl-$N^2$-[3-(4-fluorphenyl)-3-(pyridin-4-yl)propyl]-$N^3$-[3-(1H-imidazol-4-yl)propyl]guanidin

Die Herstellung erfolgt analog Beispiel 14 a) ausgehend von 1,15 g (5 mmol) 3-(4-Fluorphenyl)-3-(pyridin-4-yl)propylamin.
Ausbeute: 1,24 g (51 %) farbloser amorpher Feststoff (Schaum).
$C_{28}H_{29}FN_6O$ (484,6)
$^1$H-NMR-Daten:

($CDCl_3$, TMS als interner Standard)
$\delta$ = 1,87 (m) 2 H,
2,39 (dt) 2 H,
2,63 (t) 2 H,
3,1 - 3,8 (breit) 4 H,
4,01 (t) 1 H,
6,72 (s) 1 H
6,95 (dd) 2 H,
7,0 - 7,55 (m) 8 H,
8,09 (d) 2 H,
8,42 (d) 2 H,
10,45 (breit) 1 H, austauschbar mit $D_2O$, ppm.

b) $N^1$-[3-(4-Fluorphenyl)-3-(pyridin-4-yl)propyl]-$N^2$-[3-(1H-imidazol-4-yl)propyl]guanidin

Die Herstellung erfolgt analog Beispiel 14 b), ausgehend von 0,73 g (1,5 mmol) $N^1$-Benzoyl-$N^2$-[3-(4-fluorphenyl)-3-(pyridin-4-yl)propyl]-$N^3$-[3-(1H-imidazol-4-yl)propyl]guanidin.
Ausbeute: 0,65 g (88 %) des Trihydrochlorides in Form eines hygroskopischen amorphen Feststoffes.
$C_{21}H_{25}FN_6$ x 3HCl (489.9)
MS (FAB-Methode): m/z (rel. Int. [%]) = 381 ($[M+H]^+$, 73), 214 (22), 187 (34), 186 (8), 109 (100), 100 (36).
$^1$H-NMR-Daten:
(DMSO-$d_6$, TMS als interner Standard)
$\delta$ = 1,85 (m) 2 H,
2,39 (m) 2 H,
2,73 (t) 2 H,
3,11 (dt) 2 H,
3,19 (dt) 2 H,
4,64 (t) 1 H,
7,20 (dd) 2 H,
7,49 (s) 1 H,
7,51 (dd) 2 H,
7,63 (s) 2 H, austauschbar mit $D_2O$,
8,05 - 8,2 (m) 2 H, austauschbar mit $D_2O$,
8,07 (d) 2 H,
8,86 (d) 2 H,
9,06 (s) 1 H,
14,5 (breit) 2 H, austauschbar mit $D_2O$, ppm.

Beispiel 26

$N^1$-[3-(1H-Imidazol-4-yl)propyl]-$N^2$-[3-(pyridin-2-yl)-3-(3-trifluormethyl-phenyl)propyl]guanidin

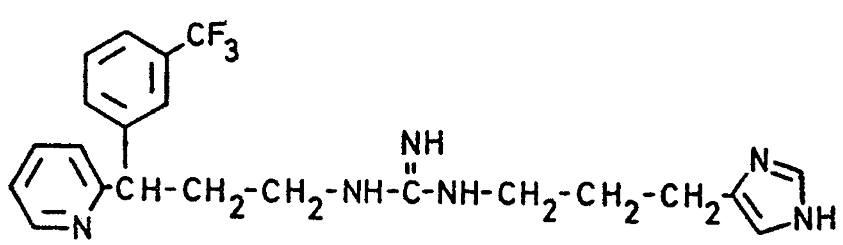

a) $N^1$-Benzoyl-$N^2$-[3-(1H-imidazol-4-yl)propyl]-$N^3$-[3-(pyridin-2-yl)-3-(3-trifluormethyl-phenyl)propyl]guanidin

Die Herstellung erfolgt analog Beispiel 14 a) ausgehend von 1,12 g (4 mmol) 3-(Pyridin-2-yl)-3-(3-trifluormethylphenyl)propylamin.
Ausbeute: 1,2 g (56 %) farbloser amorpher Feststoff.

$C_{29}H_{29}F_3N_6O$ (534,6)
$^1$H-NMR-Daten:
($CDCl_3$, TMS als interner Standard)
δ = 1,96 (m) 2 H,
2,32 (m) 1 H,
2,6 - 2,75 (m) 1 H,
2,70 (t) 2 H,
3,37 (breit) 2 H,
3,55 (breit) 1 H,
3,90 (breit) 1 H,
4,26 (dd) 1 H,
6,74 (s) 1 H,
7,1 - 7,8 (m) 11 H,
8,12 (d) 2 H,
8,60 (d) 1 H,
10,3 - 10,9 (breit) 1 H, austauschbar mit $D_2O$, ppm.

b) $N^1$-[3-(1H-Imidazol-4-yl)propyl]-$N^2$-[3-(pyridin-2-yl)-3-(3-trifluormethyl-phenyl)propyl]guanidin

Die Herstellung erfolgt analog Beispiel 14 b) ausgehend von 0,65 g (1,2 mmol) $N^1$-Benzoyl-$N^2$-[3-(1H-imidazol-4-yl)propyl]-$N^3$-[3-(pyridin-2-yl)-3-(3-trifluormethyl-phenyl)propyl]guanidin.
Ausbeute: 0,6 g (93 %) des Trihydrochlorides in Form eines hygroskopischen amorphen Feststoffes.
$C_{22}H_{25}F_3N_6$ x 3HCl (539,9)
Molmasse (MS):
Ber.: 430,20928
Gef.: 430,20926
MS (FAB-Methode): m/z (rel. Int. [%]) = 431 ($[M+H]^+$, 43), 264 (100), 237 (14), 236 (12), 109 (64), 100 (39).
$^1$H-NMR-Daten:
(DMSO-$d_6$, TMS als interner Standard)
δ = 1,85 (m), 2 H,
2,41 (m) 1 H,
2,64 (m) 1 H,
2,73 (t) 2 H,
3,0 - 3,3 (m) 4 H,
4,85 (t) 1 H,
7,4 - 8,2 (m) 11 H, 4 H austauschbar mit $D_2O$,
8,33 (dd) 1 H,
8,76 (d) 1 H,
9,07 (s) 1 H,
14,5 (breit) 1 H, austauschbar mit $D_2O$,
14,85 (breit) 1 H, austauschbar mit $D_2O$, ppm.

Beispiel 27

$N^1$-[3-(1H-Imidazol-4-yl)propyl]-$N^2$-[3-(pyridin-2-yl)-3-(4-trifluormethyl-phenyl)propyl]guanidin

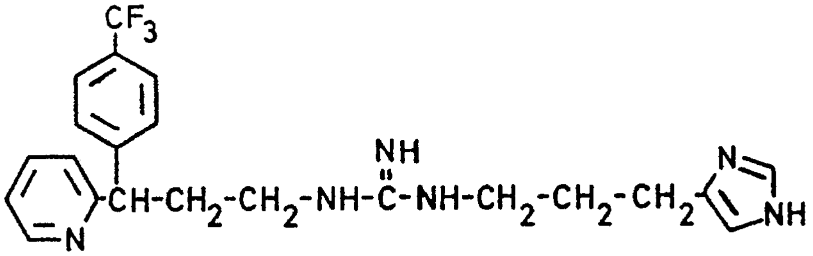

a) N[1]-Benzoyl-N[2]-[3-(1H-imidazol-4-yl)propyl]-N[3]-[3-(pyridin-2-yl)-3-(4-trifluormethyl-phenyl)propyl]guanidin

Die Herstellung erfolgt analog Beispiel 14 a) ausgehend von 1,12 g (4 mmol) 3-(Pyridin-2-yl)-3-(4-trifluormethylphenyl)propylamin.
Ausbeute: 1,0 g ( 47 %) farbloser amorpher Feststoff.
$C_{29}H_{29}F_3N_6O$ (534,6)

b) N[1]-[3-(1H-imidazol-4-yl)propyl]-N[2]-[3-(pyridin-2-yl)-3-(4-trifluormethyl-phenyl)propyl]guanidin

Die Herstellung erfolgt analog Beispiel 14 b) ausgehend von 0,65 g (1,2 mmol) N[1]-Benzoyl-N[2]-[3-(1H-imidazol-4-yl)propyl]-N[3]-[3-(pyridin-2-yl)-3-(4-trifluormethyl-phenyl)propyl]guanidin.
Ausbeute: 0,57 g (88 %) des Trihydrochlorides in Form eines hygroskopischen amorphen Feststoffes.
$C_{22}H_{25}F_3N_6$ x 3HCl (539,9)
$^1$H-NMR-Daten:
(DMSO-$d_6$, TMS als interner Standard)
$\delta$ = 1,85 (m) 2 H,
2,1 - 3,45 (m) 8 H,
4,90 (t) 1 H,
7,4 - 8,2 (m) 11 H, 4 H austauschbar mit $D_2O$,
8,32 (dd) 1 H,
8,78 (d) 1 H,
9,05 (s) 1 H,
14,45 (breit) 1 H, austauschbar mit $D_2O$,
14,8 (breit) 1 H, austauschbar mit $D_2O$, ppm.

Beispiel 28

N[1]-[3-(4-Fluorphenyl)-3-phenylpropyl]-N[2]-[3-(1H-imidazol-4-yl)propyl]guanidin

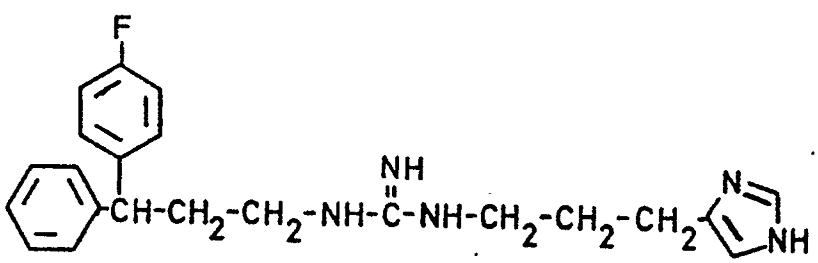

a) N[1]-Benzoyl-$N_2$-[3-(4-fluorphenyl)-3-phenylpropyl]-N[3]-[3-(1H-imidazol-4-yl)propyl]guanidin

Die Herstellung erfolgt analog Beispiel 14 a) ausgehend von 1,15 g (5 mmol) 3-(4-Fluorphenyl)-3-phenylpropylamin.
Ausbeute: 1,26 g (52 %) eines farblosen Schaumes, der beim Verreiben mit Ethylacetat/Ether kristallisiert.
Schmp. 127° C.
$C_{29}H_{30}FN_5O$ (483,6)
$^1$H-NMR-Daten:
($CDCl_3$, TMS als interner Standard)
$\delta$ = 1,86 (m) 2 H,
2,40 (dt) 2 H,
2,63 (t) 2 H,
3,4 (breit) 2 H,
3,55 (breit) 2 H,
4,04 (t) 1 H,
6,71 (s) 1 H,
6,95 (dd) 2 H,
7,1 - 7,6 (m) 11 H,

8,12 (d) 2 H,
10,2 - 10,9 (breit) 1 H, austauschbar mit $D_2O$, ppm.

b) N[1]-[3-(4-Fluorphenyl)-3-phenylpropyl]-N[2]-[3-(1H-imidazol-4-yl)propyl]guanidin

Die Herstellung erfolgt analog Beispiel 14 b) ausgehend von 0,58 g (1,2 mmol) N[1]-Benzoyl-N[2]-[3-(4-fluorphenyl)-3-phenylpropyl]-N[3]-[3-(1H-imidazol-4-yl)propyl]guanidin.
Ausbeute: 0,49 g (92 %) des Dihydrochlorides in Form eines hygroskopischen amorphen Feststoffes.
$C_{22}H_{26}FN_5$ x 2HCl (452,4)
MS (FAB-Methode): m/z (rel. Int. [%]) = 380 ($[M+H]^+$, 84), 272 (10), 185 (26), 109 (100), 100 (38), 95 (14), 91 (23).
$^1$H-NMR-Daten:
(DMSO-$d_6$, TMS als interner Standard)
$\delta$ = 1,82 (m) 2 H,
2,2 - 2,35 (m) 2 H,
2,70 (t) 2 H,
3,02 (dt) 2 H,
3,16 (dt) 2 H,
4,16 (t) 1 H,
7,1 - 7,6 (m) 12 H, 2 H austauschbar mit $D_2O$,
7,92 (breit) 2 H austauschbar mit $D_2O$,
9,03 (d) 1 H,
14,5 (breit) 2 H, austauschbar mit $D_2O$, ppm.

Beispiel 29

(N[1]-[3,3-Bis(4-fluorphenyl)propyl]-N[2]-[3-(1H-imidazol-4-yl)propyl]guanidin

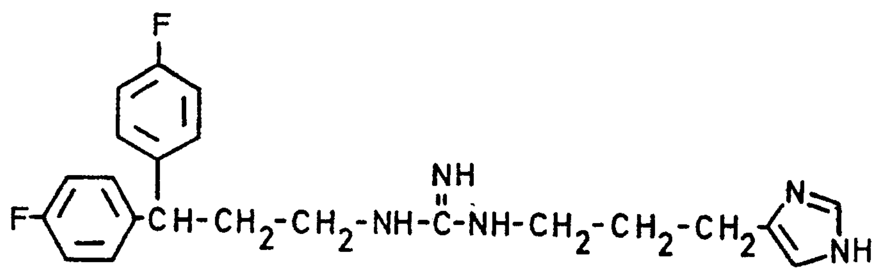

a) N[1]-Benzoyl-N[2]-[3,3-bis(4-fluorphenyl)propyl]-N[3]-[3-(1H-imidazol-4-yl)propyl]guanidin

Die Herstellung erfolgt analog Beispiel 14 a) ausgehend von 1,24 g (5 mmol) 3,3-Bis(4-fluorphenyl)-propylamin.
Ausbeute: 1,35 g (54 %), Schmp. 158 °C (Ether).
$C_{29}H_{29}F_2N_5O$ (501,6).
Analyse:
Ber.: C 69,44 H 5,83 N 13,96
Gef.: C 69,83 H 5,88 N 14,09
$^1$H-NMR-Daten:
($CDCl_3$, TMS als interner Standard)
$\delta$ = 1,88 (m) 2 H,
2,3 - 2,45 (m) 2 H,
2,65 (t) 2 H,
3,35 (breit) 2 H,
3,55 (breit) 2 H,
4,04 (t) 1 H,

6,75 (s) 1 H,
6,96 (dd) 4 H,
7,1-7,5 (m) 8 H,
8,10 (m) 2 H,
10,5 (breit) 1 H austauschbar mit $D_2O$,ppm.

b) $N^1$-[3,3-Bis(4-fluorphenyl)propyl]-$N^2$-[3-(1H-imidazol-4-yl)propyl]guanidin

Die Herstellung erfolgt analog Beispiel 14 b) ausgehend von 0,6 g (1,2 mmol) $N^1$-Benzoyl-$N^2$-[3,3-bis(4-fluorphenyl)propyl]-$N^3$-[3-(1H-imidazol-4-yl)propyl]guanidin.
Ausbeute: 0,5 g (88 %) des Dihydrochlorides in Form eines hygroskopischen amorphen Feststoffes.
$C_{22}H_{25}F_2N_5$ x 2HCl (470,4)
$^1$H-NMR-Daten:
(DMSO-$d_6$, TMS als interner Standard)
$\delta$ = 1,83 (m) 2 H,
2,2 - 2,35 (m) 2 H,
2,71 (t) 2 H,
3,03 (dt) 2 H,
3,17 (dt) 2 H,
4,18 (t) 1 H,
7,1 - 7,55 (m) 11 H, 2 H austauschbar mit $D_2O$,
7,9 (breit) 2 H, austauschbar mit $D_2O$,
9,02 (d) 1 H,
14,5 (breit) 2 H, austauschbar mit $D_2O$, ppm.

Beispiel 30

$N^1$-[3-(4-Chlorphenyl)-3-phenylpropyl]-$N^2$-[3-(1H-imidazol-4-yl)propyl]guanidin

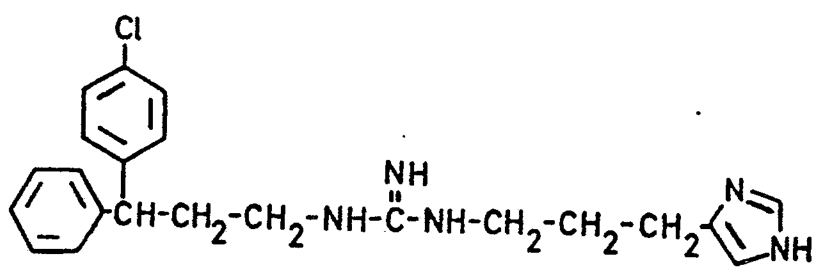

a) $N^1$-Benzoyl-$N^2$-[3-(4-chlorphenyl)-3-phenylpropyl]-$N^3$-[3-(1H-imidazol-4-yl)propyl]guanidin

Die Herstellung erfolgt analog Beispiel 14 a) ausgehend von 1,23 g (5 mmol) 3-(4-Chlorphenyl)-3-phenylpropylamin.
Ausbeute: 0,95 g (38 %) farbloser amorpher Feststoff.
$C_{29}H_{30}ClN_5O$ (500,0)
$^1$H-NMR-Daten:
($CDCl_3$, TMS als interner Standard)
$\delta$ = 1,86 (m) 2 H,
2,39 (dt) 2 H,
2,63 (m) 2 H,
3,1 - 3,8 (breit) 4 H,
4,02 (t) 1 H,
6,71 (s) 1 H,
7,05 - 7,55 (m) 13 H,
8,12 (d) 2 H,

10,4 (breit) 1 H, austauschbar mit $D_2O$, ppm.

b) $N^1$-[3-(4-Chlorphenyl)-3-phenylpropyl]-$N^2$-[3-(1H-imidazol-4-yl)propyl]guanidin

Die Herstellung erfolgt analog Beispiel 14 b) ausgehend von 0,55 g (1,1 mmol) $N^1$-Benzoyl-$N^2$-[3-(4-chlorphenyl)-3-phenylpropyl]-$N^3$-[3-(1H-imidazol-4-yl)propyl]guanidin.
Ausbeute: 0,45 g (91 %) des Dihydrochlorides in Form eines hygroskopischen amorphen Feststoffes.
$C_{22}H_{26}ClN_5O$ x 2HCl (449,4)
MS (FAB-Methode): m/z (rel. Int. [%]) = 396 ($[M+H]^+$, 38), 201 (10), 109 (100), 100 (41), 91 (25).
$^1$H-NMR-Daten:
(DMSO-$d_6$, TMS als interner Standard)
$\delta$ = 1,83 (m) 2 H,
2,26 (dt) 2 H,
2,71 (t) 2 H,
3,03 (dt) 2 H,
3,17 (dt) 2 H,
4,18 (t) 1 H,
7,2 - 7,6 (m) 12 H, 2 H austauschbar mit $D_2O$,
7,96 (breit) 2 H, austauschbar mit $D_2O$,
9,04 (d) 1 H,
14,5 (breit) 2 H, austauschbar mit $D_2O$, ppm.

Beispiel 31

$N^1$-[3-(3,4-Dichlorphenyl)-3-phenylpropyl]-$N^2$-[3-(1H-imidazol-4-yl)propyl]guanidin

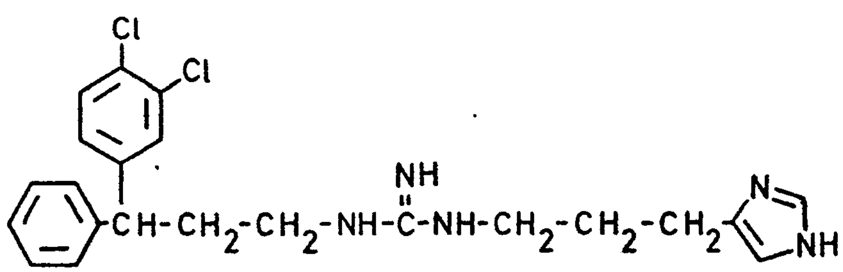

a) $N^1$-Benzoyl-$N^2$-[3-(3,4-dichlorphenyl)-3-phenylpropyl]-$N^3$-[3-(1H-imidazol-4-yl)propyl]guanidin

Die Herstellung erfolgt analog Beispiel 14 a) ausgehend von 1,4 g (5 mmol) 3-(3,4-Dichlorphenyl)-3-phenylpropylamin.
Ausbeute: 1,2 g (45 %) farbloser amorpher Feststoff.
$C_{29}H_{29}Cl_2N_5O$ (534,5)
$^1$H-NMR-Daten:
($CDCl_3$, TMS als interner Standard)
$\delta$ = 1,88 (m) 2 H,
2,40 (dt) 2 H,
2,66 (m) 2 H,
3,1 - 3,8 (breit) 4 H,
4,03 (t) 1 H,
6,76 (s) 1 H,
7,09 (d) 1 H,
7,15 - 7,5 (m) 11 H,
8,12 (d) 2 H,
10,5 (breit) 1 H, austauschbar mit $D_2O$, ppm.

b) $N^1$-[3-(3,4-Dichlorphenyl)-3-phenylpropyl]-$N^2$-[3-(1H-imidazol-4-yl)propyl]guanidin

Die Herstellung erfolgt analog Beispiel 14 b) ausgehend von 0,75 h (1,4 mmol) $N^1$-Benzoyl-$N^2$-[3-(3,4-dichlorphenyl)-3-phenylpropyl]-$N^3$-[3-(1H-imidazol-4-yl)propyl]guanidin.
Ausbeute: 0,57 g (87 %) des Dihydrochlorides in Form eines hygroskopsichen amorphen Feststoffes.
$C_{22}H_{25}Cl_2N_5$ x 2HCl (467,9)
MS (FAB-Methode): m/z (rel. Int. [%]) = 430 ($[M+H]^+$, 19), 165 (16), 109 (100), 100 (34), 91 (21).
$^1$H-NMR-Daten:
(DMSO-$d_6$, TMS als interner Standard)
$\delta$ = 1,84 (m) 2 H,
2,28 (dt) 2 H,
2,72 (t) 2 H,
3,04 (dt) 2 H,
3,17 (dt) 2 H,
4,25 (t) 1 H,
7,15 - 7,7 (m) 11 H, 2 H austauschbar mit $D_2O$,,
8,02 (m) 2 H, austauschbar mit $D_2O$,
9,06 (s) 1 H,
14,6 (breit) 2 H, austauschbar mit $D_2O$, ppm.

Beispiel 32

$N^1$-[3-Hydroxy-3-(4-fluorphenyl)-3-(pyridin-2-yl)propyl]-$N^2$-[3-(1H-imidazol-4-yl)propyl]guanidin

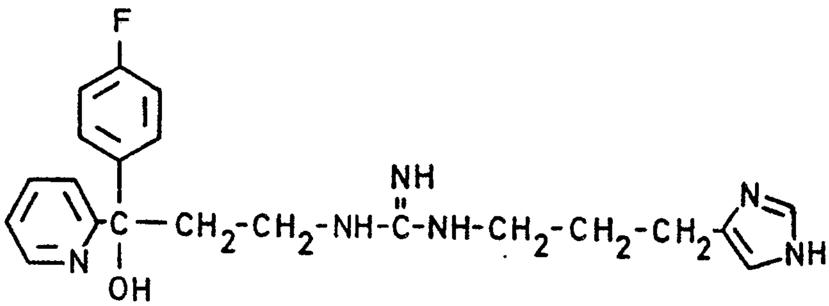

a) $N^1$-Benzoyl-$N^2$-[3-hydroxy-3-(4-fluorphenyl)-3-(pyridin-2-yl)propyl]-$N^3$-[ -(1H-imidazol-4-yl)propyl]-thioharnstoff

3,69 g (15 mmol) 3-Hydroxy-3-(4-fluorphenyl)-3-(pyridin-2-yl)propylamin und 2,45 g (15 mmol) Benzoylisothiocyanat werden in 150 ml Chloroform 40 min unter Rückfluß erhitzt. Anschließend wird das Lösungsmittel i. Vak. abdestilliert und der Rückstand mit Ether zur Kristallisation gebracht.
Ausbeute: 5,4 g (88 %) farbloser Feststoff, der nach Umkristallisation aus Ethanol/Wasser bei 138-139 °C schmilzt.
$C_{22}H_{20}FN_3O_2S$ (409,5)
Analyse:
Ber.: C 64,53 H 4,92 N 10,26
Gef.: C 64,70 H 4,91 N 10,18

b) N-[3-Hydroxy-3-(4-fluorphenyl)-3-(pyridin-2-yl)propyl]thioharnstoff

3,07 g (7,5 mmol) $N^1$-Benzoyl-$N^2$-[3-hydroxy-3-(4-fluorphenyl)-3-(pyridin-2-yl)propyl]-$N^3$-[ -(1H-imidazol-4-yl)propyl]guanidin werden mit 2,1 g Kaliumcarbonat in einer Mischung aus 30 ml Wasser und 70 ml Methanol 1 h unter Rückfluß erhitzt. Beim Einengen i. Vak. fallen 1,92 g (84 %) eines farblosen Feststoffes aus, der nach Umkristallisation aus Ethanol/Wasser bei 148-149 °C schmilzt.
$C_{15}H_{16}FN_3OS$ (305,4)
Analyse:
Ber.: C 59,00 H 5,28 N 13,76

Gef.: C 59,20 H 5,33 N 13,71

c) N-[3-Hydroxy-3-(4-fluorphenyl)-3-(pyridin-2-yl)propyl]-S-methyl-isothiuroniumiodid

1,53 g (5 mmol) N-[3-Hydroxy-3-(4-fluorphenyl)-3-(pyridin-2-yl)propyl]thioharnstoff werden 12 h mit 0,4 ml Methyliodid bei Raumtemperatur gerührt. Nach dem Eindampfen krsitallisiert das Isothiuroniumiodid beim Verreiben mit Ether und wenig Ethanol.
Ausbeute: 1,79 g (84 %). Schmp. nach Kristallisation aus Aceton/Ether und Trocknung der Kristalle bei 60 °C/0,05: 114 - 115 °C.
$C_{16}H_{18}FN_3OS$ x HI (447,3)
Analyse:
Ber.: C 42,96 H 4,28 N 9,39
Gef.: C 42,58 H 4,25 N 9,23
$^1$H-NMR-Daten:
(DMSO-$d_6$, TMS als interner Standard)
$\delta$ = 2,55 (s) 3 H,
2,62 (m) 1 H,
2,77 (m) 1 H,
3,23 (m) 2 H,
6,21 (s) 1 H, austauschbar mit $D_2O$,
7,14 (dd) 2 H,
7,29 (dd) 1 H,
7,51 (dd) 2 H,
7,64 (d) 1 H,
7,82 (dd) 1 H,
8,54 (d) 1 H,
9,0 (breit) 2 H, austauschbar mit $D_2O$,
9,25 (breit) 1 H, austauschbar mit $D_2O$, ppm.

d) $N^1$-[3-Hydroxy-3-(4-fluorphenyl)-3-(pyridin-2-yl)propyl]-$N^2$-[3-(1H-imidazol-4-yl)propyl]guanidin

1,5 g (3,5 mmol) N-[3-Hydroxy-3-(4-fluorphenyl)-3-(pyridin-2-yl)propyl]-S-methyl-isothiuroniumiodid werden mit 0,48 g (3,8 mmol) 3-(1H-Imidazol-4-yl)propylamin 5 h in 50 ml Pyridin bei 80 °C gerührt. Nach dem Eindampfen des Reaktionsansatzes i. Vak. wird das Produkt durch präparative Schichtchromatographie an Kieselgel 60 $PF_{254}$ gipshaltig isoliert und gereinigt (Fließmittel" Chloroform/Methanol, 90/10, Ammoniakatmosphäre).
Ausbeute: 1,0 g (55 %) des Hydroiodides in Form eines amorphen Feststoffes.
$C_{21}H_{25}FN_6O$ x HI (524,4)
MS (FAB-Methode): m/z (rel. Int. [%]) = 397 ($[M+H]^+$, 89), 230 (66), 212 (22), 200 (42),185 (13), 152 (11), 151 (15), 109 (100), 100 (44), 95 (12).
$^1$H-NMR-Daten:
(DMSO-$d_6$, TMS als interner Standard)
$\delta$ = 1,75 (m) 2 H,
2,5 - 2,9 (m) 4 H,
3,07 (m) 2 H,
3,14 (dt) 2 H,
6,22 (s) 1 H, austauschbar mit $D_2O$,
6,91 (s) 1 H,
7,11 (dd) 2 H,
7,27 (dd) 1 H,
7,4 (m) 4 H, austauschbar mit $D_2O$,
7,52 (dd) 2 H,
7,64 (d) 1 H,
7,78 (dd) 1 H,
7,81 (s) 1 H,
8,53 (d) 1 H, ppm.

Beispiel 33

N[1]-[3-(4-Methoxyphenyl)-3-(pyridin-2-yl)propyl]-N[2]-[3-(1H-imidazol-4-yl)propyl]guanidin

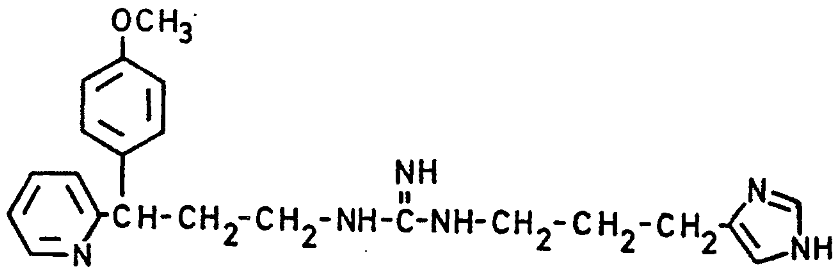

a) N-[3-(4-Methoxyphenyl)-3-(pyridin-2-yl)propyl]-S-methyl-isothiuroniumiodid

Die Herstellung erfolgt ausgehend von 3,63 g (15 mmol) 3-(4-Methoxyphenyl)-3-(pyridin-2-yl)propylamin analog Beispiel 32 a-c. Das Isothiuroniumiodid kristallisiert beim Verreiben des zunächst erhaltenen Schaumes mit Ether.
Ausbeute: 3,2 g (72 %) farblose Kristalle vom Schmp. 123 - 125 °C (Aceton/Ether).
$C_{17}H_{21}N_3OS$ x HI (443,4)
Analyse:
Ber.: C 46,06 H 5,00 N 9,48
C 45,93 H 5,00 N 9,44
$^1$H-NMR-Daten:
($DMSO-d_6$, TMS als interner Standard)
$\delta$ = 2,2 - 2,75 (m) 2 H,
2,59 (s) 3 H,
3,0 - 3,4 (m) 2 H,
3,72 (s) 3 H,
4,01 (t) 1 H,
6,86 (d) 2 H,
7,05 - 7,45 (m) 2 H,
7,25 (d) 2 H,
7,72 (dd) 1 H,
8,61 (d) 1 H,
9,2 (breit) 3 H, austauschbar mit $D_2O$, ppm.

b) N[1]-[3-(4-Methoxyphenyl)-3-(pyridin-2-yl)propyl]-N[2]-[3-(1H-imidazol-4-yl)propyl]guanidin

2,66 g (6 mmol) N-[3-(4-Methoxyphenyl)-3-(pyridin-2-yl)propyl]-S-methyl-isothiuroniumiodid werden mit 0,8 g (6,4 mmol) 3-(1H-Imidazol-4-yl)propylamin in 50 ml Acetonitril unter Rückfluß erhitzt. Nach dem Eindampfen des Reaktionsansatzes i. Vak. wird das Produkt durch präparative Schichtchromatographie analog Beispiel 32 d) isoliert.
Ausbeute: 1,96 g (63 %) des Hydroiodides in Form eines amorphen Feststoffes.
$C_{22}H_{28}N_6O$ x HI (520,4)
MS (FAB-Methode): m/z (rel. Int. [%]) = 393 ([M+H]$^+$, 71), 226 (100), 199 (10), 198 (9), 184 (11), 167 (12), 118 (12), 109 (58), 100 (36).
$^1$H-NMR-Daten:
($DMSO-d_6$, TMS als interner Standard)
$\delta$ = 1,75 (m) 2 H,
2,19 (m) 1 H,
2,45 (m) 1 H,
2,51 (t) 2 H,
3,03 (dt) 2 H,
3,17 (dt) 2 H,
3,70 (s) 3 H,
4,11 (t) 1 H,
6,80 (m) 1 H,

6,84 (d) 2 H,
7,18 - 7,27 (m) 4 H,
7,4 (breit) 4 H, austauschbar mit $D_2O$,
7,52 (d) 1 H,
7,69 (dd) 1 H,
8,51 (d) 1 H
11,85 (breit) 1 H, austauschbar mit $D_2O$, ppm.

Beispiel 34

$N^1$-[3-(4-Hydroxyphenyl)-3-(pyridin-2-yl)propyl]-$N^2$-[3-(1H-imidazol-4-yl)propyl]guanidin

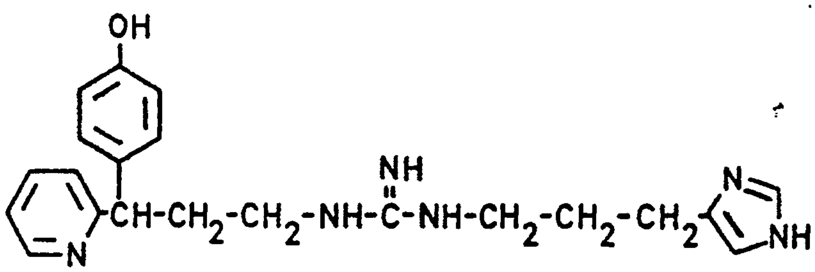

a) $N^1$-Benzoyl-$N^2$-[3-(1H-imidazol-4-yl)propyl]-$N^3$-[3-(4-methoxyphenyl)-3-(pyridin-2-yl)propyl]guanidin

Die Herstellung erfolgt analog Beispiel 14 a) ausgehend von 1,21 g (5 mmol) 3-(4-Methoxyphenyl)-3-(pyridin-2-yl)propylamin.
Ausbeute: 1,19 g (48 %) farbloser amorpher Feststoff (Schaum).
$C_{29}H_{32}N_6O_2$ (496,6)
$^1H$-NMR-Daten:
($CDCl_3$, TMS als interner Standard)
$\delta$ = 1,95 (m) 2 H,
2,29 (breit) 1 H,
2,5 - 2,75 (m) 3 H,
3,1 - 4,1 (breit) 4 H,
3,76 (s) 3 H,
4,15 (dd) 1 H,
6,72 (s) 1 H,
6,82 (d) 2 H,
7,0 - 7,75 (m) 9 H,
8,12 (d) 2 H,
8,55 (d) 1 H
10,75 (breit) 1 H, austauschbar mit $D_2O$, ppm.

b) $N^1$-[3-(4-Hydroxyphenyl)-3-(pyridin-2-yl)propyl]-$N^2$-[3-(1H-imidazol-4-yl)propyl]guanidin

0,6 g (1,2 mmol) $N^1$-Benzoyl-$N^2$-[3-(1H-imidazol-4-yl)propyl]-$N^3$-[3-(4-methoxyphenyl)-3-(pyridin-2-yl)-propyl]guanidin werden 10 h in 48prozentiger wäßriger Bromwasserstoffsäure unter Rückfluß erhitzt. Die saure Lösung wird mit Wasser verdünnt, 4mal mit Ether extrahiert und i. Vak. zur Trockne eingedampft. Nach Trocknung im Hochvakuum verbleiben 0,63 g (85 %) des Trihydrobromides in Form eines hygroskopischen amorphen Feststoffes.

Alternativ kann $N^1$-[3-(4-Hydroxyphenyl)-3-(pyridin-2-yl)propyl]-$N^2$-[3-(1H-imidazol-4-yl)propyl]guanidin nach folgendem Verfahren hergestellt werden:
0,9 g (1,7 mmol) $N^1$-[3-(4-Methoxyphenyl)-3-(pyridin-2-yl)propyl]-$N^2$-[3-(1H-imidazol-4-yl propyl]guanidin-hydroiodid (Beispiel 33) werden mit ethanolischer Pikrinsäurelösung in das Pikrat und anschließend durch Extraktion der Pikrinsäure aus einer salzsauren Lösung mit Ether und Eindampfen der wäßrigen Lösung i. Vak. in das Trihydrochlorid übergeführt. Der erhaltene amorphe Feststoff wird 10 h in 48prozentiger wäßriger Bromwasserstoffsäure unter Rückfluß erhitzt. Nach dem Eindampfen des Reaktionsansatzes i. Vak.

und Trocknung des Rückstandes im Hochvakuum verbleiben 0,84 g (84 %) des Trihydrobromides in Form eines hygroskopischen amorphen Feststoffes.
$C_{21}H_{26}N_6O$ x 3HBr (621,2)
MS (FAB-Methode): m/z (rel. Int. [%]) = 379 ([M+H]$^+$, 86), 213 (14), 212 (87), 195 (15), 189 (11), 168 (14), 167 (16), 157 (17), 151 (14), 131 (12), 126 (13), 118 (15), 117 (11) 115 (45), 110 (22), 109 (100), 107 (15), 100 (44), 95 (29).
$^1$H-NMR-Daten:
(DMSO-$d_6$, TMS als interner Standard)
δ = 1,82 (m) 2 H,
2,25 - 2,65 (m) 2 H,
2,70 (t) 2 H,
3,10 (dt) 2 H,
3,19 (dt) 2 H,
4,43 (t) 1 H,
6,75 (d) 2 H,
7,24 (d) 2 H,
7,43 (m) 2 H, austauschbar mit $D_2O$
7,51 (s) 1 H,
7,58 (m) 2 H, austauschbar mit $D_2O$,
7,79 (dd) 1 H,
7,99 (d) 1 H,
8,40 (dd) 1 H,
8,78 (d) 1 H,
9,09 (s) 1 H,
9,5 (breit) 1 H, austauschbar mit $D_2O$,
14,06 (breit) 1 H, austauschbar mit $D_2O$,
14,24 (breit) 1 H, austauschbar mit $D_2O$, ppm.

**Patentansprüche**

**Patentansprüche für folgende Vertragsstaaten : AT, BE, CH, DE, FR, GB, IT, LI, LU, NL, SE**

1. Imidazolylguanidinderivate der allgemeinen Formel I

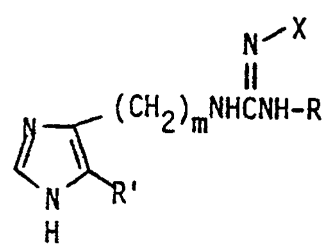

(I)

in der R die Gruppierung

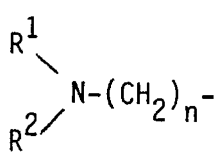

bedeutet, wobei $R^1$ für eine unsubstituierte oder eine ein- bis dreifach mit $C_1$-$C_3$-Alkyl, $C_1$-$C_3$-Alkoxy, Halogen, Trifluormethyl und/oder Hydroxy substituierte Phenylgruppe oder einen unsubstituierten oder einen ein- bis dreifach mit $C_1$-$C_3$-Alkyl, $C_1$-$C_3$-Alkoxy und/oder
Halogen substituierten Pyridinring steht, $R^2$ für ein Wasserstoffatom, eine $C_1$-$C_3$-Alkylgruppe, eine gegebenenfalls ein- bis dreifach mit $C_1$-$C_3$-Alkyl, $C_1$-$C_3$-Alkoxy, Halogen, Trifluormethyl und/oder

Hydroxy substituierte Phenylgruppe, eine unsubstituierte oder eine ein- bis dreifach mit $C_1$-$C_3$-Alkyl, $C_1$-$C_3$-Alkoxy, Halogen und/oder Trifluormethyl substituierte Benzylgruppe oder eine unsubstituierte oder eine ein- bis dreifach mit $C_1$-$C_3$-Alkyl, $C_1$-$C_3$-Alkoxy und/oder Halogen substituierte Thiophenylmethyl-, Furanylmethyl- oder Pyridylmethylgruppe steht und n den Wert 2, 3 oder 4, hat, oder

in der R die Gruppierung

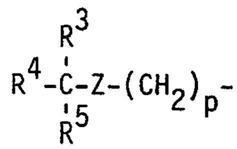

bedeutet, worin $R^3$ für eine unsubstituierte oder eine ein- bis dreifach mit $C_1$-$C_3$-Alkyl, $C_1$-$C_3$-Alkoxy, Halogen, Trifluormethyl und/oder Hydroxy substituierte Phenylgruppe oder einen unsubstituierten oder einen ein- bis dreifach mit $C_1$-$C_3$-Alkyl, $C_1$-$C_3$-Alkoxy und/oder Halogen substituierten Pyridinring steht, $R^4$ ein Wasserstoffatom oder eine gegebenenfalls ein- bis dreifach mit $C_1$-$C_3$-Alkyl, $C_1$-$C_3$-Alkoxy, Halogen, Trifluormethyl und/oder Hydroxy substituierte Phenylgruppe bedeutet, $R^5$ für ein Wasserstoffatom oder eine Methyl- oder Hydroxygruppe und Z für eine Einfachbindung oder ein Sauerstoffatom stehen und p den Wert 2 oder 3 hat,

X ein Wasserstoffatom oder eine Benzoylgruppe bedeutet, m den Wert 2 oder 3 hat und R' ein Wasserstoffatom oder eine Methylgruppe bedeutet,

sowie die physiologisch annehmbaren Salze davon.

2. Imidazolylguanidinderivate nach Anspruch 1, dadurch **gekennzeichnet,** daß R für die Gruppierung

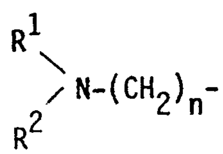

steht, wobei $R^1$ für einen unsubstituierten oder ein- bis dreifach mit $C_1$-$C_3$-Alkyl, $C_1$-$C_3$-Alkoxy und/oder Halogen substituierten Pyridinring steht, $R^2$ eine gegebenenfalls ein- bis dreifach mit $C_1$-$C_3$-Alkyl, $C_1$-$C_3$-Alkoxy, Halogen und/oder Trifluormethyl substituierte Benzylgruppe oder eine unsubstituierte oder ein- bis dreifach mit $C_1$-$C_3$-Alkyl, $C_1$-$C_3$-Alkoxy und/oder Halogen substituierte Thiopenylmethyl-, Furanylmethyl- oder Pyridylmethylgruppe bedeutet, n den Wert 2, 3 oder 4 hat, X und R' jeweils für ein Wasserstoffatom stehen und m den Wert 3 hat.

3. Imidazolylguanidinderivate nach Anspruch 1, dadurch **gekennzeichnet,** daß R für die Gruppierung

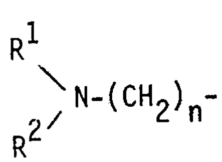

steht, wobei $R^1$ für einen unsubstituierten oder ein- bis dreifach mit $C_1$-$C_3$-Alkyl, $C_1$-$C_3$-Alkoxy, Halogen, Trifluormethyl und/oder Hydroxy substituierten Phenylring steht, $R^2$ eine unsubstituierte oder ein- bis dreifach mit $C_1$-$C_3$-Alkyl, $C_1$-$C_3$-Alkoxy und/oder Halogen substituierte Thiophenylmethyl-, Furanylmethyl- oder Pyridylmethylgruppe bedeutet, n den Wert 2, 3 oder 4 hat, X und R' jeweils für ein Wasserstoffatom stehen und m den Wert 3 hat.

4. Imidazolylguanidinderivate nach Anspruch 1, dadurch **gekennzeichnet,** daß R für die Gruppierung

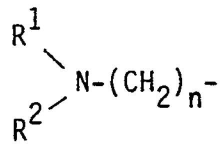

steht, worin $R^1$ für einen unsubstituierten oder ein- bis dreifach mit $C_1$-$C_3$-Alkyl, $C_1$-$C_3$-Alkoxy und/oder Halogen substituierten Pyridinring und $R^2$ für ein Wasserstoffatom stehen, n den Wert 2, 3 oder 4 hat, X und R' jeweils für ein Wasserstoffatom stehen und m den Wert 3 hat.

5. Imidazolylguanidinderivate nach Anspruch 1, dadurch **gekennzeichnet,** daß R für die Gruppierung

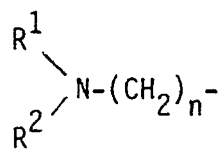

steht, wobei $R^1$ für einen unsubstituierten oder ein- bis dreifach mit $C_1$-$C_3$-Alkyl, $C_1$-$C_3$-Alkoxy und/oder Halogen substituierten Pyridinring und $R^2$ für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe steht, n den Wert 2, 3 oder 4 hat, X und R' jeweils für ein Wasserstoffatom stehen und m den Wert 3 hat.

6. Imidazolylguanidinderivate nach Anspruch 1, dadurch **gekennzeichnet,** daß R für die Gruppierung

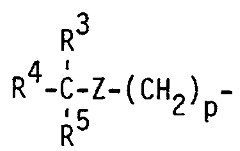

steht, worin $R^3$ und $R^4$, die gleich oder verschieden sein können, jeweils für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe stehen, $R^5$ für ein Wasserstoffatom oder eine Methylgruppe steht und Z für eine Einfachbindung steht, p den Wert 2 oder 3 hat, X und R' jeweils für ein Wasserstoffatom stehen und m den Wert 3 hat.

7. Imidazolylguanidinderivate nach Anspruch 1, dadurch **gekennzeichnet,** daß R für die Gruppierung

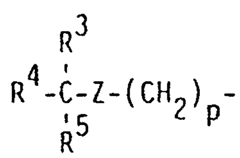

steht, worin $R^3$ für einen unsubstituierten oder ein- bis dreifach mit $C_1$-$C_3$-Alkyl, $C_1$-$C_3$-Alkoxy und/oder Halogen substituierten Pyridinring und $R^4$ für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe stehen, $R^5$ für ein Wasserstoffatom oder eine Methyl- oder Hydroxygruppe steht, Z eine Einfachbindung bedeutet, p den Wert 2 oder 3 hat, X für ein Wasserstoffatom und R' für ein Wasserstoffatom oder eine Methylgruppe stehen und m den Wert 2 oder 3 hat.

8. Imidazolylguanidinderivate nach Anspruch 1, dadurch **gekennzeichnet,** daß R für die Gruppierung

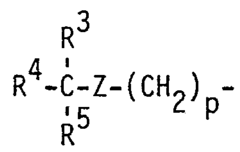

steht, worin $R^3$ für einen unsubstituierten oder ein- bis dreifach mit $C_1$-$C_3$-Alkyl, $C_1$-$C_3$-Alkoxy und/oder Halogen substituierten Pyridinring steht, $R^4$ und $R^5$ jeweils für ein Wasserstoffatom stehen, Z eine Einfachbindung bedeutet und p den Wert 2, 3 oder 4 hat, X und R' jeweils für ein Wasserstoffatom stehen und m den Wert 3 hat.

**9.** Imidazolylguanidinderivate nach Anspruch 1, dadurch **gekennzeichnet,** daß R für die Gruppierung

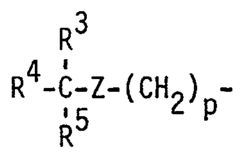

steht, worin $R^3$ und $R^4$, die gleich oder verschieden sein können, jeweils für einen unsubstituierten oder ein- bis dreifach mit $C_1$-$C_3$-Alkyl, $C_1$-$C_3$-Alkoxy, Halogen, Trifluormethyl und/oder Hydroxy substituierten Phenylring stehen, $R^5$ ein Wasserstoffatom und Z ein Sauerstoffatom bedeuten, p den Wert 2 oder 3 hat, X und R' jeweils für ein Wasserstoffatom stehen und m den Wert 3 hat.

**10.** $N^1$-[3-(3,4-Dichlorphenyl)-3-(pyridin-2-yl)propyl]-$N^2$-[3-(1H-imidazol-4-yl)propyl]guanidin und die physiologisch annehmbaren Salze davon.

**11.** $N^1$-[3-(3,4-Difluorphenyl)-3-(pyridin-2-yl)propyl]-$N^2$-[3-(1H-imidazol-4-yl)propyl]guanidin und die physiologisch annehmbaren Salze davon.

**12.** $N^1$-[3(4-Fluorphenyl)-3-phenylpropyl]-$N^2$-[3-(1H-imidazol-4-yl)-propyl]guanidin und die pyhsiologisch annehmbaren Salze davon.

**13.** $N^1$[3-(2,4-Dichlorphenyl)-3-(pyridin-2-yl)propyl]-$N^2$-[3-(1H-imidazol-4-yl)propyl]guanidin und die physiologisch annehmbaren Salze davon.

**14.** $N^1$-[3-(3-Chlorphenyl)-3-(pyridin-2-yl)propyl]-$N^2$-[3-(1H-imidazol-4-yl)propyl]guanidin und die physiologisch annehmbaren Salze davon.

**15.** $N^1$-[3-(3-Fluorphenyl)-3-(pyridin-3-yl)propyl]-$N^2$-[3-(1H-imidazol-4-yl)propyl]guanidin und die physiologisch annhembaren Salze davon.

**16.** $N^1$-[3-(3,5-Difluorphenyl)-3-(pyridin-2-yl)propyl]-$N^2$-[3-(1H-imidazol-4-yl)propyl]guanidin und die physiologisch annehmbaren Salze davon.

**17.** Verfahren zur Herstellung von Imidazolylguanidinderivaten nach den Ansprüchen 1 bis 16 sowie der physiologisch annehmbaren Salze davon, dadurch **gekennzeichnet,** daß man

a) zur Herstellung von Verbindungen der allgemeinen Formel I, in denen R, R' und m wie in Anspruch 1 definiert sind und X für eine Benzoylgruppe steht,

$a_1$) eine Verbindung der allgemeinen Formel II

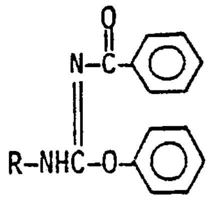

(II)

in der R wie in Anspruch 1 definiert ist, mit einer Verbindung der allgemeinen Formel III

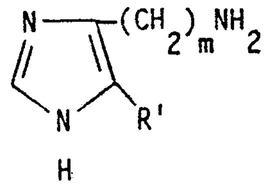

(III)

in der R' und m wie in Anspruch 1 definiert sind, zu einer Verbindung der allgemeinen Formel I umsetzt oder

$a_2$) eine Verbindung der allgemeinen Formel IV

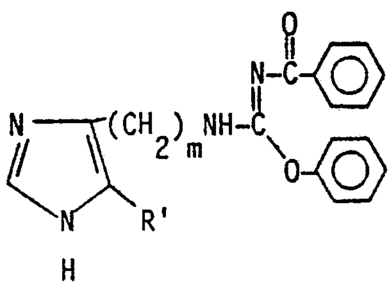

(IV)

in der R' und m wie in Anspruch 1 definiert sind, mit einer Verbindung der allgemeinen Formel V

$R\text{-}NH_2$ (V)

in der R wie in Anspruch 1 definiert ist, zu einer Verbindung der allgemeinen Formel I umsetzt oder

b) zur Herstellung von Verbindungen der allgemeinen Formel I, in der R, R' und m wie in Anspruch 1 definiert sind und X für ein Wasserstoffatom steht,

$b_1$) eine Verbindung der allgemeinen Formel Ia

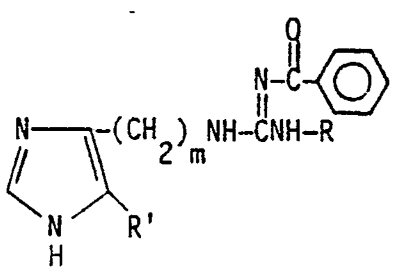

(Ia)

in der R, R' und m wie in Anspruch 1 definiert sind, hydrolysiert oder

$b_2$) eine Verbindung der allgemeinen Formel VI

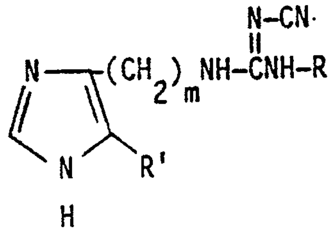

(VI)

in der R, R' und m wie in Anspruch 1 definiert sind, mit Hilfe einer Säure zu einer Verbindung der allgemeinen Formel I hydrolysiert oder

$b_3$) eine Verbindung der allgemeinen Formel VII

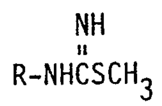

(VII)

in der R wie in Anspruch 1 definiert ist, mit einer Verbindung der allgemeinen Formel III

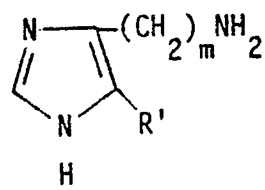

(III)

in der R' und m wie in Anspruch 1 definiert sind, zu einer Verbindung der allgemeinen Formel I umsetzt oder

$b_4$) eine Verbindung der allgemeinen Formel VIII

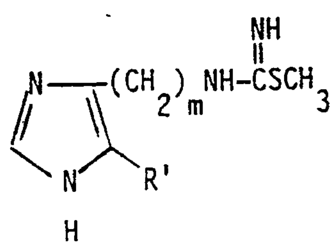

(VIII)

in der R' und m wie in Anspruch 1 definiert sind, mit einer Verbindung der allgemeinen Formel V

$R\text{-}NH_2$ (V)

in der R wie in Anspruch 1 definiert ist, zu einer Verbindung der allgemeinen Formel I umsetzt

und daß man gegebenenfalls die nach a) und b) erhaltenen Verbindungen in an sich bekannter Weise in ihr physiologisch annehmbares Salz umwandelt.

18. Arzneimittel, dadurch **gekennzeichnet,** daß es eine Verbindung nach den Ansprüchen 1 bis 16 und mindestens einen inerten, pharmazeutisch annehmbaren Träger oder ein inertes, pharmazeutisch annehmbares Verdünnungsmittel enthält.

**Patentansprüche für folgende Vertragsstaaten : ES, GR**

1. Verfahren zur Herstellung von Imidazolylguanidinderivaten der allgemeinen Formel I

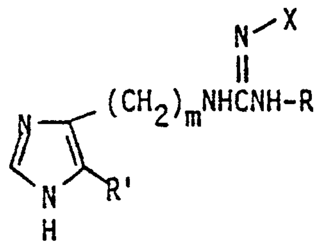

(I)

in der R die Gruppierung

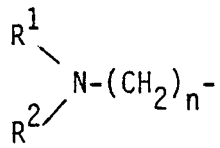

bedeutet, wobei $R^1$ für eine unsubstituierte oder eine ein- bis dreifach mit $C_1$-$C_3$-Alkyl, $C_1$-$C_3$-Alkoxy, Halogen, Trifluormethyl und/oder Hydroxy substituierte Phenylgruppe oder einen unsubstituierten oder einen ein- bis dreifach mit $C_1$-$C_3$-Alkyl, $C_1$-$C_3$-Alkoxy und/oder
Halogen substituierten Pyridinring steht, $R^2$ für ein Wasserstoffatom, eine $C_1$-$C_3$-Alkylgruppe, eine gegebenenfalls ein- bis dreifach mit $C_1$-$C_3$-Alkyl, $C_1$-$C_3$-Alkoxy, Halogen, Trifluormethyl und/oder Hydroxy substituierte Phenylgruppe, eine unsubstituierte oder eine ein- bis dreifach mit $C_1$-$C_3$-Alkyl, $C_1$-$C_3$-Alkoxy, Halogen und/oder Trifluormethyl substituierte Benzylgruppe oder eine unsubstituierte oder eine ein- bis dreifach mit $C_1$-$C_3$-Alkyl, $C_1$-$C_3$-Alkoxy und/oder Halogen substituierte Thiophenylmethyl-, Furanylmethyl- oder Pyridylmethylgruppe steht und n den Wert 2, 3 oder 4, hat, oder

in der R die Gruppierung

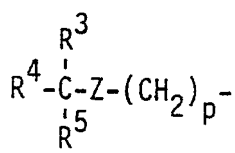

bedeutet, worin $R^3$ für eine unsubstituierte oder eine ein- bis dreifach mit $C_1$-$C_3$-Alkyl, $C_1$-$C_3$-Alkoxy, Halogen, Trifluormethyl und/oder Hydroxy substituierte Phenylgruppe oder einen unsubstituierten oder einen ein- bis dreifach mit $C_1$-$C_3$-Alkyl, $C_1$-$C_3$-Alkoxy und/oder
Halogen substituierten Pyridinring stet, $R^4$ ein Wasserstoffatom oder eine gegebenenfalls ein- bis dreifach mit $C_1$-$C_3$-Alkyl, $C_1$-$C_3$-Alkoxy, Halogen, Trifluormethyl und/oder Hydroxy substituierte Phenylgruppe bedeutet, $R^5$ für ein Wasserstoffatom oder eine Methyl- oder Hydroxygruppe und Z für eine Einfachbindung oder ein Sauerstoffatom stehen und p den Wert 2 oder 3 hat,

X ein Wasserstoffatom oder eine Benzoylgruppe bedeutet, m den Wert 2 oder 3 hat und R' ein Wasserstoffatom oder eine Methylgruppe bedeutet, sowie der physiologisch annehmbaren Salze davon, dadurch **gekennzeichnet,** daß man

a) zur Herstellung von Verbindungen der allgemeinen Formel I, in denen R, R' und m wie oben definiert sind und X für eine Benzoylgruppe steht,
 $a_1$) eine Verbindung der allgemeinen Formel II

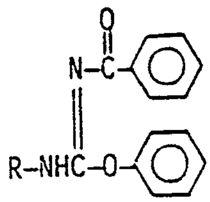

(II)

in der R die oben angegebene Bedeutung besitzt, mit einer Verbindung der allgemeinen Formel III

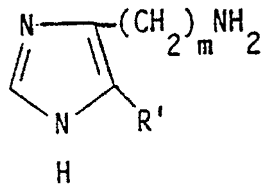

(III)

in der R' und m die oben angegebenen Bedeutungen besitzen, zu einer Verbindung der allgemeinen Formel I umsetzt oder

$a_2$) eine Verbindung der allgemeinen Formel IV

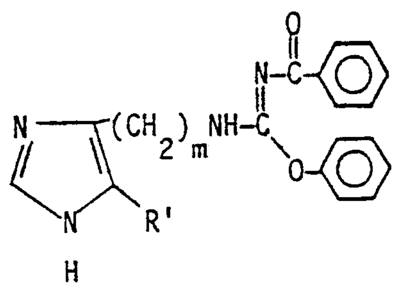

(IV)

in der R' und m die oben angegebenen Bedeutungen besitzen, mit einer Verbindung der allgemeinen Formel V

$R\text{-}NH_2$ (V)

in der R wie oben definiert ist, zu einer Verbindung der allgemeinen Formel I umsetzt oder

b) zur Herstellung von Verbindungen der allgemeinen Formel I, in der R, R' und m wie oben definiert sind und X für ein Wasserstoffatom steht,

$b_1$) eine Verbindung der allgemeinen Formel Ia

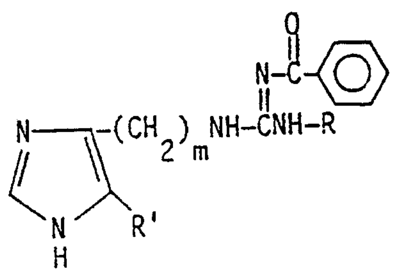

(Ia)

in der R, R' und m wie oben definiert sind, hydrolysiert oder

$b_2$) eine Verbindung der allgemeinen Formel VI

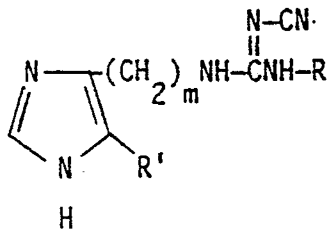

(VI)

in der R, R' und m die oben angegebenen Bedeutungen besitzen, mit Hilfe einer Säure zu einer Verbindung der allgemeinen Forme I hydrolysiert oder

$b_3$) eine Verbindung der allgemeinen Formel VII

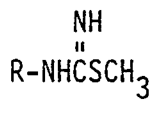

(VII)

in der R die oben angegebene Bedeutung besitzt, mit einer Verbindung der allgemeinen Formel III

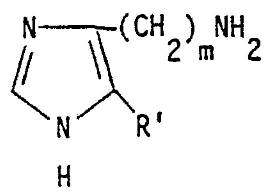

(III)

in der R' und m die oben angegebenen Bedeutungen haben, zu einer Verbindung der allgemeinen Formel I umsetzt oder

$b_4$) eine Verbindung der allgemeinen Formel VIII

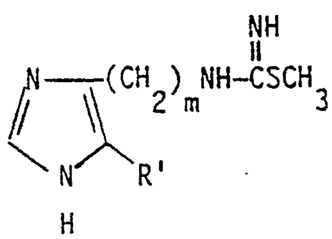

(VIII)

in der R' und m wie oben definiert sind, mit einer Verbindung der allgemeinen Formel V

$R\text{-}NH_2$ (V)

in der R die oben angegebene Bedeutung besitzt, zu einer Verbindung der allgemeinen Formel I umsetzt

und daß man gegebenenfalls die nach a) und b) erhaltenen Verbindungen in an sich bekannter Weise in ihr physiologisch annehmbares Salz umwandelt.

2. Verfahren nach Anspruch 1 zur Herstellung von Verbindungen, bei denen R für die Gruppierung

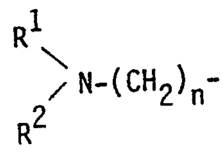

steht, wobei $R^1$ für einen unsubstituierten oder ein- bis dreifach mit $C_1$-$C_3$-Alkyl $C_1$-$C_3$-Alkoxy und/oder Halogen substituierten Pyridinring steht, $R^2$ eine gegebenenfalls ein- bis dreifach mit $C_1$-$C_3$-Alkyl, $C_1$-$C_3$-Alkoxy, Halogen und/oder Trifluormethyl substituierte Benzylgruppe oder eine unsubstituierte oder ein- bis dreifach mit $C_1$-$C_3$-Alkyl, $C_1$-$C_3$-Alkoxy und/oder Halogen substituierte Thiopenylmethyl-, Furanylmethyl- oder Pyridylmethylgruppe bedeutet, n den Wert 2, 3 oder 4 hat, X und R' jeweils für ein Wasserstoffatom stehen und m den Wert 3 hat.

**3.** Verfahren nach Anspruch 1 zur Herstellung von Verbindungen, bei denen R für die Gruppierung

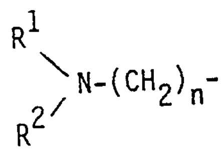

steht, wobei $R^1$ für einen unsubstituierten oder ein- bis dreifach mit $C_1$-$C_3$-Alkyl, $C_1$-$C_3$-Alkoxy, Halogen, Trifluormethyl und/oder Hydroxy substituierten Phenylring steht, $R^2$ eine unsubstituierte oder ein- bis dreifach mit $C_1$-$C_3$-Alkyl, $C_1$-$C_3$-Alkoxy und/oder Halogen substituierte Thiophenylmethyl-, Furanylmethyl- oder Pyridylmethylgruppe bedeutet, n den Wert 2, 3 oder 4 hat, X und R' jeweils für ein Wasserstoffatom stehen und m den Wert 3 hat.

**4.** Verfahren nach Anspruch 1 zur Herstellung von Verbindungen, bei denen R für die Gruppierung

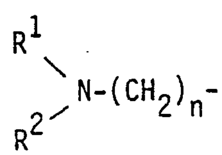

steht, worin $R^1$ für einen unsubstituierten oder ein- bis dreifach mit $C_1$-$C_3$-Alkyl, $C_1$-$C_3$-Alkoxy und/oder Halogen substituierten Pyridinring und $R^2$ für ein Wasserstoffatom stehen, n den Wert 2, 3 oder 4 hat, X und R' jeweils für ein Wasserstoffatom stehen und m den Wert 3 hat.

**5.** Verfahren nach Anspruch 1 zur Herstellung von Verbindungen, bei denen R für die Gruppierung

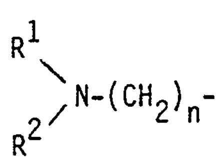

steht, wobei $R^1$ für einen unsubstituierten oder ein- bis dreifach mit $C_1$-$C_3$-Alkyl, $C_1$-$C_3$-Alkoxy und/oder Halogen substituierten Pyridinring und $R^2$ für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe steht, n den Wert 2, 3 oder 4 hat, X und R' jeweils für ein Wasserstoffatom stehen und m den Wert 3 hat.

**6.** Verfahren nach Anspruch 1 zur Herstellung von Verbindungen, bei denen R für die Gruppierung

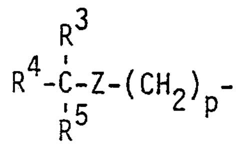

steht, worin $R^3$ und $R^4$, die gleich oder verschieden sein können, jeweils für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe stehen, $R^5$ für ein Wasserstoffatom oder eine Methylgruppe steht und Z für eine Einfachbindung steht, p den Wert 2 oder 3 hat, X und R' jeweils für ein Wasserstoffatom stehen und m den Wert 3 hat.

7. Verfahren nach Anspruch 1 zur Herstellung von Verbindungen, bei denen R für die Gruppierung

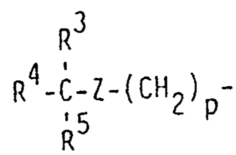

steht, worin $R^3$ für einen unsubstituierten oder ein- bis dreifach mit $C_1$-$C_3$-Alkyl, $C_1$-$C_3$-Alkoxy und/oder Halogen substituierten Pyridinring und $R^4$ für eine unsubstituierte oder ein- bis dreifach mit $C_1$-$C_3$-Alkyl, $C_1$-$C_3$-Alkoxy, Halogen, Trifluormethyl und/oder Hydroxy substituierte Phenylgruppe stehen, $R^5$ für ein Wasserstoffatom oder eine Methyl- oder Hydroxygruppe steht, Z eine Einfachbindung bedeutet, p den Wert 2 oder 3 hat, X für ein Wasserstoffatom und R' für ein Wasserstoffatom oder eine Methylgruppe stehen und m den Wert 2 oder 3 hat.

8. Verfahren nach Anspruch 1 zur Herstellung von Verbindungen, bei denen R für die Gruppierung

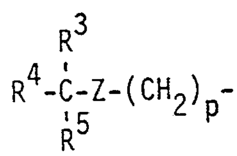

steht, worin $R^3$ für einen unsubstituierten oder ein- bis dreifach mit $C_1$-$C_3$-Alkyl, $C_1$-$C_3$-Alkoxy und/oder Halogen substituierten Pyridinring steht, $R^4$ und $R^5$ jeweils für ein Wasserstoffatom stehen, Z eine Einfachbindung bedeutet und p den Wert 2, 3 oder 4 hat, X und R' jeweils für ein Wasserstoffatom stehen und m den Wert 3 hat.

9. Verfahren nach Anspruch 1 zur Herstellung von Verbindungen, bei denen R für die Gruppierung

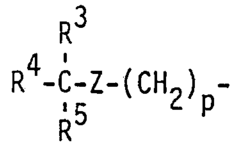

steht, worin $R^3$ und $R^4$, die gleich oder verschieden sein können, jeweils für einen unsubstituierten oder ein- bis dreifach mit $C_1$-$C_3$-Alkyl, $C_1$-$C_3$-Alkoxy, Halogen, Trifluormethyl und/oder Hydroxy substituierten Phenylring stehen, $R^5$ ein Wasserstoffatom und Z ein Sauerstoffatom bedeuten, p den Wert 2 oder 3 hat, X und R' jeweils für ein Wasserstoffatom stehen und m den Wert 3 hat.

**10.** Verfahren nach Anspruch 1 zur Herstellung von $N^1$[-3-(3,4-Dichlorphenyl)-3-(pyridin-2-yl)propyl]-$N^2$-[3-(1H-imidazol-4-yl)propyl]guanidin und der physiologisch annehmbaren Salze davon.

**11.** Verfahren nach Anspruch 1 zur Herstellung von $N^1$-[3-(3,4-Difluorphenyl)-3-(pyridin-2-yl)propyl]-$N^2$-[3-(1H-imidazol-4-yl)propyl]guanidin und der physiologisch annehmbaren Salze davon.

**12.** Verfahren nach Anspruch 1 zur Herstellung von $N^1$-[3-(4-Fluorphenyl)-3-phenylpropyl]-$N^2$-[3-(1H-imidazol-4-yl)propyl]guanidin und der physiologisch annehmbaren Salze davon.

**13.** Verfahren nach Anspruch 1 zur Herstellung von $N^1$[-3-(2,4-Dichlorphenyl)-3-(pyridin-2-yl)propyl]-$N^2$-[3-(1H-imidazol-4-yl)propyl]guanidin und der physiologisch annehmbaren Salze davon.

**14.** Verfahren nach Anspruch 1 zur Herstellung von $N^1$[-3-(3-Chlorphenyl)-3-(pyridin-2-yl)propyl]-$N^2$-[3-(1H-imidazol-4-yl)propyl]guanidin und der physiologisch annehmbaren Salze davon.

**15.** Verfahren nach Anspruch 1 zur Herstellung von $N^1$-[3-(4-Fluorphenyl)-3-(pyridin-3-yl)propyl]-$N^2$-[3-(1H-imidazol-4-yl)propyl]guanidin und der physiologisch annehmbaren Salze davon.

**16.** Verfahren nach Anspruch 1 zur Herstellung von $N^1$-[3-(3,5-Difluorphenyl)-3-(pyridin-2-yl)propyl]-$N^2$-[3-(1H-imidazol-4-yl)propyl]guanidin und der physiologisch annehmbaren Salze davon.

**Claims**
**Claims for the following Contracting States : AT, BE, CH, DE, FR, GB, IT, LI, LU, NL, SE**

**1.** Imidazolylguandine derivatives corresponding to the general formula I

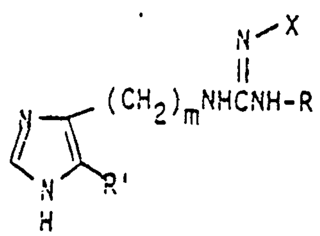

(I)

in which R denotes the group

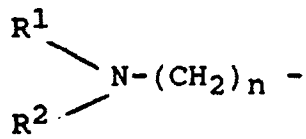

wherein $R^1$ stands for a phenyl group which is unsubstituted or mono- to tri- substituted with $C_1$-$C_3$-alkyl, $C_1$-$C_3$-alkoxy, halogen, trifluoromethyl and/or hydroxy or for a pyridine ring which is unsubstituted or mono- to tri- substituted with $C_1$-$C_3$-alkyl, $C_1$-$C_3$-alkoxy and/or halogen, $R^2$ stands for a hydrogen atom, a $C_1$-$C_3$-alkyl group, a phenyl group optionally mono- to tri- substituted with $C_1$-$C_3$-alkyl, $C_1$-$C_3$-alkoxy, halogen, trifluoromethyl and/or hydroxy, a benzyl group which is unsubstituted or mono- to try-substituted with $C_1$-$C_3$-alkyl, $C_1$-$C_3$-alkoxy, halogen and/or trifluoromethyl, or a thiophenylmethyl, furanylmethyl or pyridylmethly group, which groups are unsubstituted or mono- to tri- substituted with $C_1$-$C_3$-alkyl, $C_1$-$C_3$-alkoxy and/or halogen, and n has the value 2, 3 or 4
or in which
R denotes the group

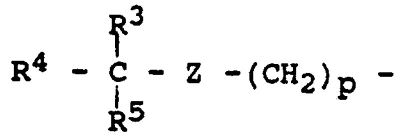

wherein $R^3$ stands for a phenyl group which is unsubstituted or mono- to tri- substituted with $C_1$-$C_3$-alkyl, $C_1$-$C_3$-alkoxy, halogen, trifluoromethly and/or hydyroxy or for a pyridine ring which is unsubstituted or mono- to tri- substituted with $C_1$-$C_3$-alkyl, $C_1$-$C_3$-alkoxy and/or halogen, $R^4$ stands for a hydrogen atom or for a phenyl group which is optionally mono- to tri- substituted with $C_1$-$C_3$-alkyl, $C_1$-$C_3$-alkoxy, halogen, trifluoromethly and/or hydroxy, $R^5$ stands for a hydrogen atom or a methyl or hydroxy group and Z stands for a single bond or an oxygen atom, and p has the value 2 or 3, and

X denotes a hydrogen atom or a benzoyl group, m has the value 2 or 3 and R' denotes a hydrogen atom or a methyl group,

and the physiologically acceptable salts thereof.

2. Imidazolyguanidine derivatives according to claim 1, characterised in that R stands for the group

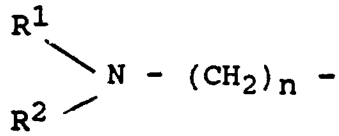

wherein $R^1$ stands for a pyridine ring which is unsubstituted or mono- to tri- substituted with $C_1$-$C_3$-alkyl, $C_1$-$C_3$-alkoxy and/or halogen, $R^2$ stands for a benzyl group optionally mono- to tri- subsituted with $C_1$-$C_3$-alkyl, $C_1$-$C_3$-alkoxy, halogen and/or trifluoromethyl or for a thiophenylmethyl, furanylmethyl or pyridylmethyl group, which groups are unsubstituted or mono- to tri- substituted with $C_1$-$C_3$-alkyl, $C_1$-$C_3$-alkoxy and/or halogen, n has the value 2, 3 or 4, X and R' each stand for a hydrogen atom and m has the value 3.

3. Imidazolyguanidine derivatives according to claim 1, characterised in that R stands for the group

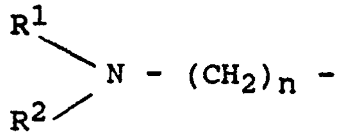

wherein $R^1$ stands for a phenyl ring which is unsubstituted or mono- to tri- substituted with $C_1$-$C_3$-alkly, $C_1$-$C_3$-alkoxy, halogen, trifluoromethyl and/or hydroxy, $R^2$ stands for a thiophenylmethyl, furanylmethyl or pyridylmethyl group, which groups are unsubstituted or mono- to tri- substituted with $C_1$-$C_3$-alkyl, $C_1$-$C_3$-alkoxy and/or halogen, n has the value 2, 3 or 4, X and R' each stand for a hydrogen atom and m has the value 3.

4. Imidazoylguanidine derivatives according to claim 1, characterised in that R stands for the group

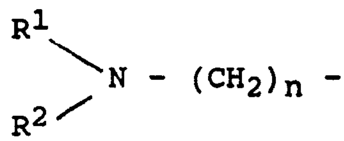

wherein $R^1$ stands for a pyridine ring which is unsubstituted or mono- to tri- substituted with $C_1$-$C_3$-alkyl, $C_1$-$C_3$-alkoxy and/or halogen and $R^2$ stands for a hydrogen atom, n has the value 2, 3 or 4, X and R' stand each for a hydrogen atom and m has the value 3.

**5.** Imidazolylguanidine derivatives according to claim 1, characterised in that R stands for the group

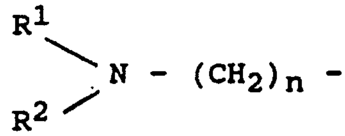

wherein $R^1$ stands for a pyridine ring which is unsubstituted or mono- to tri- substituted with $C_1$-$C_3$-alkyl, $C_1$-$C_3$-alkoxy and/or halogen and $R^2$ stands for a phenyl group which is unsubstituted or mono- to tri- substituted with $C_1$-$C_3$-alkyl, $C_1$-$C_3$-alkoxy, halogen, trifluoromethyl and/or hydroxy, n has the value 2, 3 or 4, X and R' stand each for a hydrogen atom and m has the value 3.

**6.** Imidazolylguanidine derivatives according to claim 1, characterised in that R stands for the group

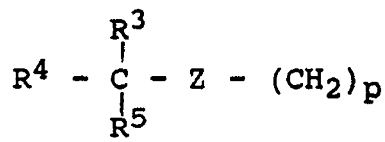

wherein $R^3$ and $R^4$, which may be identical or different, stand each for a phenyl group which is unsubstituted or mono- to tri- substituted with $C_1$-$C_3$-alkyl, $C_1$-$C_3$-alkoxy, halogen, trifluoromethyl and/or hydroxy, $R^5$ stands for a hydrogen atom or a methyl group and Z stands for a single bond, p has the value 2 or 3, X and R' stand each for a hydrogen atom and m has the value 3.

**7.** Imidazolylguanidine derivatives according to claim 1, characterised in that R stands for the group

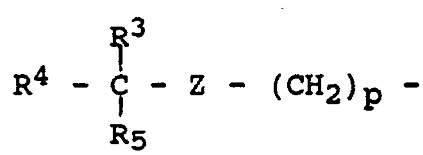

wherein $R^3$ stands for a pyridine ring which is unsubstituted or mono- to tri- substituted with $C_1$-$C_3$-alkyl, $C_1$-$C_3$-alkoxy and/or halogen and $R^4$ stands for a phenyl group which is unsubstituted or mono- to tri- substituted with $C_1$-$C_3$-alkyl, $C_1$-$C_3$-alkoxy, halogen, trifluoromethyl and/or hydroxy, $R^5$ stands for a hydrogen atom or a methyl or a hydroxy group, Z denotes a single bond, p has the value 2 or 3, X stands for a hydrogen atom and R' for a hydrogen atom or a methyl group and m has the value 2 or 3.

**8.** Imidazolylguanidine derivatives according to claim 1, characterised in that R stands for the group

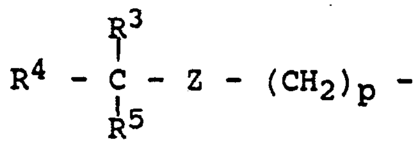

wherein $R^3$ stands for a pyridine ring which is unsubstituted or mono- to tri- substituted with $C_1$-$C_3$-alkyl, $C_1$-$C_3$-alkoxy and/or halogen, $R^4$ and $R^5$ stand each for a hydrogen atom, Z denotes a single bond and p has the value 2, 3 or 4, X and R' stand each for a hydrogen atom and m has the value 3.

9. Imidazolylguanidine derivatives according to claim 1, characterised in that R stands for the group

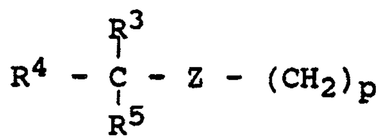

wherein $R^3$ and $R^4$, which may be identical or different, stand for a phenyl ring which unsubstituted or mono- to tri- substituted with $C_1$-$C_3$-alkyl, $C_1$-$C_3$-alkoxy, halogen, trifluoromethyl and/or hydroxy, $R^5$ denotes a hydrogen atom and Z an oxygen atom, p has the value 2 or 3, X and R' stand each for a hydrogen atom and m has the value 3.

10. $N^1$-[3-(3,4-dichlorophenyl)-3-(pyridin-2-yl)-propyl]-$N^2$-[3-1H-imidazol-4-yl)propyl]-guanidine and the physiologically acceptable salts thereof.

11. $N^1$-(3-(3,4-difluorophenyl)-3-(pyridin-2-yl)propyl]-$N^2$-[3-1H-imidazol-4-yl)propyl]guanidine and the physiologically acceptable salts thereof.

12. $N^1$-[3-(4-fluorophenyl)-3-phenylpropyl]-$N^2$-[3-1H-imidazol-4-yl)-propyl]guanidine and the physiologically acceptable salts thereof.

13. $N^1$[3-(2,4-dichlorophenyl)-3-(pyridin-2-yl)propyl]-$N^2$-[3-(1H-imidazol-4-yl)propyl]guanidine and the physiologically acceptable salts thereof.

14. $N^1$-[3-(3-chlorophenyl)-3(pyridin-2-yl)propyl]-$N^2$-[3-(1H-imidazol-4-yl)propyl]guanidine and the physiologically acceptable salts thereof.

15. $N^1$-(3-(4-fluorophenyl)-3-(pyridin-3-yl)propyl]-$N^2$-[3-(1H-imidazol-4-yl)propyl]guanidine and the physiologically acceptable salts thereof.

16. $N^1$-[3-(3,5-difluorophenyl)-3-(pyridin-2-yl)propyl]-$N^2$-[3-(1H-imidazol-4-yl)propyl]guanidine and the physiologically acceptable salts thereof.

17. A process for the preparation of imidazolylguanidine derivatives according to claims 1 to 16 and of the physiologically acceptable salts thereof, characterised in that

a) for the preparation of compounds corresponding to the general formula I in which R, R' and m have the meanings defined in claim 1 and X stands for a benzoyl group,
$a_1$) a compound corresponding to the general formula II

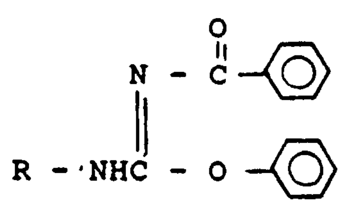

(II)

in which R has the meaning defined in claim 1 is reacted with a compound corresponding to the general formula III

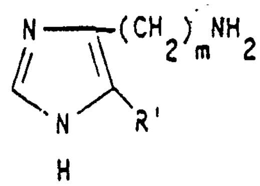

(III)

wherein R' and m have the meanings defined in claim 1 to form a compound corresponding to the general formula I or

$a_2$) a compound corresponding to the general formula IV

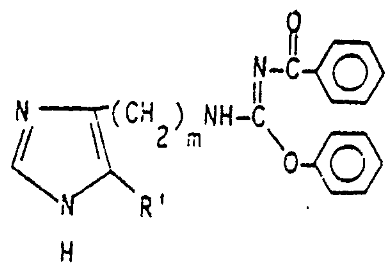

(IV)

in which R' and m have the meanings defined in claim 1 is reacted with a compound corresponding to the general formula V

$R - NH_2$ (V)

wherein R is defined as in claim 1 to form a compound corresponding to the general formula I or

b) for the preparation of compounds corresponding to the general formula I in which A, R' and m have the meanings defined in claim 1 and X stands for a hydrogen atom,

$b_1$) a compound corresponding to the general formula Ia

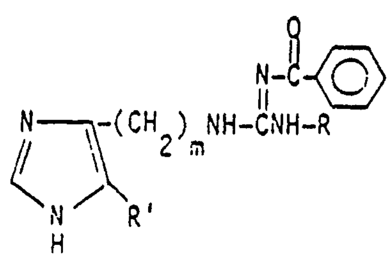

(Ia)

wherein R, R' and m have the meanings defined in claim 1 is hydrolysed or

$b_2$) a compound corrresponding to the general formula VI

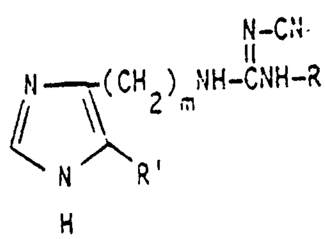

(VI)

wherein R, R' and m have the meanings defined in claim 1 is hydrolysed with an acid to form a

compound corresponding to the general formula I or

$b_3$) a compound corresponding to the general formula VII

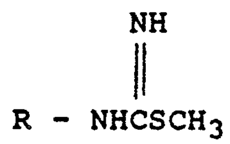

(VII)

in which R has the meaning defined in claim 1 is reacted with a compound corresponding to the general formula III

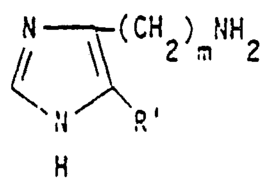

(III)

wherein R' and m have the meanings defined in claim 1 to form a compound corresponding to the general formula I or

$b_4$) a compound corresponding to the general formula VIII

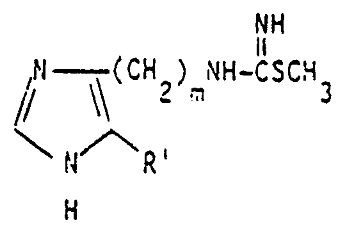

(VIII)

wherein R' and m have the meanings defined in claim 1 is reacted with a compound corresponding to the general formula V

$R - NH_2$ (V)

in which R has the meaning defined in claim 1 to form a compound corresponding to the general formula I and in that the compounds obtained according to a) and b) are optionally converted into their physiologically acceptable salts in known manner.

**18.** Pharmaceutical composition, characterised in that it contains a compound according to claims 1 to 16 and at least one inert, pharmaceutically acceptable carrier or an inert, pharmaceutically acceptable diluent.

**Claims for the following Contracting States : ES, GR**

**1.** A process for the preparation of imidazoylylguanidine derivatives corresponding to the general formula I

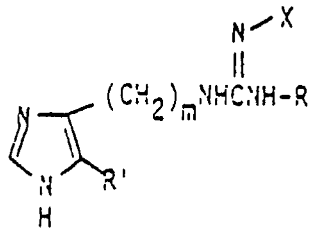

(I)

in which R denotes the group

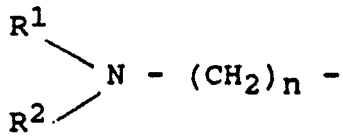

wherein $R^1$ stands for a phenyl group which is unsubstituted or mono- to tri- substituted with $C_1$-$C_3$-alkyl, $C_1$-$C_3$-alkoxy, halogen, trifluoromethyl and/or hydroxy or for a pyridine ring which is unsubstituted or mono- to tri- substituted with $C_1$-$C_3$-alkyl, $C_1$-$C_3$-alkoxy and/or halogen, $R^2$ stands for a hydrogen atom, a $C_1$ to $C_3$ alkyl group, a phenyl group optionally mono- to tri- substituted with $C_1$-$C_3$-alkyl, $C_1$-$C_3$-alkoxy, halogen, trifluoromethyl and/or hydroxy, a benzyl group which is unsubstituted or mono- to tri- substituted with $C_1$-$C_3$-alkyl, $C_1$-$C_3$-alkoxy, halogen and/or trifluoromethyl or for a thiophenylmethly, furanylmethyl or pyridylmethyl group, which groups are unsubstituted or mono- to trisubstituted with $C_1$-$C_3$-alkyl, $C_1$-$C_3$-alkoxy and/or halogen, and n has the value 2, 3 or 4,

or in which R denotes the group

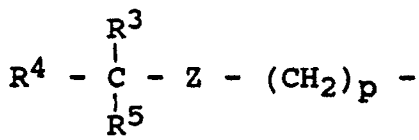

wherein $R^3$ stands for a phenyl group which is unsubstitued or mono- to tri- substituted with $C_1$-$C_3$-alkyl, $C_1$-$C_3$-alkoxy, halogen, trifluoromethyl and/or hydroxy or it stands for a pyridine ring which is unsubstituted or monoto tri- substituted with $C_1$-$C_3$-alkyl, $C_1$-$C_3$-alkoxy, and/or halogen, $R^4$ stands for a hydrogen atom or a phenyl group which is optionally mono- to tri- substituted with $C_1$-$C_3$-alkyl, $C_1$-$C_3$-alkoxy, halogen, trifluoromethyl and/or hydroxy, $R^5$ stands for a hydrogen atom or a methyl or hydroxy group and Z stands for a single bond or an oxygen atom and p has the value 2 or 3,

X denotes a hydrogen atom or a benzoyl group, m has the value 2 or 3 and R' denotes a hydrogen atom or a methyl group,
and the physiologically acceptable salts thereof, characterised in that

a) for the preparation of compounds corresponding to the general formula I in which R, R' and m have the meanings defined above and X stands for a benzoyl group,
$a_1$) a compound corresponding to the general formula II

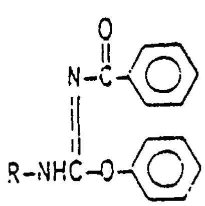

(II)

in which R has the meaning indicated above is reacted with a compound corresponding to the general formula III

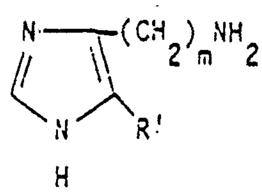

(III)

in which R' and m have the meanings indicated above to form a compound corresponding to the general formula I or

a₂) a compound corresponding to the general formula IV

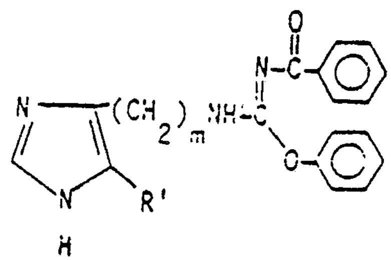

(IV)

in which $R^1$ and m have the meanings indicated above is reacted with a compound corresponding to the general formula V

$R - NH_2$ (V)

in which R has the meaning defined above to form a compound corresponding to the general formula I or

b) for the preparation of compounds corresponding to the general formula I in which R, R' and m have the meanings defined above and X stands for a hydrogen atom,

$b_1$) a compound corresponding to the general formula Ia

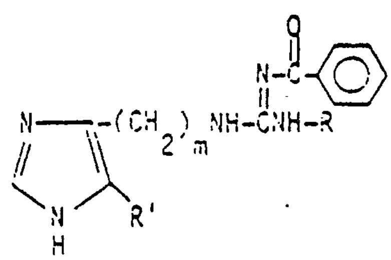

(Ia)

in which R, R' and m have the meanings defined above is hydrolysed or

$b_2$) a compound corresponding to the general formula VI

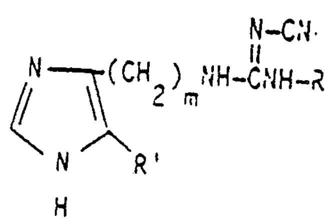

(VI)

in which R, R' and m have the meanings indicated above is hydrolysed with an acid to form a compound corresponding to the general formula I or

$b_3$) a compound corresponding to the general formula VII

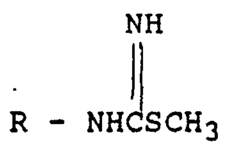

(VII)

in which R has the meaning indicated above is reacted with a compound corresponding to the general formula III

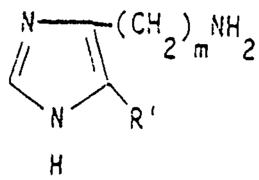

(III)

in which R' and m have the meanings indicated above to form a compound corresponding to the general formula I or

$b_4$) a compound corresponding to the general formula VIII

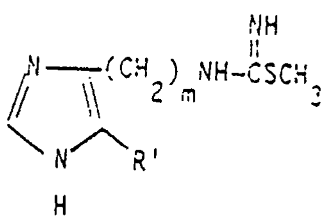

(VIII)

in which R' and m have the meanings defined above is reacted with a compound corresponding to the general formula V

$R - NH_2$ (V)

in which R has the meaning indicated above to form a compound corresponding to the general formula I

and in that the compounds obtained according to a) and b) are optionally converted into their physiologically acceptable salts in known manner.

**2.** A process according to claim 1 for the preparation of compounds in which R stands for the group

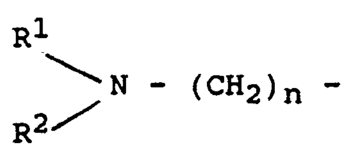

in which $R^1$ stands for a pyridine ring which is unsubstituted or mono- to tri- substituted with $C_1$-$C_3$-alkyl, $C_1$-$C_3$-alkoxy and/or halogen, $R^2$ stands for a benzyl group optionally mono- to tri- substituted with $C_1$-$C_3$-alkyl, $C_1$-$C_3$-alkoxy, halogen and/or trifluoromethyl or for a thiophenylmethyl, furanylmethyl or pyridylmethyl group, which groups are unsubstituted or mono- to tri- substituted with $C_1$-$C_3$-alkyl, $C_1$-$C_3$-alkoxy and/or halogen, n has the value 2, 3, or 4, X and $R^1$ stand each for a hydrogen atom and

m has the value 3.

3. A process according to claim 1 for the preparation of compounds in which R stands for the group

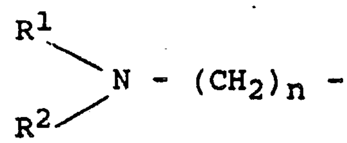

wherein $R^1$ stands for a phenyl ring which is unsubstituted or mono- to tri- substituted with $C_1$-$C_3$-alkyl, $C_1$-$C_3$-alkoxy, halogen, trifluoromethyl and/or hydroxy, $R^2$ stands for thiophenylmethyl, furanylmethyl or pyridylmethyl group, which groups are unsubstituted or mono- to tri- substituted with $C_1$-$C_3$-alkyl, $C_1$-$C_3$-alkoxy and/or halogen, n has the value 2, 3 or 4, X and R' stand each for a hydrogen atom and m has the value 3.

4. A process according to claim 1 for the preparation of compounds in which R stands for the group

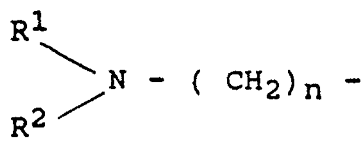

wherein $R^1$ stands for a pyridine ring which is unsubstituted or mono- to tri- substituted with $C_1$-$C_3$-alkyl, $C_1$-$C_3$-alkoxy and/or halogen and $R^2$ stands for a hydrogen atom, n has the value 2, 3, or 4, X and R' stand each for a hydrogen atom and m has the value 3.

5. A process according to claim 1 for the preparation of compounds in which R stands for the group

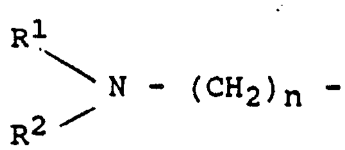

wherein $R^1$ stands for a pyridine ring which is unsubstituted or mono- to tri- substituted with $C_1$-$C_3$-alkyl, $C_1$-$C_3$-alkoxy and/or halogen, $R^2$ stands for a phenyl group which is unsubstituted or mono- to tri-substituted with $C_1$-$C_3$-alkyl, $C_1$-$C_3$-alkoxy, halogen, trifluoromethyl and/or hydroxy, n has the value 2, 3 or 4, X and R' stand each for a hydrogen atom and m has the value 3.

6. A process according to claim 1 for the preparation of compounds in which R stands for the group

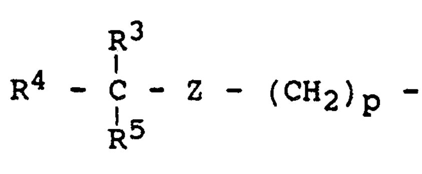

wherein $R^3$ and $R^4$, which may be identical or different, stand each for a phenyl group which is unsubstituted or mono- to tri- substituted with $C_1$-$C_3$-alkyl, $C_1$-$C_3$-alkoxy, halogen, trifluoromethyl and/or hydroxy, $R^5$ stands for a hydrogen atom or a methyl group and Z stands for a single bond, p has the value 2 or 3, X and R' stand each for a hydrogen atom and m has the value 3.

7. A process according to claim 1 for the preparation of compounds in which R stands for the group

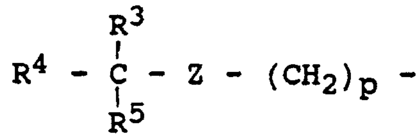

wherein $R^3$ stands for a pyridine ring which is unsubstituted or mono- to tri- substituted with $C_1$-$C_3$-alkyl, $C_1$-$C_3$-alkoxy and/or halogen, and $R^4$ stands for a phenyl group which is unsubstituted or mono- to tri- substituted with $C_1$-$C_3$-alkyl, $C_1$-$C_3$-alkoxy, halogen, trifluoromethyl and/or hydroxy, $R^5$ stands for a hydrogen atom or a methyl or a hydroxy group, Z denotes a single bond, p has the value 2 or 3, X stands for hydrogen atom and R' stands for a hydrogen atom or a methyl group and m has the value 2 or 3.

**8.** A process according to claim 1 for the preparation of compounds in which R stands for the group

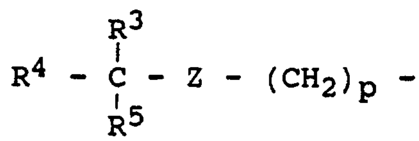

wherein $R^3$ stands for a pyridine ring which is unsubstituted or mono- to tri- substituted with $C_1$-$C_3$-alkyl, $C_1$-$C_3$-alkoxy and/or halogen, $R^4$ and $R^5$ stand each for a hydrogen atom, Z denotes a single bond and p has the value 2, 3 or 4, X and R'stand each for a hydrogen atom and m has the value 3.

**9.** A process according to claim 1 for the preparation of compounds in which R stands for the group

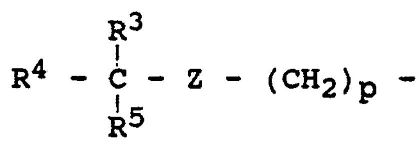

wherein $R^3$ and $R^4$, which may be identical or different, stand each for a phenyl ring which is unsubstituted or mono- to tri- substituted with $C_1$-$C_3$-alkyl, $C_1$-$C_3$-alkoxy, halogen, trifluoromethyl and/or hydroxy, $R^5$ denotes a hydrogen atom and Z an oxygen atom, p has the value 2 or 3, X and R' stand each for a hydrogen atom and m has the value 3.

**10.** A process according to claim 1 for the preparation of $N^1$-[3-(3,4-dichlorophenyl)-3-(pyridin-2-yl)propyl]-$N^2$-[3-(1H-imidazol-4-yl)propyl]guanidine and the physiologically acceptable salts thereof.

**11.** A process according to claim 1 for the preparation of $N^1$-[3-(3,4-difluorophenyl)-3-(pyridin-2-yl)propyl]-$N^2$-[3-(1H-imidazol-4-yl)propyl]guanidine and the physiologically acceptable salts thereof.

**12.** A process according to claim 1 for the preparation of $N^1$-[3-(4-fluorophenyl)-3-phenylpropyl]-$N^2$-[3-(1H-imidazol-4-yl)propyl]guanidine and the physiologically acceptable salts thereof.

**13.** A process according to claim 1 for the preparation of $N^1$-[3-(2,4-dichlorophenyl)-3-(pyridin-2-yl)propyl]-$N^2$-[3-(1H-imidazol-4-yl)propyl]guanidine and the physiologically acceptable salts thereof.

**14.** A process according to claim 1 for the preparation of $N^1$-[3-(3-chlorophenyl)-3-(pyridin-2-yl)propyl]-$N^2$-[3-(1H-imidazol-4-yl)propyl]guanidine and the physiologically acceptable salts thereof.

**15.** A process according to claim 1 for the preparation of $N^1$-[3-(4-fluorophenyl)-3-(pyridin-3-yl)propyl]-$N^2$-[3-(1H-imidazol-4-yl)propyl]guanidine and the physiologically acceptable salts thereof.

16. A process according to claim 1 for the preparation of $N^1$-[3-(3,5-difluorophenyl)-3-(pyridin-2-yl)propyl]-$N^2$-[3-(1H-imidazol-4-yl)propyl]guanidine and the physiologically acceptable salts thereof.

**Revendications**

**Revendications pour les Etats contractants suivants : AT, BE, CH, DE, FR, GB, IT, LI, LU, NL, SE**

1. Dérivés d'imidazolylguanidine de formule générale I

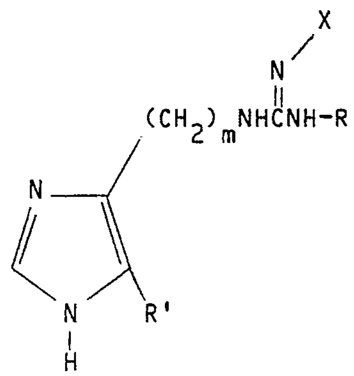

(I)

dans laquelle R représente le groupement

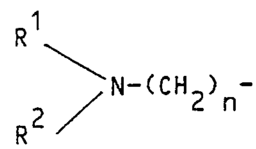

dans lequel $R^1$ représente un groupe phényle non substitué ou un groupe phényle substitué 1 à 3 fois par alkyle en $C_1$-$C_3$, alcoxy en $C_1$-$C_3$, halogène, trifluorométhyle et/ou hydroxy ou un noyau pyridine non substitué ou un noyau pyridine substitué 1 à 3 fois par alkyle en $C_1$-$C_3$, alcoxy en $C_1$-$C_3$ et/ou halogène, $R^2$ représente un atome d'hydrogène, un groupe alkyle en $C_1$-$C_3$, un groupe phényle éventuellement substitué 1 à 3 fois par alkyle en $C_1$-$C_3$, alcoxy en $C_1$-$C_3$, halogène, trifluorométhyle et/ou hydroxy, un groupe benzyle non substitué ou un groupe benzyle substitué 1 à 3 fois par alkyle en $C_1$-$C_3$, alcoxy en $C_1$-$C_3$, halogène et/ou trifluorométhyle ou un groupe thiénylméthyle, furylméthyle ou pyridylméthyle non substitué ou substitué 1 à 3 fois par alkyle en $C_1$-$C_3$,alcoxy en $C_1$-$C_3$ et/ou halogène et n est égal à 2, 3 ou 4, ou bien dans laquelle R représente le groupement

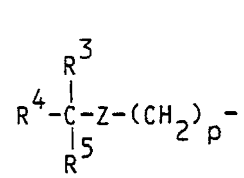

dans lequel $R^3$ représente un groupe phényle non substitué ou un groupe phényle substitué 1 à 3 fois par alkyle en $C_1$-$C_3$, alcoxy en $C_1$-$C_3$, halogène, trifluorométhyle et/ou hydroxy ou un noyau pyridine non substitué ou un noyau pyridine substitué 1 à 3 fois par alkyle en $C_1$-$C_3$, alcoxy en $C_1$-$C_3$ et/ou halogène, $R^4$ représente un atome d'hydrogène ou un groupe phényle éventuellement substitué 1 à 3 fois par alkyle en $C_1$-$C_3$, alcoxy en $C_1$-$C_3$, halogène, trifluorométhyle et/ou hydroxy, R représente un atome d'hydrogène ou un groupe méthyle ou hydroxy et Z représente une liaison simple ou un atome d'oxygène et p est égal à 2 ou 3,
X représente un atome d'hydrogène ou un groupe benzoyle, m est égal à 2 ou 3 et R' représente un atome d'hydrogène ou un groupe méthyle,
et leurs sels physiologiquement acceptables.

2. Dérivés d'imidazolylguanidine selon la revendication 1, caractérisés en ce que R représente le

groupement

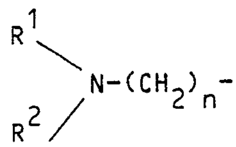

dans lequel $R^1$ représente un noyau pyridine non substitué ou substitué 1 à 3 fois par alkyle en $C_1$-$C_3$, alcoxy en $C_1$-$C_3$ et/ou halogène, $R^2$ représente un groupe benzyle éventuellement substitué 1 à 3 fois par alkyle en $C_1$-$C_3$, alcoxy en $C_1$-$C_3$, halogène et/ou trifluorométhyle ou un groupe thiénylméthyle, furylméthyle ou pyridylméthyle non substitué ou substitué 1 à 3 fois alkyle en $C_1$-$C_3$, alcoxy en $C_1$-$C_3$ et/ou halogène, n est égal à 2, 3 ou 4, X et R' représentent chacun un atome d'hydrogène et m est égal à 3.

**3.** Dérivés d'imidazolylguanidine selon la revendication 1, caractérisés en ce que R représente le groupement

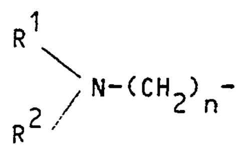

dans lequel $R^1$ représente un noyau phényle non substitué ou un noyau phényle substitué 1 à 3 fois par alkyle en $C_1$-$C_3$, alcoxy en $C_1$-$C_3$, halogène, trifluorométhyle et/ou hydroxy, $R^2$ représente un groupe thiénylméthyle, furylméthyle ou pyridylméthyle non substitué ou substitué 1 à 3 fois par alkyle en $C_1$-$C_3$, alcoxy en $C_1$-$C_3$ ou halogène, n est égal à 2, 3 ou 4, X et R' représentent chacun un atome d'hydrogène et m est égal à 3.

**4.** Dérivés d'imidazolylguanidine selon la revendication 1, caractérisés en ce que R représente le groupement

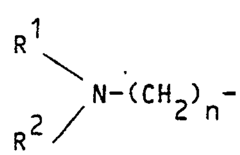

dans lequel $R^1$ représente un noyau pyridine non substitué ou substitué 1 à 3 fois par alkyle en $C_1$-$C_3$, alcoxy en $C_1$-$C_3$ et/ou halogène et $R^2$ représente un atome d'hydrogène, n est égal à 2, 3 ou 4, X et R' représentent chacun un atome d'hydrogène et m est égal à 3.

**5.** Dérivés d'imidazolylguanidine selon la revendication 1, caractérisés en ce R représente un groupement

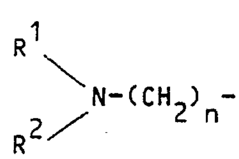

dans lequel $R^1$ représente un noyau pyridine non substitué ou substitué 1 à 3 fois par alkyle en $C_1$-$C_3$, alcoxy en $C_1$-$C_3$ et/ou halogène et $R^2$ représente un groupe phényle non substitué ou substitué 1 à 3 fois par alkyle en $C_1$-$C_3$, alcoxy en $C_1$-$C_3$, halogène, trifluorométhyle et/ou hydroxy, n est égal à 2, 3 ou 4, X et R' représentent chacun un atome d'hydrogène et m est égal à 3.

6. Dérivés d'imidazolylguanidine selon la revendication 1, caractérisés en ce que R représente le groupement

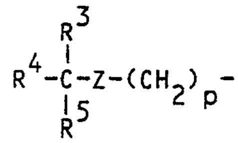

dans lequel $R^3$ et $R^4$, qui peuvent être identiques ou différents, représentent chacun un groupe phényle non substitué ou substitué 1 à 3 fois par alkyle en $C_1$-$C_3$, alcoxy en $C_1$-$C_3$, halogène, trifluorométhyle et/ou hydroxy, $R^5$ représente un atome d'hydrogène ou un groupe méthyle et Z représente un liaison simple, p est égal à 2 ou 3, X et R' représentent chacun un atome d'hydrogène et m est égal à 3.

7. Dérivés d'imidazolylguanidine selon la revendication 1, caractérisés en ce que R représente le groupement

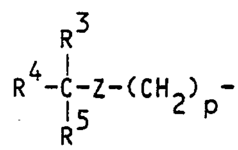

dans lequel R représente un noyau pyridine non substitué ou substitué 1 à 3 fois par alkyle en $C_1$-$C_3$, alcoxy en $C_1$-$C_3$ et/ou halogène et $R^4$ représente un groupe phényle non substitué ou substitué 1 à 3 fois par alkyle en $C_1$-$C_3$, alcoxy en $C_1$-$C_3$, halogène trifluorométhyle et/ou hydroxy, $R^5$ représente un atome d'hydrogène ou un groupe méthyle ou hydroxy, Z représente une liaison simple, p est égal à 2 ou 3, X représente un atome d'hydrogène et R' représente un atome d'hydrogène ou un groupe méthyle et m est égal à 2 ou 3.

8. Dérivés d'imidazolylguanidine selon la revendication 1, caractérisés en ce que R représente le groupement

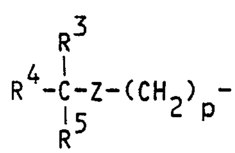

dans lequel $R^3$ représente un noyau pyridine non substitué ou substitué 1 à 3 fois par alkyle en $C_1$-$C_3$, alcoxy en $C_1$-$C_3$ et/ou halogène, $R^4$ et $R^5$ représentent chacun un atome d'hydrogène, Z représente une liaison simple et p est égal à 2, 3 ou 4, X et R' représentent chacun un atome d'hydrogène et m est égal 3.

9. Dérivés d'imidazolylguanidine selon la revendication 1, caractérisés en ce que R représente le groupement

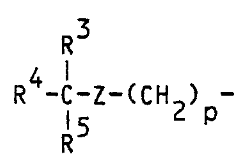

dans lequel $R^3$ et $R^4$, qui peuvent être identiques ou différents, représentent chacun un noyau phényle

non substitué ou substitué 1 à 3 fois par alkyle en $C_1$-$C_3$, alcoxy en $C_1$-$C_3$, halogène, trifluorométhyle et/ou hydroxy, $R^5$ représente un atome d'hydrogène et Z représente un atome d'oxygène, p est égal à 2 ou 3, X et R' représentent chacun un atome d'hydrogène et m est égal à 3.

**10.** La $N^1$-[3-(3,4-dichlorophényl)-3-(pyridine-2-yl)propyl]-$N^2$-[3-(1H-imidazole-4-yl)propyl]guanidine et ses sels physiologiquement acceptables.

**11.** La $N^1$-[3-(3,4-difluorophényl)-3-(pyridine-2-yl)propyl-$N^2$-[3-(1H-imidazole-4-yl)propyl]guanidine et ses sels physiologiquement acceptables.

**12.** La $N^1$-[3-(4-fluorophényl)-3-phénylpropyl]-$N^2$-[3-(1H-imidazole-4-yl)-propyl]guanidine et ses sels physiologiquement acceptables.

**13.** La $N^1$-[3-(2,4-dichlorophényl)-3-(pyridine-2-yl)propyl]-$N^2$-[3-(1H-imidazole-4-yl)propyl]guanidine et ses sels physiologiquement acceptables.

**14.** La $N^1$-[3-(3-chlorophényl)-3-(pyridine-2-yl)propyl]-$N^2$-[3-(1H-imidazole-4-yl)propyl]guanidine et ses sels physiologiquement acceptables.

**15.** La $N^1$-[3-(4-fluorophényl)-3-(pyridine-3-yl)propyl]-$N^2$-[3-(1H-imidazole-4-yl)propyl]guanidine et ses sels physiologiquement acceptables.

**16.** La $N^1$-[3-(3,5-difluorophényl)-3-(pyridine-2-yl)propyl]-$N^2$-[3-(1H-imidazole-4-yl)propyl]guanidine et ses sels physiologiquement acceptables.

**17.** Procédé pour la fabrication de dérivés d'imidazolylguanidine selon les revendications 1 à 16 et de leurs sels physiologiquement acceptables, caractérisé en ce que

a) pour la fabrication de composés de formule générale I dans laquelle R, R' et m sont définis comme à la revendication 1 et X représente un groupe benzoyle,

$a_1$) on fait réagir un composé de formule générale II

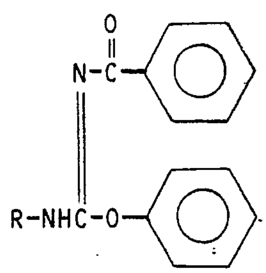

(II)

dans laquelle R est défini comme à la revendication 1, avec un composé de formule générale III

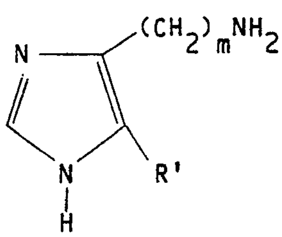

(III)

dans laquelle R' et m sont définis comme à la revendication 1, en un composé de formule générale I, ou bien

$a_2$) on fait réagir un composé de formule générale IV

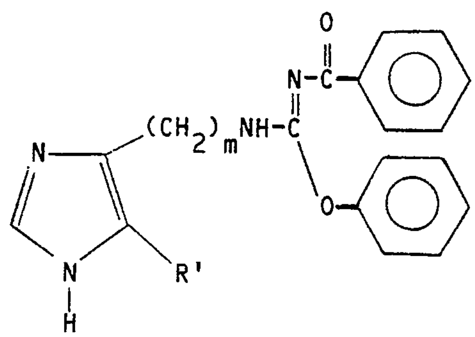

(IV)

dans laquelle R' et m sont définis comme à la revendication 1, avec un composé de formule générale V

$R\text{-}NH_2$ (V)

dans laquelle R est défini comme à la revendication 1, en un composé de formule générale I, ou bien

b) pour la fabrication de composés de formule générale I dans laquelle R, R' et m sont définis comme à la revendication 1 et X représente un atome d'hydrogène,

$b_1$) on hydrolyse un composé de formule générale Ia

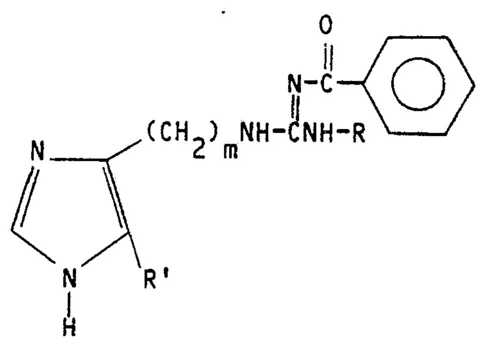

(Ia)

dans laquelle R, R' et m sont définis comme à la revendication 1, ou bien

$b_2$) on hydrolyse un composé de formule générale VI

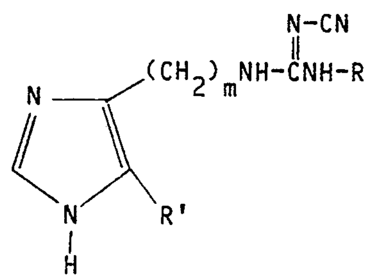

(VI)

dans laquelle R, R' et m sont définis comme à la revendication 1, à l'aide d'un acide en un composé de formule générale I, ou bien

$b_3$) on fait réagir un composé de formule générale VII

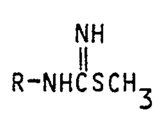

(VII)

dans laquelle R est défini comme à la revendication 1, avec un composé de formule générale III

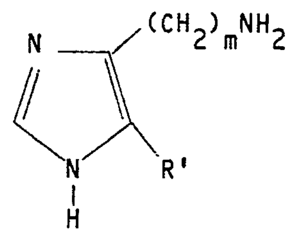

(III)

dans laquelle R' et m sont définis comme à la revendication 1, en un composé de formule générale I, ou bien

$b_4$) on fait réagir un composé de formule générale VIII

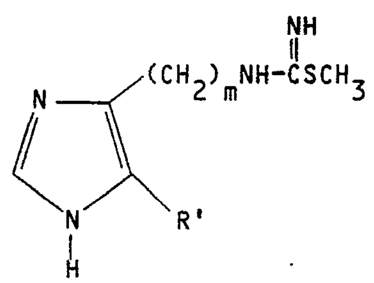

(VIII)

dans laquelle R' et m sont définis comme à la revendication 1, avec un composé de formule générale V

$R\text{-}NH_2$ (V)

dans laquelle R est défini comme à la revendication 1, en un composé de formule générale I

et en ce que l'on transforme éventuellement de manière connue les composés obtenus selon a) et b) en leur sel physiologiquement acceptable.

**18.** Médicament, caractérisé en ce qu'il contient un composé selon l'une des revendications 1 à 16 et au moins un support pharmaceutiquement acceptable ou un agent diluant inerte pharmaceutiquement acceptable.

**Revendications pour les Etats contractants suivants : ES, GR**

**1.** Procédé pour la fabrication de dérivés d'imidazolylguanidine de formule générale I

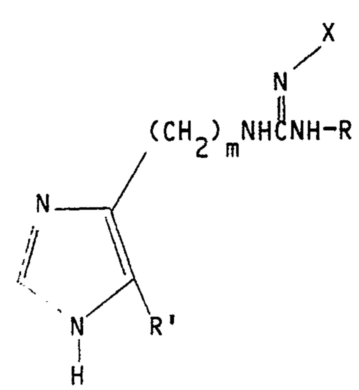

(I)

dans laquelle R représente le groupement

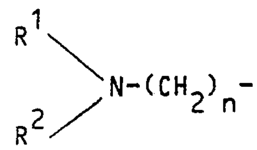

dans lequel $R^1$ représente un groupe phényle non substitué ou un groupe phényle substitué 1 à 3 fois par alkyle en $C_1$-$C_3$, alcoxy en $C_1$-$C_3$, halogène, trifluorométhyle et/ou hydroxy ou un noyau pyridine non substitué ou un noyau pyridine substitué 1 à 3 fois par alkyle en $C_1$-$C_3$, alcoxy en $C_1$-$C_3$ et/ou halogène, $R^2$ représente un atome d'hydrogène, un groupe alkyle en $C_1$-$C_3$, un groupe phényle éventuellement substitué 1 à 3 fois par alkyle en $C_1$-$C_3$, alcoxy en $C_1$-$C_3$, halogène, trifluorométhyle et/ou hydroxy, un groupe benzyle non substitué ou un groupe benzyle substitué 1 à 3 fois par alkyle en $C_1$-$C_3$, alcoxy en $C_1$-$C_3$, halogène et/ou trifluorométhyle ou un groupe thiénylméthyle, furylméthyle ou pyridylméthyle non substitué ou substitué 1 à 3 fois par alkyle en $C_1$-$C_3$,alcoxy en $C_1$-$C_3$ et/ou halogène et n est égal à 2, 3 ou 4, ou bien dans laquelle R représente le groupement

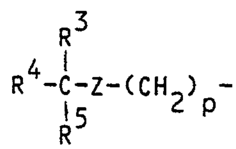

dans lequel $R^3$ représente un groupe phényle non substitué ou un groupe phényle substitué 1 à 3 fois par alkyle en $C_1$-$C_3$, alcoxy en $C_1$-$C_3$, halogène, trifluorométhyle et/ou hydroxy ou un noyau pyridine non substitué ou un noyau pyridine substitué 1 à 3 fois par alkyle en $C_1$-$C_3$, alcoxy en $C_1$-$C_3$ et/ou halogène, $R^4$ représente un atome d'hydrogène ou un groupe phényle éventuellement substitué 1 à 3 fois par alkyle en $C_1$-$C_3$, alcoxy en $C_1$-$C_3$, halogène, trifluorométhyle et/ou hydroxy, $R^5$ représente un atome d'hydrogène ou un groupe méthyle ou hydroxy et Z représente une liaison simple ou un atome d'oxygène et p est égal à 2 ou 3,

X représente un atome d'hydrogène ou un groupe benzoyle, m est égal à 2 ou 3 et R' représente un atome d'hydrogène ou un groupe méthyle, et de leurs sels physiologiquement acceptables, caractérisé en ce que

a) pour la fabrication de composés de formule générale I dans laquelle R, R' et m sont définis comme à la revendication 1 et X représente un groupe benzoyle,

$a_1$) on fait réagir un composé de formule générale II

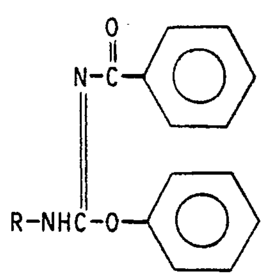

(II)

dans laquelle R a la signification indiquée ci-dessus, avec un composé de formule générale III

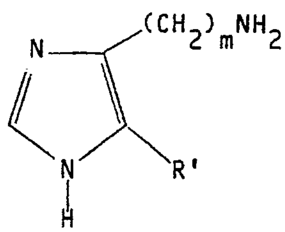

(III)

dans laquelle R' et m ont les significations ci-dessus, en un composé de formule générale I, ou bien

$a_2$) on fait réagir un composé de formule générale IV

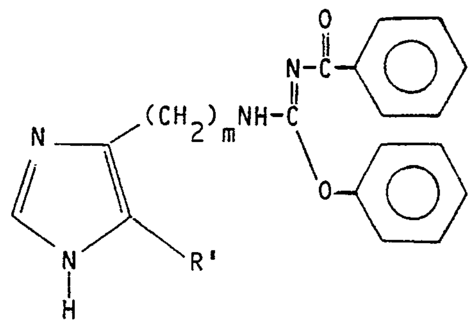

(IV)

dans laquelle R' et m ont les significations ci-dessus, avec un composé de formule générale V

$R\text{-}NH_2$ (V)

dans laquelle R est défini comme à la revendication 1, en un composé de formule générale I, ou bien

b) pour la fabrication de composés de formule générale I dans laquelle R, R' et m sont définis comme ci-dessus et X représente un atome d'hydrogène,

$b_1$) on hydrolyse un composé de formule générale Ia

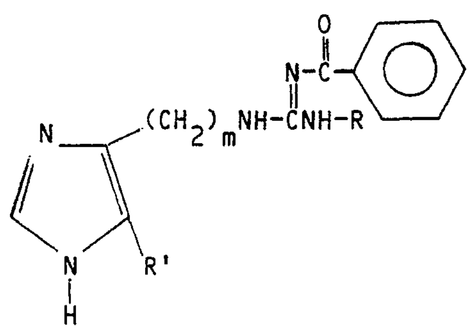

(Ia)

dans laquelle R, R' et m sont définis comme ci-dessus ou bien

$b_2$) on hydrolyse un composé de formule générale VI

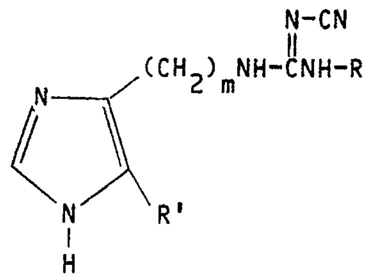

(VI)

dans laquelle R, R' et m ont les significations ci-dessus, à l'aide d'un acide en un composé de formule générale I, ou bien

$b_3$) on fait réagir un composé de formule générale VII

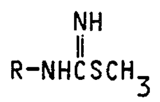

(VII)

dans laquelle R a la signification ci-dessus, avec un composé de formule générale III

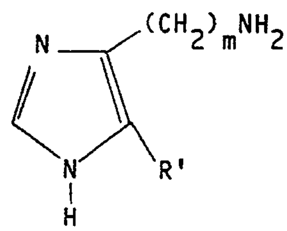

(III)

dans laquelle R' et m ont les significations ci-dessus, en un composé de formule générale I, ou bien

$b_4$) on fait réagir un composé de formule générale VIII

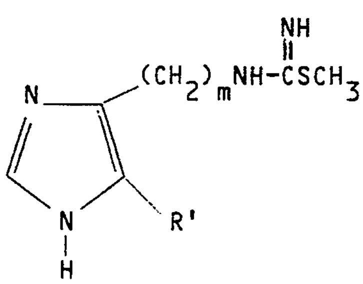

(VIII)

dans laquelle R' et m sont définis comme ci-dessus, avec un composé de formule générale V

$R\text{-}NH_2$ (V)

dans laquelle R a la signification ci-dessus, en un composé de formule générale I

et en ce que l'on transforme éventuellement de manière connue les composés obtenus selon a) et b) en leur sel physiologiquement acceptable.

**2.** Procédé selon la revendication 1, pour la fabrication de composés dans lesquels R représente le groupement

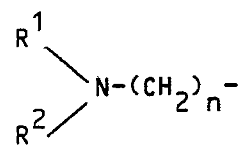

dans lequel $R^1$ représente un noyau pyridine non substitué ou substitué 1 à 3 fois par alkyle en $C_1$-$C_3$, alcoxy en $C_1$-$C_3$ et/ou halogène, $R^2$ représente un groupe benzyle éventuellement substitué 1 à 3 fois par alkyle en $C_1$-$C_3$, alcoxy en $C_1$-$C_3$, halogène et/ou trifluorométhyle ou un groupe thiénylméthyle, furylméthyle ou pyridylméthyle non substitué ou substitué 1 à 3 fois alkyle en $C_1$-$C_3$, alcoxy en $C_1$-$C_3$ et/ou halogène, n est égal à 2, 3 ou 4, X et R' représentent chacun un atome d'hydrogène et m est égal à 3.

**3.** Procédé selon la revendication 1, pour la fabrication de composés dans lesquels R représente le groupement

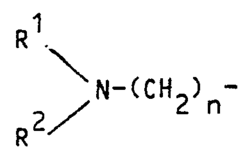

dans lequel $R^1$ représente un noyau phényle non substitué ou un noyau phényle substitué 1 à 3 fois par alkyle en $C_1$-$C_3$, alcoxy en $C_1$-$C_3$, halogène, trifluorométhyle et/ou hydroxy, $R^2$ représente un groupe thiénylméthyle, furylméthyle ou pyridylméthyle non substitué ou substitué 1 à 3 fois par alkyle en $C_1$-$C_3$, alcoxy en $C_1$-$C_3$ ou halogène, n est égal à 2, 3 ou 4, x et R' représentent chacun un atome d'hydrogène et m est égal à 3.

**4.** Procédé selon la revendication 1, pour la fabrication de composés dans lesquels R représente le groupement

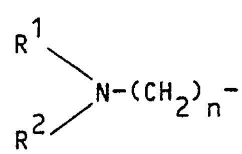

dans lequel $R^1$ représente un noyau pyridine non substitué ou substitué 1 à 3 fois par alkyle en $C_1$-$C_3$, alcoxy en $C_1$-$C_3$ et/ou halogène et $R^2$ représente un atome d'hydrogène, n est égal à 2, 3 ou 4, X et R' représentent chacun un atome d'hydrogène et m est égal à 3.

**5.** Procédé selon la revendication 1, pour la fabrication de composés dans lesquels R représente le groupement

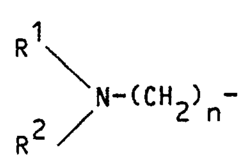

dans lequel $R^1$ représente un noyau pyridine non substitué ou substitué 1 à 3 fois par alkyle en $C_1$-$C_3$, alcoxy en $C_1$-$C_3$ et/ou halogène et $R^2$ représente un groupe phényle non substitué ou substitué 1 à 3 fois par alkyle en $C_1$-$C_3$, alcoxy en $C_1$-$C_3$, halogène, trifluorométhyle et/ou hydroxy, n est égal à 2, 3 ou 4, X et R' représentent chacun un atome d'hydrogène et m est égal à 3.

**6.** Procédé selon la revendication 1, pour la fabrication de composés dans lesquels R représente le

groupement

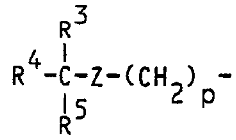

dans lequel $R^3$ et $R^4$, qui peuvent être identiques ou différents, représentent chacun un groupe phényle non substitué ou substitué 1 à 3 fois par alkyle en $C_1$-$C_3$, alcoxy en $C_1$-$C_3$, halogène, trifluorométhyle et/ou hydroxy, $R^5$ représente un atome d'hydrogène ou un groupe méthyle et Z représente un liaison simple, p est égal à 2 ou 3, X et R' représentent chacun un atome d'hydrogène et m est égal à 3.

7. Procédé selon la revendication 1, pour la fabrication de composés dans lesquels R représente le groupement

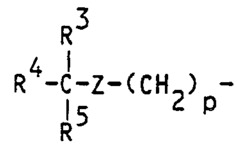

dans lequel $R^3$ représente un noyau pyridine non substitué ou substitué 1 à 3 fois par alkyle en $C_1$-$C_3$, alcoxy en $C_1$-$C_3$ et/ou halogène et $R^4$ représente un groupe phényle non substitué ou substitué 1 à 3 fois par alkyle en $C_1$-$C_3$, alcoxy en $C_1$-$C_3$, halogène, trifluorométhyle et/ou hydroxy, $R^5$ représente un atome d'hydrogène ou un groupe méthyle ou hydroxy, Z représente une liaison simple, p est égal à 2 ou 3, X représente un atome d'hydrogène et R' représente un atome d'hydrogène ou un groupe méthyle et m est égal à 2 ou 3.

8. Procédé selon la revendication 1, pour la fabrication de composés dans lesquels R représente le groupement

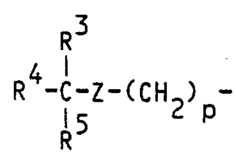

dans lequel $R^3$ représente un noyau pyridine non substitué ou substitué 1 à 3 fois par alkyle en $C_1$-$C_3$, alcoxy en $C_1$-$C_3$ et/ou halogène, $R^4$ et $R^5$ représentent chacun un atome d'hydrogène, Z représente une liaison simple et p est égal à 2, 3 ou 4, X et R' représentent chacun un atome d'hydrogène et m est égal 3.

9. Procédé selon la revendication 1, pour la fabrication de composés dans lesquels R représente le groupement

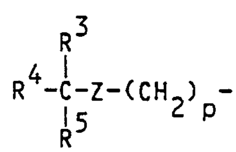

dans lequel $R^3$ et $R^4$, qui peuvent être identiques ou différents, représentent chacun un noyau phényle non substitué ou substitué 1 à 3 fois par alkyle en $C_1$-$C_3$, alcoxy en $C_1$-$C_3$, halogène, trifluorométhyle et/ou hydroxy, $R^5$ représente un atome d'hydrogène et Z représente un atome d'oxygène, p est égal à

2 ou 3, X et R' représentent chacun un atome d'hydrogène et m est égal à 3.

**10.** Procédé selon la revendication 1, pour la fabrication de la $N^1$-[3-(3,4-dichlorophényl)-3-(pyridine-2-yl)-propyl]-$N^2$-[3-(1H-imidazole-4-yl)propyl]guanidine et de ses sels physiologiquement acceptables.

**11.** Procédé selon la revendication 1, pour la fabrication de la $N^1$-[3-(3,4-difluorophényl)-3-(pyridine-2-yl)-propyl]-$N^2$-[3-(1H-imidazole-4-yl)propyl]guanidine et de ses sels physiologiquement acceptables.

**12.** Procédé selon la revendication 1, pour la fabrication de la $N^1$-[3-(4-fluorophényl)-3-phénylpropyl]-$N^2$-[3-(1H-imidazole-4-yl)propyl]guanidine et de ses sels physiologiquement acceptables.

**13.** Procédé selon la revendication 1, pour la fabrication de la $N^1$-[3-(2,4-dichlorophényl)-3-(pyridine-2-yl)-propyl]-$N^2$-[3-(1H-imidazole-4yl)propyl]guanidine et de ses sels physiologiquement acceptables.

**14.** Procédé selon la revendication 1, pour la fabrication de la $N^1$-[3-(3-chlorophényl)-3-(pyridine-2-yl)-propyl]-$N^2$-[3-(1H-imidazole-4-yl)propyl]guanidine et de ses sels physiologiquement acceptables.

**15.** Procédé selon la revendication 1, pour la fabrication de la $N^1$-[3-(4-fluorophényl)-3-(pyridine-3-yl)-propyl]-$N^2$-[3-(1H-imidazole-4-yl)propyl]guanidine et de ses sels physiologiquement acceptables.

**16.** Procédé selon la revendication 1, pour la fabrication de la $N^1$-[3-(3,5-difluorophényl)-3-(pyridine-2-yl)-propyl]-$N^2$-[3-(1H-imidazole-4-yl)propyl]guanidine et de ses sels physiologiquement acceptables.